(19) Europäisches Patentamt
European Patent Office
Office européen des brevets

(11) EP 4 585 602 A1

(12) **EUROPEAN PATENT APPLICATION**
published in accordance with Art. 153(4) EPC

(43) Date of publication:
**16.07.2025 Bulletin 2025/29**

(21) Application number: **23932519.4**

(22) Date of filing: **14.04.2023**

(51) International Patent Classification (IPC):
***C07H 15/04*** (2006.01) ***A61K 31/7088*** (2006.01)
***A61K 31/713*** (2006.01)

(52) Cooperative Patent Classification (CPC):
Y02P 20/55

(86) International application number:
**PCT/CN2023/088483**

(87) International publication number:
**WO 2024/212237 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Rigerna Therapeutics (Beijing) Co., Ltd.**
**Beijing 102629 (CN)**

(72) Inventors:
- **HUANG, Yuanyu**
  **Beijing 102629 (CN)**
- **HAN, Xiaofeng**
  **Beijing 102629 (CN)**

(74) Representative: **Maiwald GmbH**
**Elisenhof**
**Elisenstraße 3**
**80335 München (DE)**

Remarks:
The complete document including Reference Table(s) and the Sequence Listing(s) can be downloaded from the EPO website

(54) **COMPOUND, CONJUGATE, COMPOSITION, AND USES THEREOF**

(57) The present disclosure relates to a compound, a conjugate, a composition, and uses thereof. Specifically, the present disclosure relates to a compound having a structure represented by specific formula (Ia), a stereoisomer, pharmaceutically acceptable salt, or prodrug thereof. The compound can be used for preventing and/or treating the expression or activity of a specific target gene.

EP 4 585 602 A1

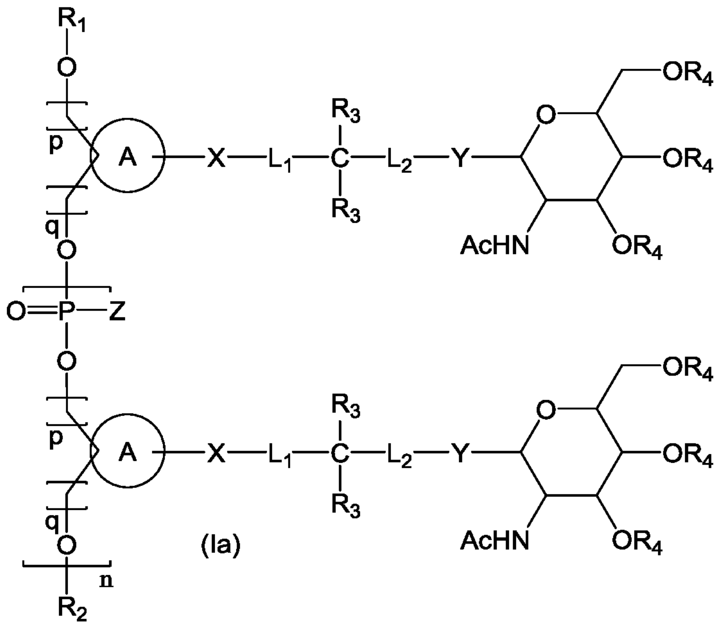

(Ia)

## Description

## FIELD

The present disclosure relates to the technical field of pharmaceuticals, and in particular to a compound, a conjugate or a composition thereof, and use thereof.

## BACKGROUND

Small nucleic acid drugs, including typical examples of small interference RNA (siRNA), antisense oligodeoxynucleotide (ASODN), and nucleic acid stimulatory motifs (CpG), play an increasingly significant role in gene therapy. Some drugs have already been approved for marketing by the Food and Drug Administration (FDA), while many more nucleic acid drugs are currently in preclinical and clinical trials. Nucleic acid drugs refer to nucleic acid sequences that specifically target disease-causing genes or proteins through binding or cleavage, thereby inhibiting/promoting the expression of certain genes/proteins. The nucleic acid drugs include normal human genes for use in the replacement of defective genes, antisense nucleic acids that can block gene expression, and single-stranded nucleic acids that can promote triplex formation of nucleic acid, such as siRNA, DNA, MicroRNA or CpG.

Delivery system is one of the key technologies in the development of small nucleic acid drugs. Globally, the most extensively studied delivery system for small nucleic acids is the targeted delivery technology via conjugation. The development of a new drug conjugate with high *in vivo* delivery efficiency, low toxicity, and high activity remains an urgent demand in this field.

## SUMMARY

The present disclosure aims to address, to some extent, at least one of the technical problems of the prior art.

In view of this, in a first aspect of the present disclosure, provided is a compound represented by formula (Ia), a stereoisomer, a pharmaceutically acceptable salt, or a prodrug thereof,

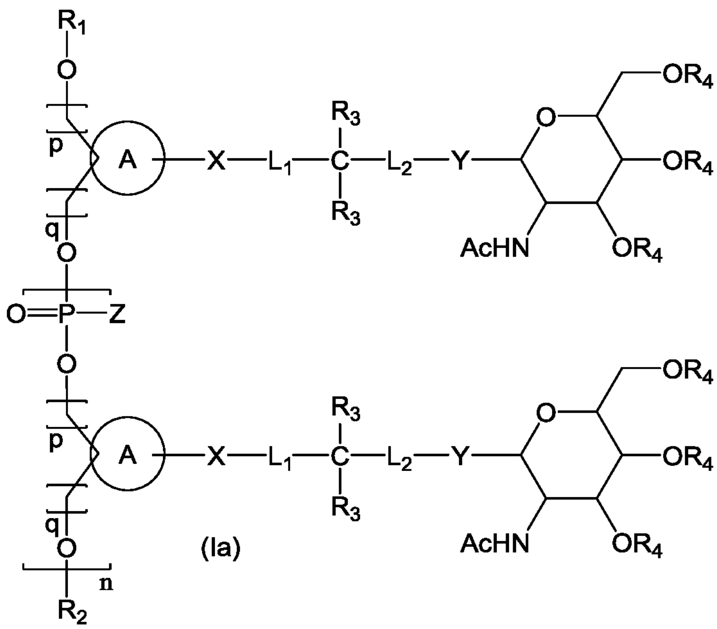

(Ia)

in formula (Ia), $R_1$ represents a hydroxyl protecting group,

$R_2$ is selected from the group consisting of:

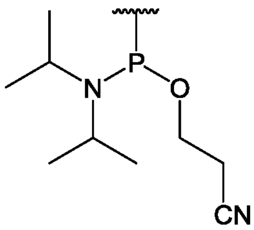,
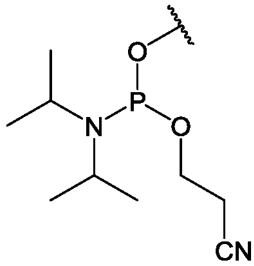,

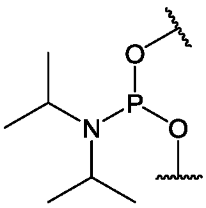,
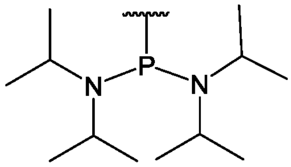

and

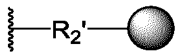,

wherein

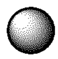

represents a solid phase support, and $R_2$' is a covalent linking group connected to the solid phase support,

n is selected from the group consisting of 0, 1, 2 and 3,

each Z is independently selected from the group consisting of hydroxyl and mercapto,

each p is independently selected from the group consisting of 1, 2 and 3,

each q is independently selected from the group consisting of 1, 2 and 3,

each A is independently an unsubstituted or substituted 4 to 10-membered aliphatic ring,

each X is independently selected from the group consisting of NH, O and S,

each $L_1$ is independently selected from the group consisting of

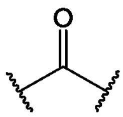,
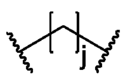

and

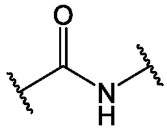,

wherein j is selected from the group consisting of 1, 2, 3, 4, 5, 6, 7, 8, 9 and 10,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

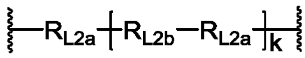,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10,

each Y is independently selected from the group consisting of NH, O and S, and

each $R_4$ is independently selected from the group consisting of:

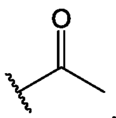, 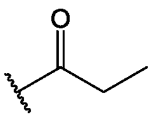,

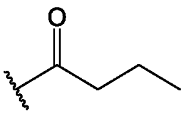, 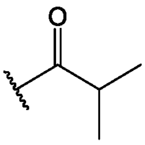, 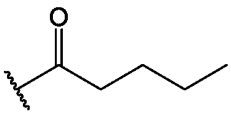, 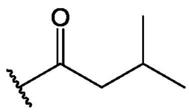, 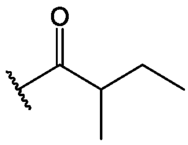,

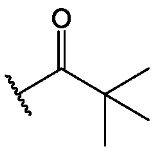, 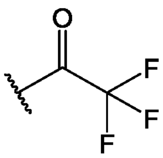 and 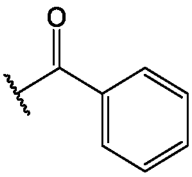.

Exemplarily, when n is 0, the compound has a structure represented by formula (Ia-0):

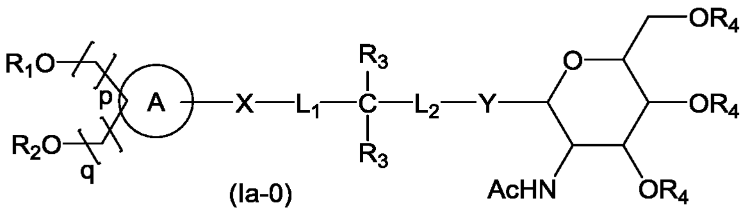

(Ia-0).

In some embodiments of the present disclosure, in formula (Ia), $R_1$ is selected from the group consisting of trityl (Tr), 4-methoxytrityl (MMTr), 4,4'-dimethoxytrityl (DMTr) and 4,4',4"-trimethoxytrityl (TMTr).

In some specific embodiments of the present disclosure, in formula (Ia), $R_1$ is 4,4'-dimethoxytrityl (DMTr).

In some specific embodiments of the present disclosure, in formula (Ia), $R_2$

is 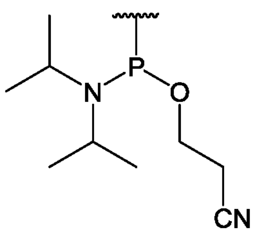.

In some specific embodiments of the present disclosure, in formula (Ia), $R_2$ is

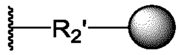,

wherein

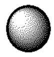

represents a solid phase support, and $R_2'$ is

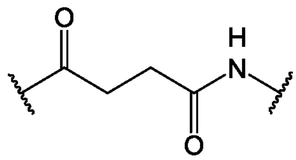.

That is, $R_2$ is

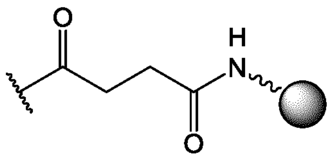

in formula (Ia).

In some specific embodiments of the present disclosure, each Z in formula (Ia) is hydroxyl.

In some embodiments of the present disclosure, each p in formula (Ia) is independently selected from the group consisting of 1 and 2.

In some specific embodiments of the present disclosure, each p in formula (Ia) is 1.

In some embodiments of the present disclosure, each q in formula (Ia) is independently selected from the group consisting of 1 and 2.

In some specific embodiments of the present disclosure, each q in formula (Ia) is 1.

In some specific embodiments of the present disclosure, in formula (Ia), each p is 1 and each q is 1.

In some embodiments of the present disclosure, each A in formula (Ia) is independently selected from the group consisting of an unsubstituted or substituted 4- to 10-membered cycloalkane group and an unsubstituted or substituted 4- to 10-membered cycloolefine group.

In some embodiments of the present disclosure, each A in formula (Ia) is independently an unsubstituted or substituted 4- to 10-membered cycloalkane group.

In some embodiments of the present disclosure, each A in formula (Ia) is independently a 4- to 10-membered cycloalkane group, such as a monocyclic ring, a spirocyclic ring, or a bridged ring.

In some embodiments of the present disclosure, each A in formula (Ia) is independently selected from the group consisting of

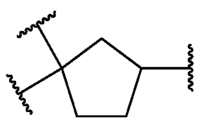, 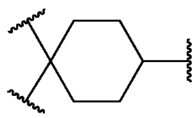, 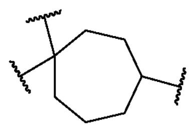,

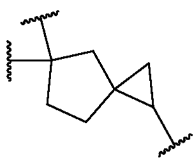 and 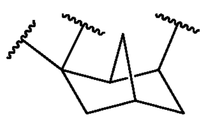.

In some specific embodiments of the present disclosure, each A in formula (Ia) is

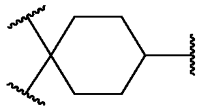.

In some specific embodiments of the present disclosure, each X in formula (Ia) is NH.

In some specific embodiments of the present disclosure, each $L_1$ in formula (Ia) is

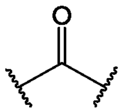.

In some specific embodiments of the present disclosure, each $R_3$ in formula (Ia) is H.

In some embodiments of the present disclosure, in formula (Ia), each $L_2$ is independently selected from the group consisting of $C_1$-$C_{10}$ alkylidene and

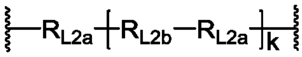,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.

In some specific embodiments of the present disclosure, each $R_{L2b}$ is -NH-C(O)-.

In some specific embodiments of the present disclosure, k is 1.

In some embodiments of the present disclosure, in formula (Ia), each $L_2$ is independently selected from the group consisting of:

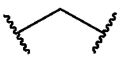, 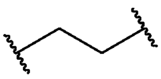, 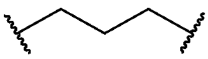, 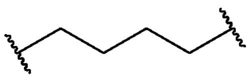, 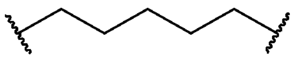,

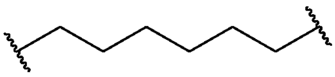, 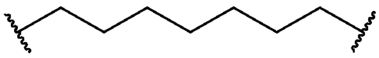, 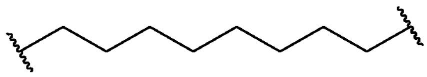,

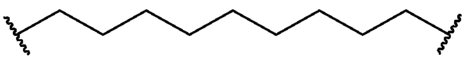, 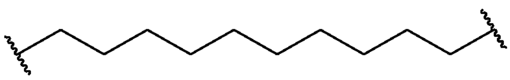,

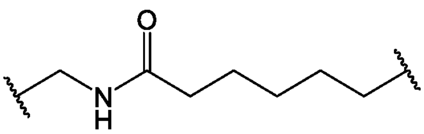, 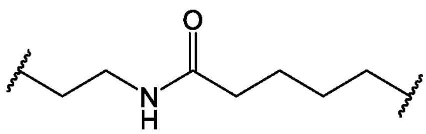,

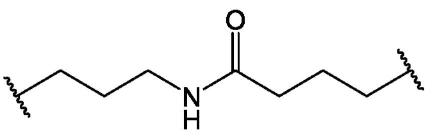, 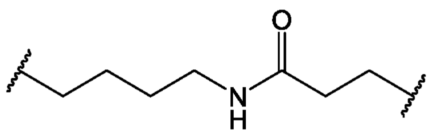

and

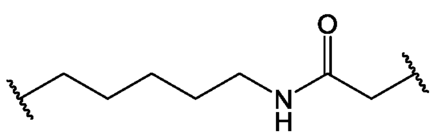.

In some specific embodiments of the present disclosure, in formula (Ia), each Y is O.

In some specific embodiments of the present disclosure, in formula (Ia), each $R_4$ is

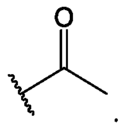

.

In some embodiments of the present disclosure, the compound has a structure represented by formula (IIa),

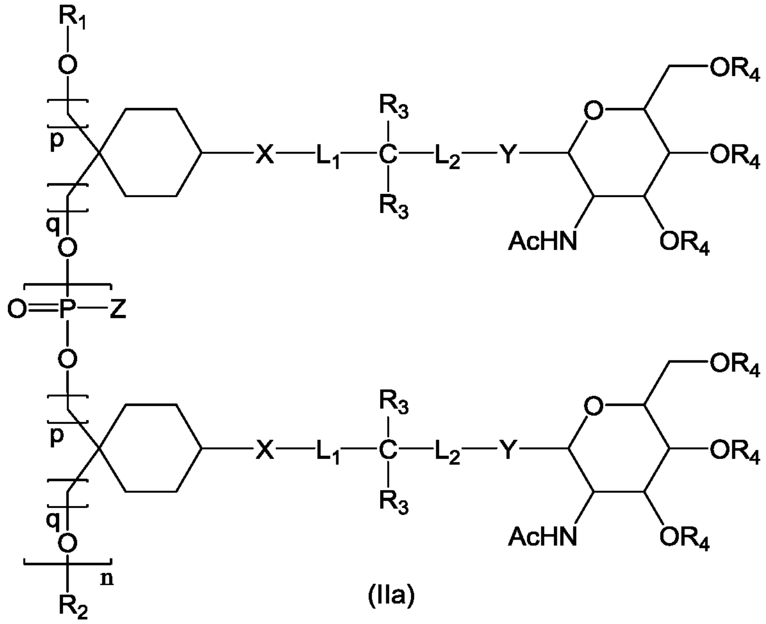

(IIa)

in formula (IIa), $R_1$ represents a hydroxyl protecting group,

$R_2$ is selected from the group consisting of

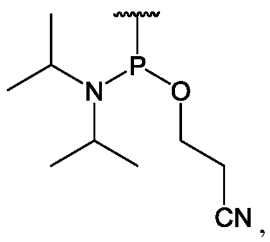, 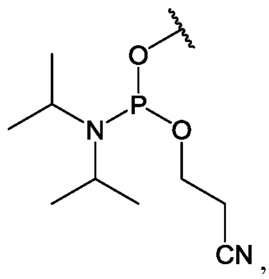,

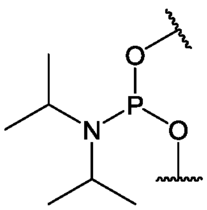, 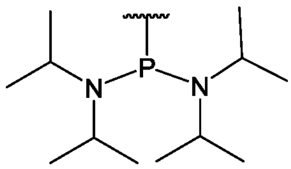 and 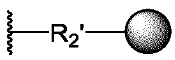,

wherein

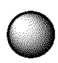

represents a solid phase support, and $R_2$' is a covalent linking group connected to the solid phase support,

n is selected from the group consisting of 0, 1, 2 and 3,

each Z is independently selected from the group consisting of hydroxyl and mercapto,

each p is independently selected from the group consisting of 1, 2 and 3,

each q is independently selected from the group consisting of 1, 2 and 3,

each X is independently selected from the group consisting of NH, O and S,

each $L_1$ is independently selected from the group consisting of

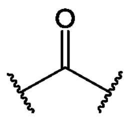 , 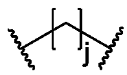

and

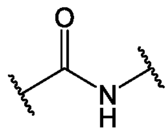 ,

, wherein j is selected from the group consisting of 1, 2, 3, 4, 5, 6, 7, 8, 9 and 10,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

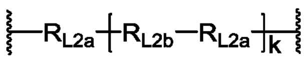 ,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10,

each Y is independently selected from the group consisting of NH, O and S, and

each $R_4$ is independently selected from the group consisting of:

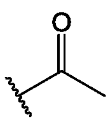 , 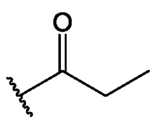 ,

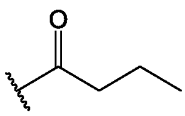 , 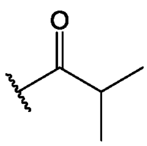 , 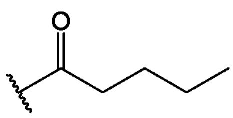 , 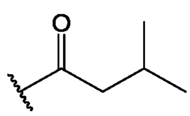 , 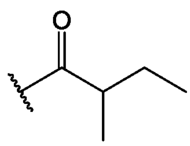 ,

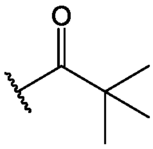, 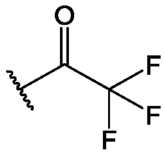 and 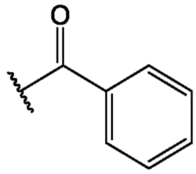.

Exemplarily, when n is 0, the compound has the following structure:

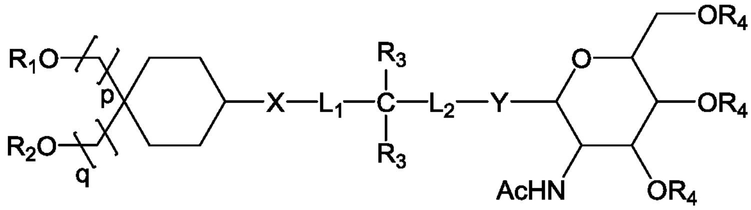.

In some embodiments of the present disclosure, in formula (IIa), $R_1$ is selected from the group consisting of trityl (Tr), 4-methoxytrityl (MMTr), 4,4'-dimethoxytrityl (DMTr) and 4,4',4"-trimethoxytrityl (TMTr).

In some specific embodiments of the present disclosure, in formula (IIa), $R_1$ is 4,4'-dimethoxytrityl (DMTr).

In some specific embodiments of the present disclosure, in formula (IIa), $R_2$ is

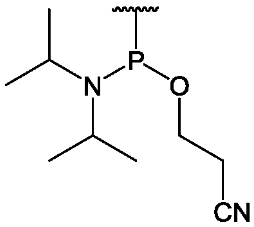.

In some specific embodiments of the present disclosure, in formula (IIa), $R_2$ is

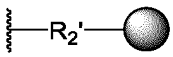,

wherein

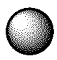

represents a solid phase support, and $R_2$' is

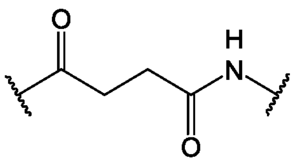.

That is, in formula (IIa), $R_2$ is

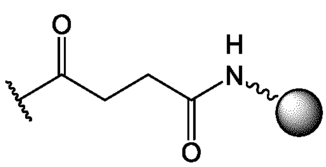.

Wherein, in the context of the present disclosure, a solid phase support represented by

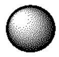

may also be represented by R-SC, and

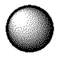

has the same meaning as R-SC. Exemplarily, the solid phase support may be, for example, controlled pore glass (CPG), and solid polymeric support (SPS, e.g., polystyrene (PS) resin).

In some specific embodiments of the present disclosure, each Z in formula (IIa) is hydroxyl.

In some embodiments of the present disclosure, each p in formula (IIa) is independently selected from the group consisting of 1 and 2.

In some specific embodiments of the present disclosure, each p in formula (IIa) is 1.

In some embodiments of the present disclosure, each q in formula (IIa) is independently selected from the group consisting of 1 and 2.

In some specific embodiments of the present disclosure, each q in formula (IIa) is 1.

In some specific embodiments of the present disclosure, in formula (IIa), each p is 1 and each q is 1.

In some specific embodiments of the present disclosure, each X in formula (IIa) is NH.

In some specific embodiments of the present disclosure, each $L_1$ in formula (IIa) is

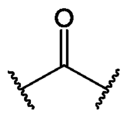.

In some specific embodiments of the present disclosure, each $R_3$ in formula (IIa) is H.

In some embodiments of the present disclosure, in formula (IIa), each $L_2$ is independently selected from the group consisting of $C_1$-$C_{10}$ alkylidene and

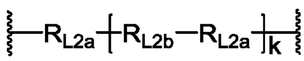,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.

In some specific embodiments of the present disclosure, each $R_{L2b}$ is -NH-C(O)-.

In some specific embodiments of the present disclosure, k is 1.

In some embodiments of the present disclosure, in formula (IIa), each $L_2$ is independently selected from the group consisting of:

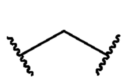, 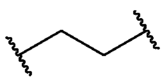, 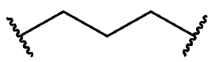, 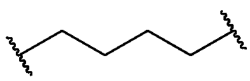, 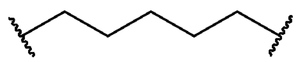,

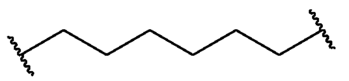, 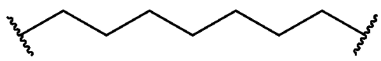, 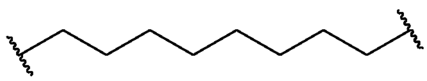,

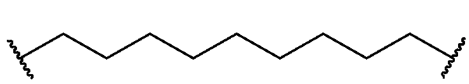, 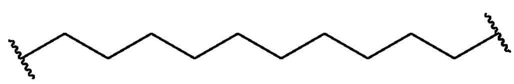,

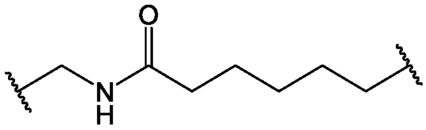,
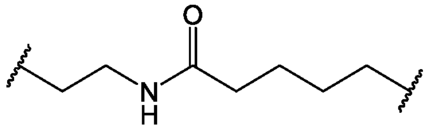,

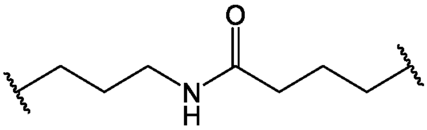,
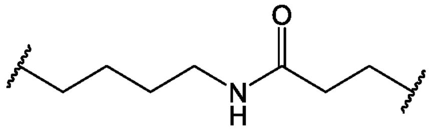

and

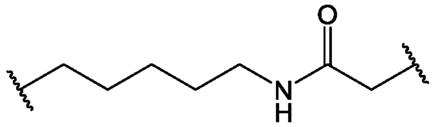.

In some specific embodiments of the present disclosure, in formula (IIa), each Y is O.

In some specific embodiments of the present disclosure, in formula (IIa), each $R_4$ is

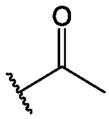.

In some embodiments of the present disclosure, the compound has a structure represented by formula (IIIa),

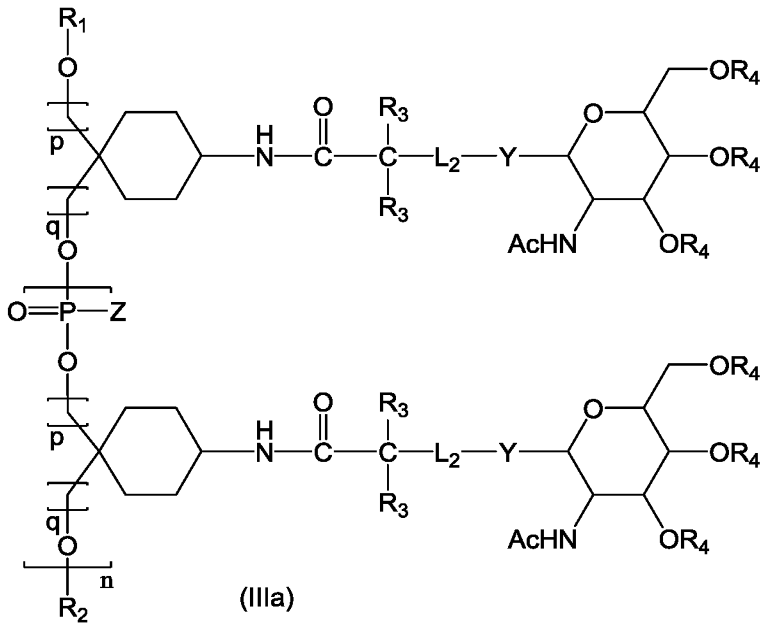

(IIIa)

in formula (IIIa), $R_1$ represents a hydroxyl protecting group,

$R_2$ is selected from the group consisting of:

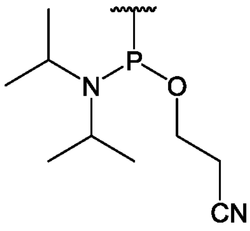, 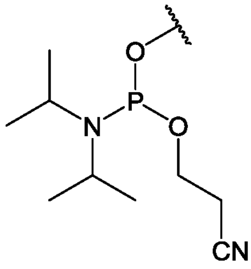,

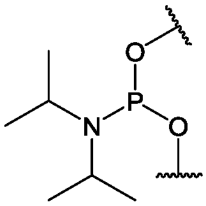, 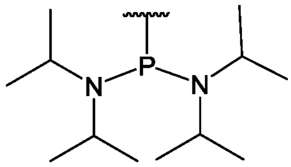 and 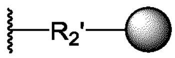,

wherein

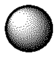

represents a solid phase support, and $R_2$' is a covalent linking group connected to the solid phase support,

n is selected from the group consisting of 0, 1, 2 and 3,

each Z is independently selected from the group consisting of hydroxyl and mercapto,

each p is independently selected from the group consisting of 1, 2 and 3,

each q is independently selected from the group consisting of 1, 2 and 3,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

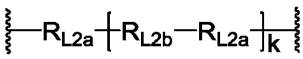,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10,

each Y is independently selected from the group consisting of NH, O and S, and

each $R_4$ is independently selected from the group consisting of:

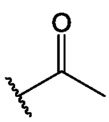, 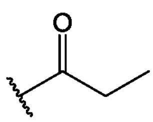,

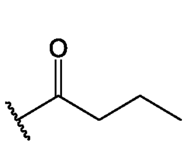, 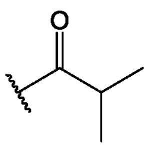, 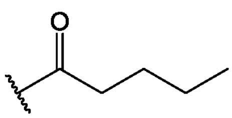, 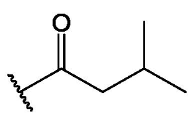, 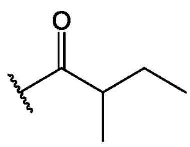,

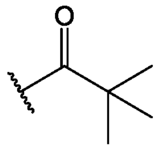,
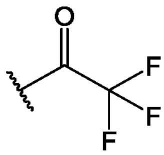 and
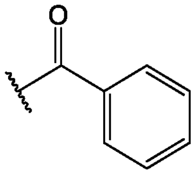.

Exemplarily, when n is 0, the compound has the following structure:

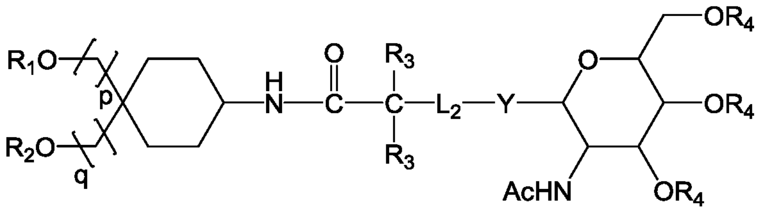.

In some embodiments of the present disclosure, in formula (IIIa), $R_1$ is selected from the group consisting of trityl (Tr), 4-methoxytrityl (MMTr), 4,4'-dimethoxytrityl (DMTr) and 4,4',4"-trimethoxytrityl (TMTr).

In some specific embodiments of the present disclosure, in formula (IIIa), $R_1$ is 4,4'-dimethoxytrityl (DMTr).

In some specific embodiments of the present disclosure, in formula (IIIa), $R_2$ is

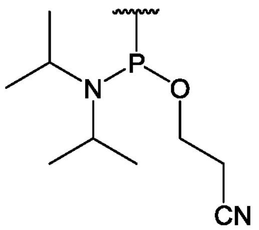.

In some specific embodiments of the present disclosure, in formula (IIIa), $R_2$ is

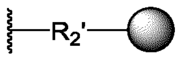,

wherein

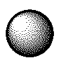

represents a solid phase support, and $R_2$' is

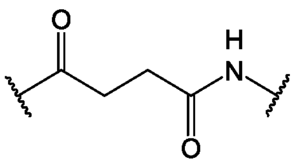.

That is, in formula (IIIa), $R_2$ is

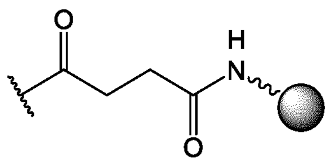.

In some specific embodiments of the present disclosure, each Z in formula (IIIa) is hydroxyl.

In some embodiments of the present disclosure, each p in formula (IIIa) is independently selected from the group consisting of 1 and 2.

In some specific embodiments of the present disclosure, each p in formula (IIIa) is 1.

In some embodiments of the present disclosure, each q in formula (IIIa) is independently selected from the group consisting of 1 and 2.

In some specific embodiments of the present disclosure, each q in formula (IIIa) is 1.

In some specific embodiments of the present disclosure, in formula (IIIa), each p is 1 and each q is 1.

In some specific embodiments of the present disclosure, each $R_3$ in formula (IIIa) is H.

In some embodiments of the present disclosure, in formula (IIIa), each $L_2$ is independently selected from the group consisting of $C_1$-$C_{10}$ alkylidene and

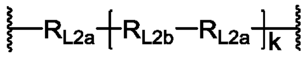,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.

In some specific embodiments of the present disclosure, each $R_{L2b}$ is -NH-C(O)-.

In some specific embodiments of the present disclosure, k is 1.

In some embodiments of the present disclosure, in formula (IIIa), each $L_2$ is independently selected from the group consisting of:

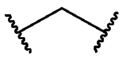, 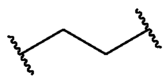, 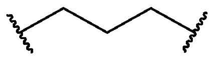, 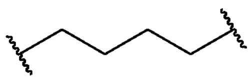, 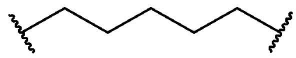,

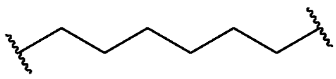, 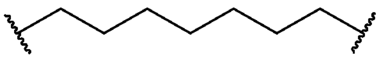, 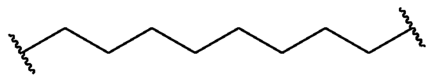,

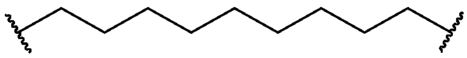, 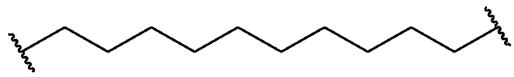,

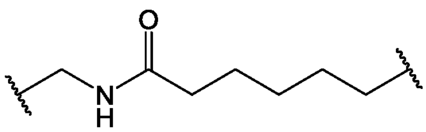, 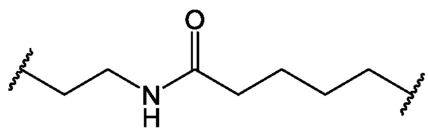,

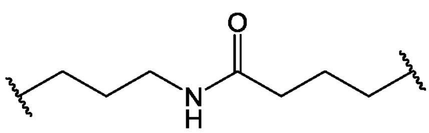, 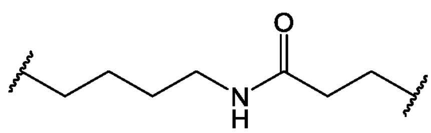

and

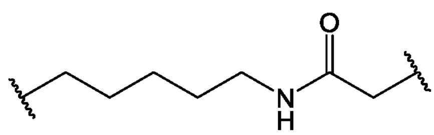.

In some specific embodiments of the present disclosure, in formula (IIIa), each Y is O.

In some specific embodiments of the present disclosure, in formula (IIIa), each $R_4$ is

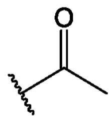.

In some embodiments of the present disclosure, the compound has a structure represented by formula (IVa),

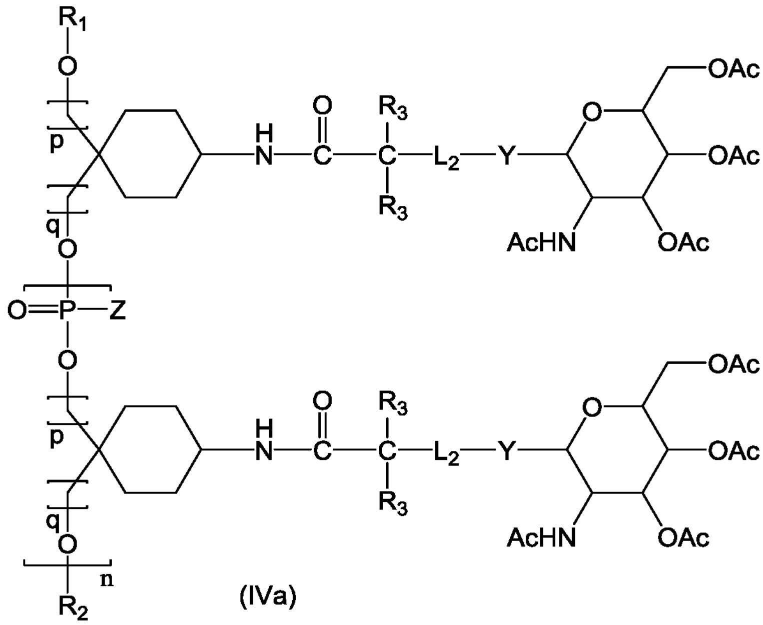

(IVa)

in formula (IVa), $R_1$ represents a hydroxyl protecting group,

$R_2$ is selected from the group consisting of:

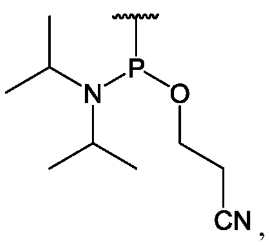, 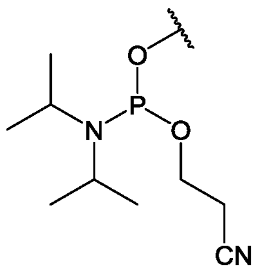,

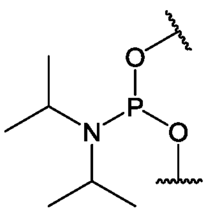, 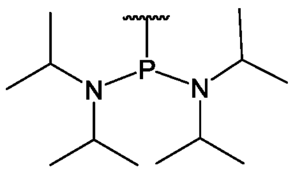 and 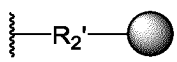,

wherein

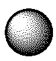

represents a solid phase support, and $R_2$' is a covalent linking group connected to the solid phase support,

n is selected from the group consisting of 0, 1, 2 and 3,

each Z is independently selected from the group consisting of hydroxyl and mercapto,

each p is independently selected from the group consisting of 1, 2 and 3,

each q is independently selected from the group consisting of 1, 2 and 3,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

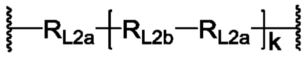

,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10, and

each Y is independently selected from the group consisting of NH, O and S.

Exemplarily, when n is 0, the compound has the following structure:

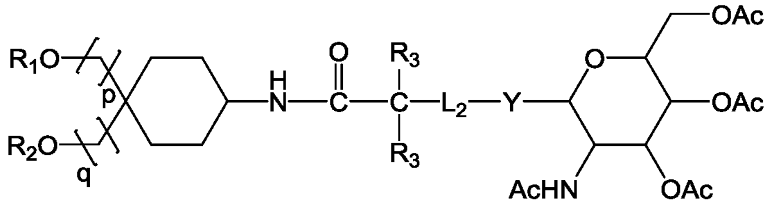

.

In some embodiments of the present disclosure, in formula (IVa), $R_1$ is selected from the group consisting of trityl (Tr), 4-methoxytrityl (MMTr), 4,4'-dimethoxytrityl (DMTr) and 4,4',4"-trimethoxytrityl (TMTr).

In some specific embodiments of the present disclosure, in formula (IVa), $R_1$ is 4,4'-dimethoxytrityl (DMTr).

In some specific embodiments of the present disclosure, in formula (IVa), $R_2$

is

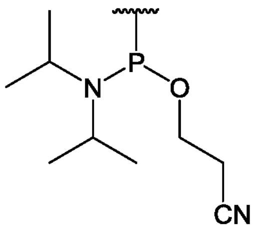

.

In some specific embodiments of the present disclosure, in formula (IVa), $R_2$ is

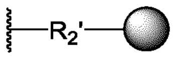

,

wherein

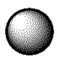

represents a solid phase support, and $R_2$' is

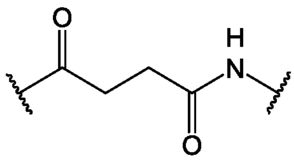

.

That is, in formula (IVa), $R_2$ is

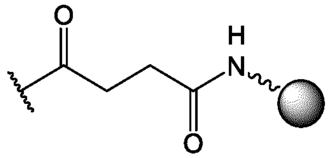

.

In some specific embodiments of the present disclosure, each Z in formula (IVa) is hydroxyl.

In some embodiments of the present disclosure, each p in formula (IVa) is independently selected from the group consisting of 1 and 2.

In some specific embodiments of the present disclosure, each p in formula (IVa) is 1.

In some embodiments of the present disclosure, each q in formula (IVa) is independently selected from the group consisting of 1 and 2.

In some specific embodiments of the present disclosure, each q in formula (IVa) is 1.

In some specific embodiments of the present disclosure, in formula (IVa), each p is 1 and each q is 1.

In some specific embodiments of the present disclosure, each $R_3$ in formula (IVa) is H.

In some embodiments of the present disclosure, in formula (IVa), each $L_2$ is independently selected from the group consisting of $C_1$-$C_{10}$ alkylidene and

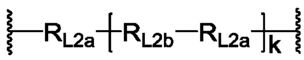

,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.

In some specific embodiments of the present disclosure, each $R_{L2b}$ is -NH-C(O)-.

In some specific embodiments of the present disclosure, k is 1.

In some embodiments of the present disclosure, in formula (IVa), each $L_2$ is independently selected from the group consisting of:

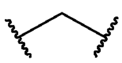 , 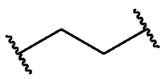 , 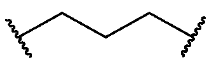 , 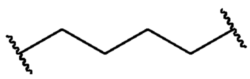 , 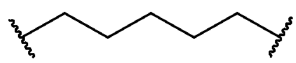 ,

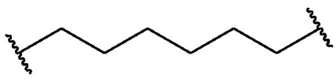 , 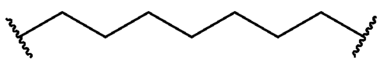 , 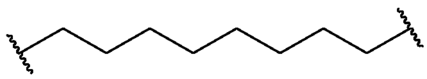 ,

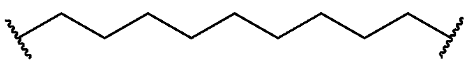 , 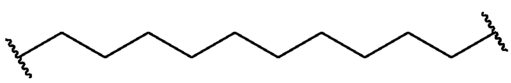 ,

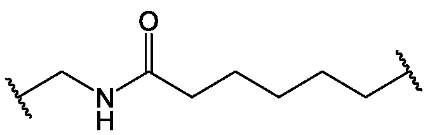 , 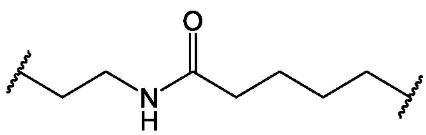 ,

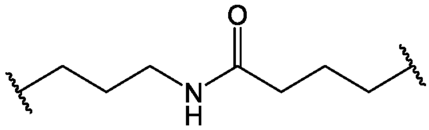

,

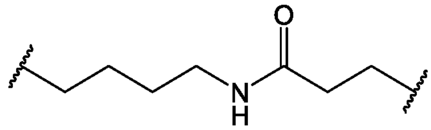

and

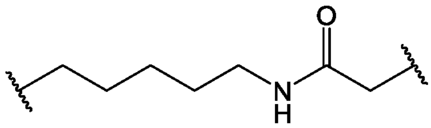

.

In some specific embodiments of the present disclosure, in formula (IVa), each Y is O.

In some specific embodiments of the present disclosure, the compound has a structure selected from the group consisting of:

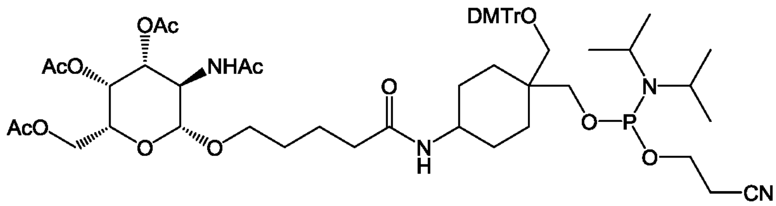

,

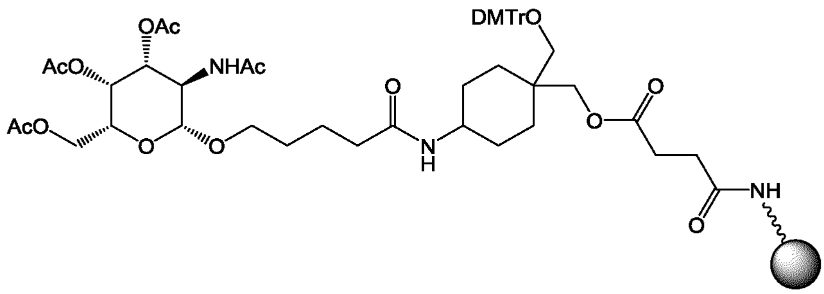

,

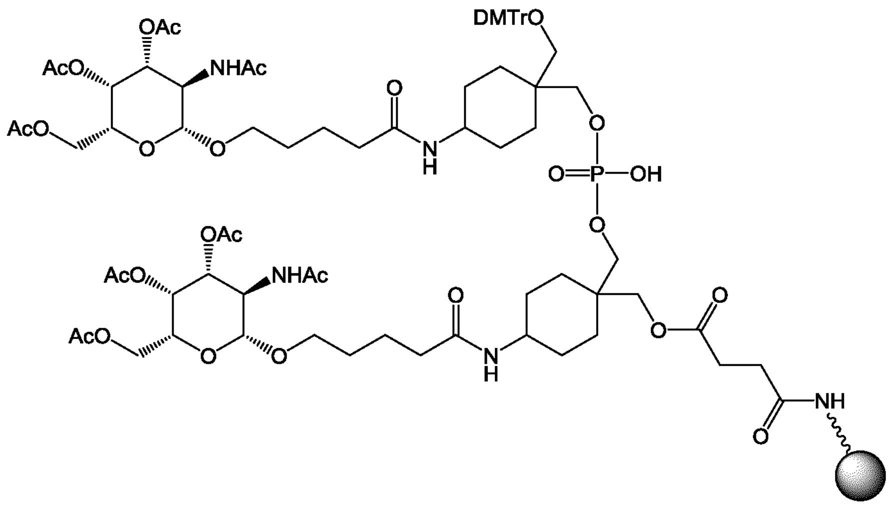

,

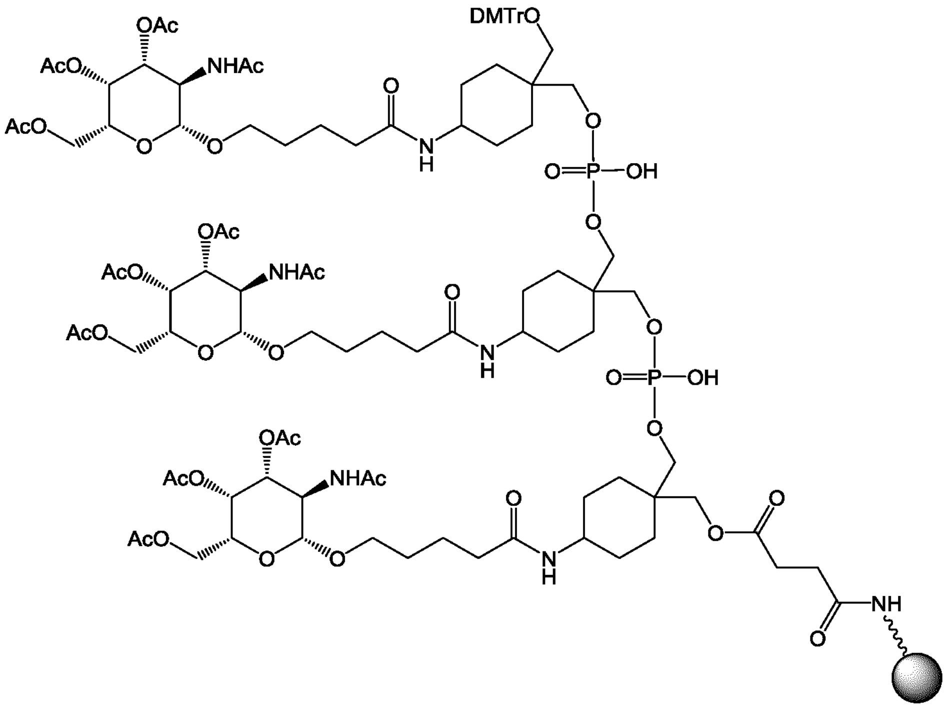

,

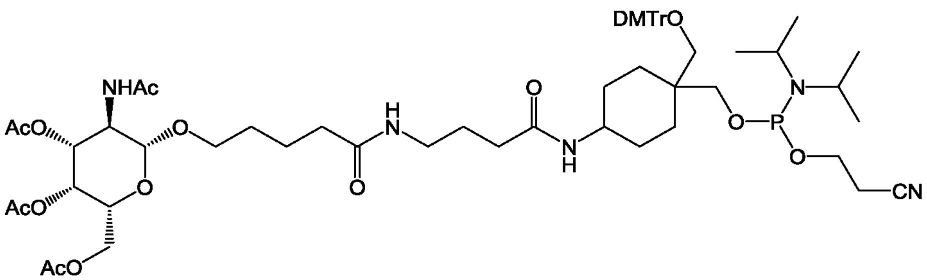

,

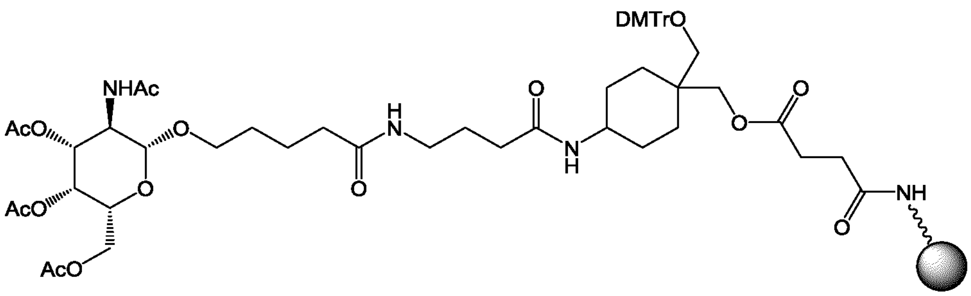

,

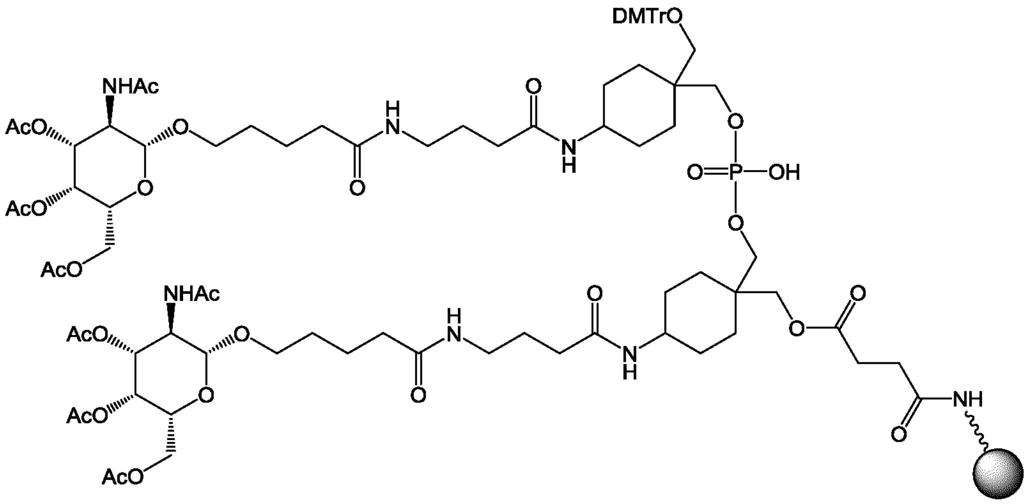

,

and

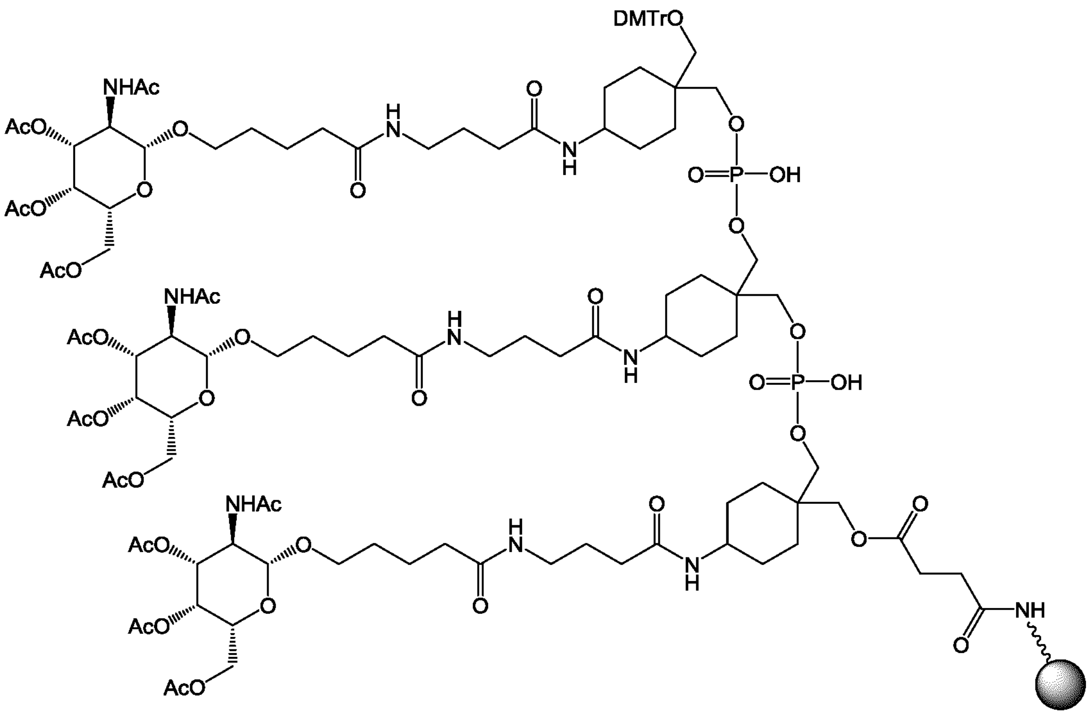

.

Wherein,

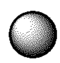

represents a solid phase support.

In a second aspect of the present disclosure, provided is a compound represented by formula (Ib), a stereoisomer, a pharmaceutically acceptable salt, or a prodrug thereof,

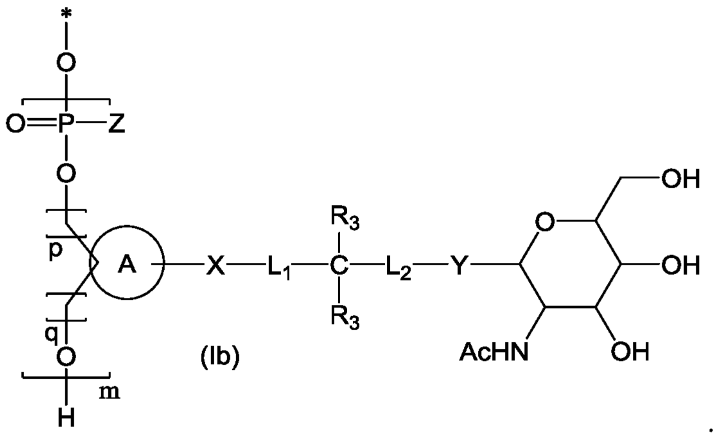

(Ib)

In formula (Ib), * represents a site of attachment for an active drug molecule,

m is selected from the group consisting of 1, 2, 3 and 4,

each Z is independently selected from the group consisting of hydroxyl and mercapto,

each p is independently selected from the group consisting of 1, 2 and 3,

each q is independently selected from the group consisting of 1, 2 and 3,

each A is independently an unsubstituted or substituted 4- to10-membered aliphatic ring,

each X is independently selected from the group consisting of NH, O and S,

each $L_1$ is independently selected from the group consisting of

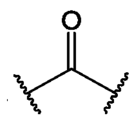, 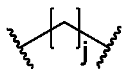

and

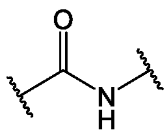,

wherein j is selected from the group consisting of 1, 2, 3, 4, 5, 6, 7, 8, 9 and 10,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

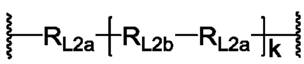,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10, and

each Y is independently selected from the group consisting of NH, O and S.

In some specific embodiments of the present disclosure, m is 3.

In some embodiments of the present disclosure, each Z in formula (Ib) is hydroxyl.

In some embodiments of the present disclosure, each p in formula (Ib) is independently selected from the group consisting of 1 and 2.

In some specific embodiments of the present disclosure, each p in formula (Ib) is 1.

In some embodiments of the present disclosure, each q in formula (Ib) is independently selected from the group consisting of 1 and 2.

In some specific embodiments of the present disclosure, each q in formula (Ib) is 1.

In some specific embodiments of the present disclosure, in formula (Ib), each p is 1 and each q is 1.

In some embodiments of the present disclosure, each A in formula (Ib) is independently selected from the group consisting of an unsubstituted or substituted 4- to 10-membered cycloalkane group and an unsubstituted or substituted 4- to 10-membered cycloolefine group.

In some embodiments of the present disclosure, each A in formula (Ib) is independently an unsubstituted or substituted 4- to 10-membered cycloalkane group.

In some embodiments of the present disclosure, each A in formula (Ib) is independently a 4- to 10-membered cycloalkane group, such as a monocyclic ring, a spirocyclic ring, and a bridged ring.

In some embodiments of the present disclosure, each A in formula (Ib) is independently selected from the group consisting of

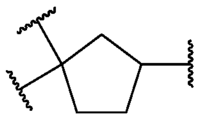, 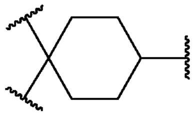, 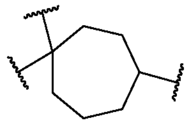,

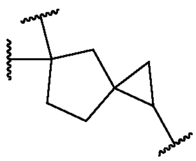 and 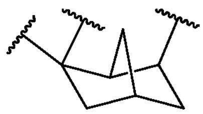.

In some specific embodiments of the present disclosure, each A in formula (Ib) is

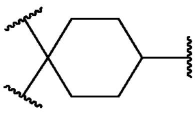.

In some specific embodiments of the present disclosure, each X in formula (Ib) is NH.

In some specific embodiments of the present disclosure, each $L_1$ in formula (Ib) is

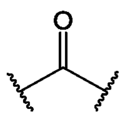.

In some specific embodiments of the present disclosure, each $R_3$ in formula (Ib) is H.

In some embodiments of the present disclosure, in formula (Ib), each $L_2$ is independently selected from the group consisting of $C_1$-$C_{10}$ alkylidene and

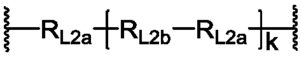,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.

In some specific embodiments of the present disclosure, each $R_{L2b}$ is -NH-C(O)-.

In some specific embodiments of the present disclosure, k is 1.

In some embodiments of the present disclosure, in formula (Ib), each $L_2$ is independently selected from the group

consisting of:

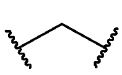, 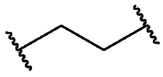, 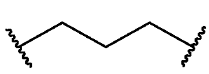, 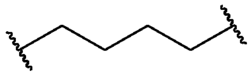, 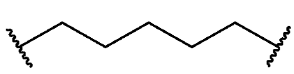,

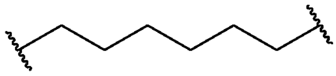, 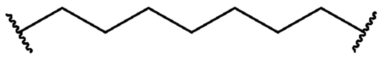, 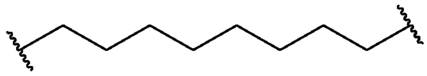,

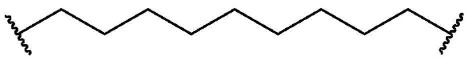, 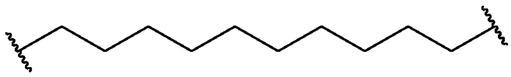,

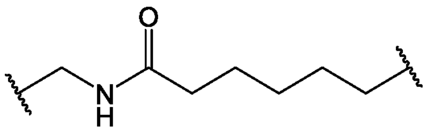, 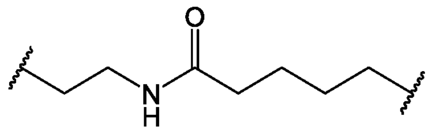,

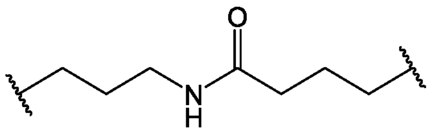, 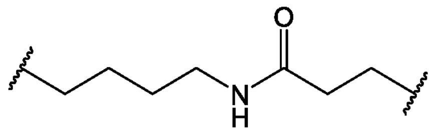

and

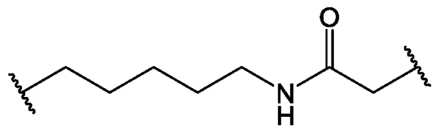.

In some specific embodiments of the present disclosure, in formula (Ib), each Y is O.

In some embodiments of the present disclosure, the active drug molecule is a small molecule drug (e.g., entecavir (ETV), small molecule chemical drugs of statins, febuxostat, allopurinol, etc.), an antibody, or an oligonucleotide.

In some specific embodiments of the present disclosure, the active drug molecule is an oligonucleotide.

In some embodiments of the present disclosure, the oligonucleotide is a single-stranded oligonucleotide or a double-stranded oligonucleotide.

In some embodiments of the present disclosure, the compound has a structure represented by formula (IIb),

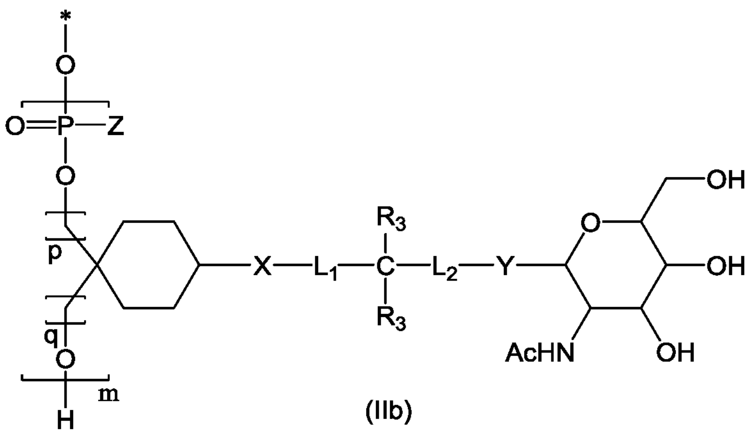

(IIb)

.

In formula (IIb), * represents a site of attachment for an active drug molecule,

m is selected from the group consisting of 1, 2, 3 and 4,

each Z is independently selected from the group consisting of hydroxyl and mercapto,

each p is independently selected from the group consisting of 1, 2 and 3,

each q is independently selected from the group consisting of 1, 2 and 3,

each X is independently selected from the group consisting of NH, O and S,

each $L_1$ is independently selected from the group consisting of

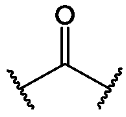 , 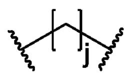

and

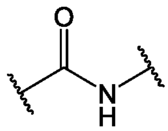 ,

wherein j is selected from the group consisting of 1, 2, 3, 4, 5, 6, 7, 8, 9 and 10,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

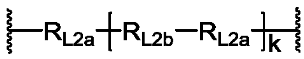 ,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10, and

each Y is independently selected from the group consisting of NH, O and S.

In some specific embodiments of the present disclosure, m in formula (IIb) is 3.
In some embodiments of the present disclosure, each Z in formula (IIb) is hydroxyl.
In some embodiments of the present disclosure, each p in formula (IIb) is independently selected from the group consisting of 1 and 2.
In some specific embodiments of the present disclosure, each p in formula (IIb) is 1.
In some embodiments of the present disclosure, each q in formula (IIb) is independently selected from the group consisting of 1 and 2.
In some specific embodiments of the present disclosure, each q in formula (IIb) is 1.
In some specific embodiments of the present disclosure, in formula (IIb), each p is 1 and each q is 1.
In some embodiments of the present disclosure, each X in formula (IIb) is NH.
In some specific embodiments of the present disclosure, each $L_1$ in formula (IIb) is

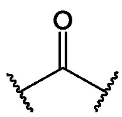 .

In some specific embodiments of the present disclosure, each $R_3$ in formula (IIb) is H.
In some embodiments of the present disclosure, in formula (IIb), each $L_2$ is independently selected from the group

consisting of $C_1$-$C_{10}$ alkylidene and

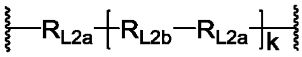,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.

In some specific embodiments of the present disclosure, each $R_{L2b}$ is -NH-C(O)-.

In some specific embodiments of the present disclosure, k is 1.

In some embodiments of the present disclosure, in formula (IIb), each $L_2$ is independently selected from the group consisting of:

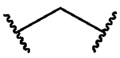, 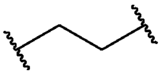, 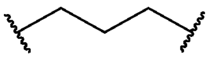, 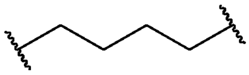, 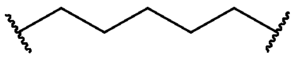,

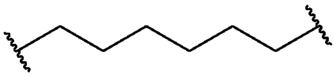, 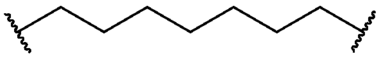, 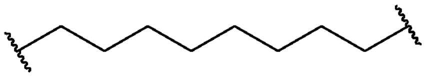,

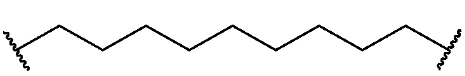, 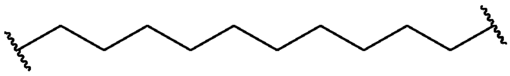,

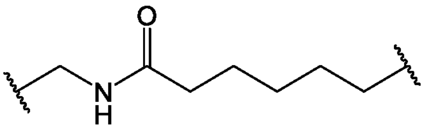, 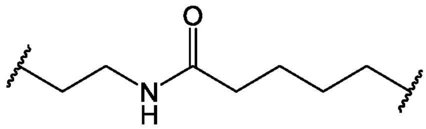,

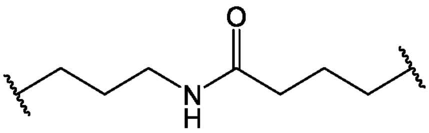, 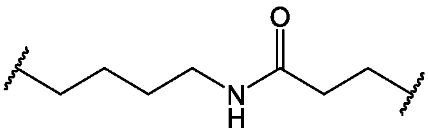,

and

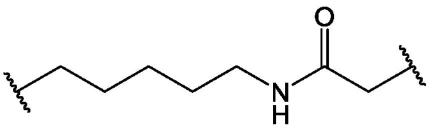.

In some specific embodiments of the present disclosure, in formula (IIb), each Y is O.

In some embodiments of the present disclosure, the compound has a structure represented by formula (IIIb),

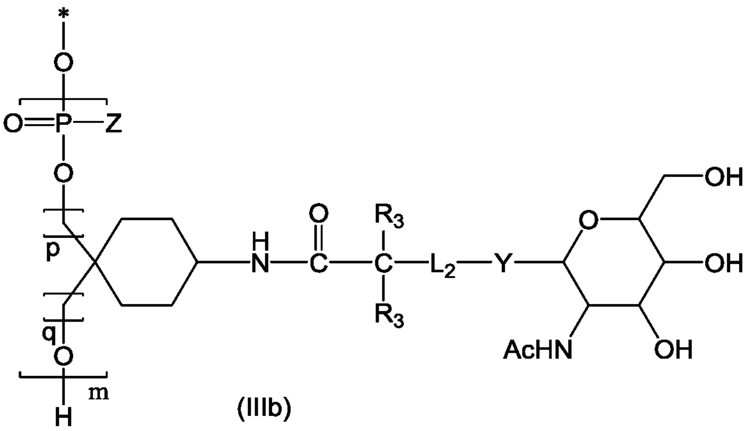

(IIIb)

.

In formula (IIIb), * represents a site of attachment for an active drug molecule,

m is selected from the group consisting of 1, 2, 3 and 4,

each Z is independently selected from the group consisting of hydroxyl and mercapto,

each p is independently selected from the group consisting of 1, 2 and 3,

each q is independently selected from the group consisting of 1, 2 and 3,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

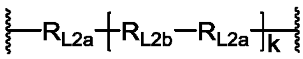

,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10, and

each Y is independently selected from the group consisting of NH, O and S.

In some specific embodiments of the present disclosure, m in formula (IIIb) is 3.
In some embodiments of the present disclosure, each Z in formula (IIIb) is hydroxyl.
In some embodiments of the present disclosure, each p in formula (IIIb) is independently selected from the group consisting of 1 and 2.
In some specific embodiments of the present disclosure, each p in formula (IIIb) is 1.
In some embodiments of the present disclosure, each q in formula (IIIb) is independently selected from the group consisting of 1 and 2.
In some specific embodiments of the present disclosure, each q in formula (IIIb) is 1.
In some specific embodiments of the present disclosure, in formula (IIIb), each p is 1 and each q is 1.
In some embodiments of the present disclosure, each $R_3$ in formula (IIIb) is H.
In some embodiments of the present disclosure, in formula (IIIb), each $L_2$ is independently selected from the group consisting of $C_1$-$C_{10}$ alkylidene and

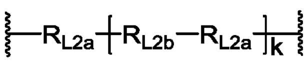

,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.
In some specific embodiments of the present disclosure, each $R_{L2b}$ is -NH-C(O)-.
In some specific embodiments of the present disclosure, k is 1.

In some embodiments of the present disclosure, in formula (IIIb), each $L_2$ is independently selected from the group consisting of:

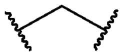
,
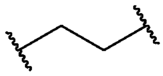
,
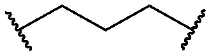
,
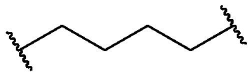
,
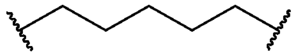
,
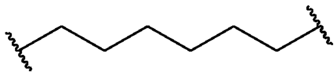
,
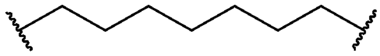
,
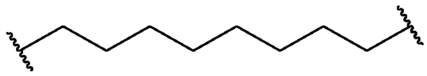
,
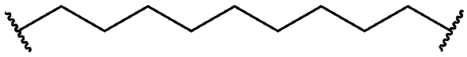
,
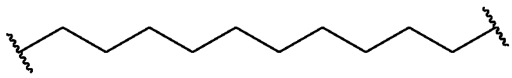
,
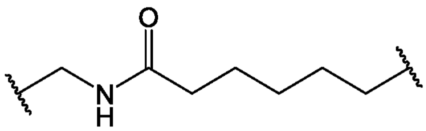
,
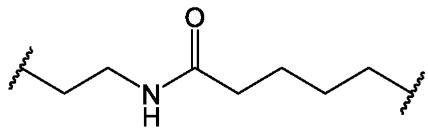
,
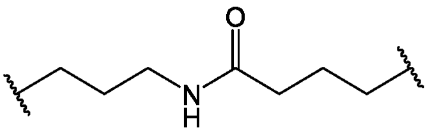
,
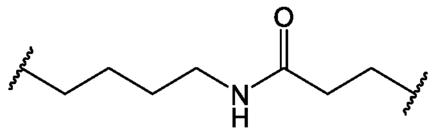

and

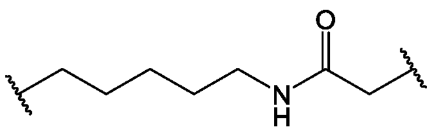
.

In some specific embodiments of the present disclosure, in formula (IIIb), each Y is O.

In some specific embodiments of the present disclosure, the compound has a structure selected from the group consisting of

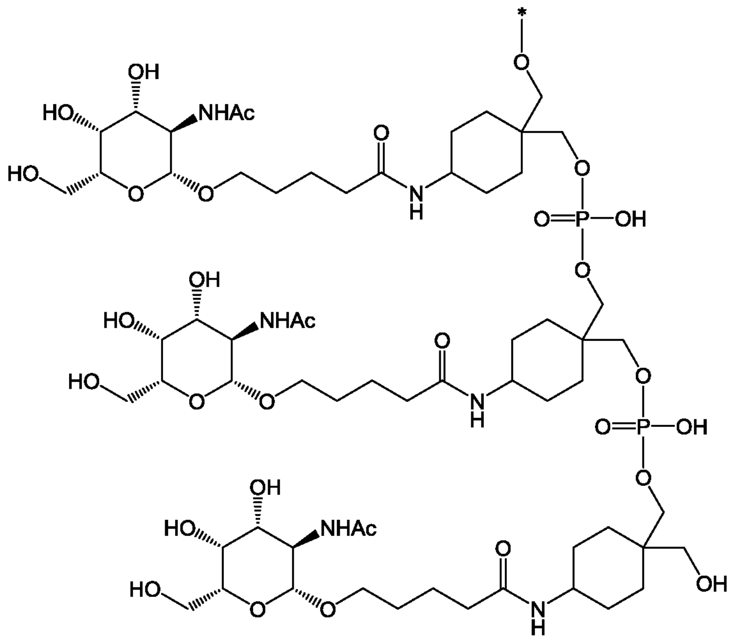

,

and

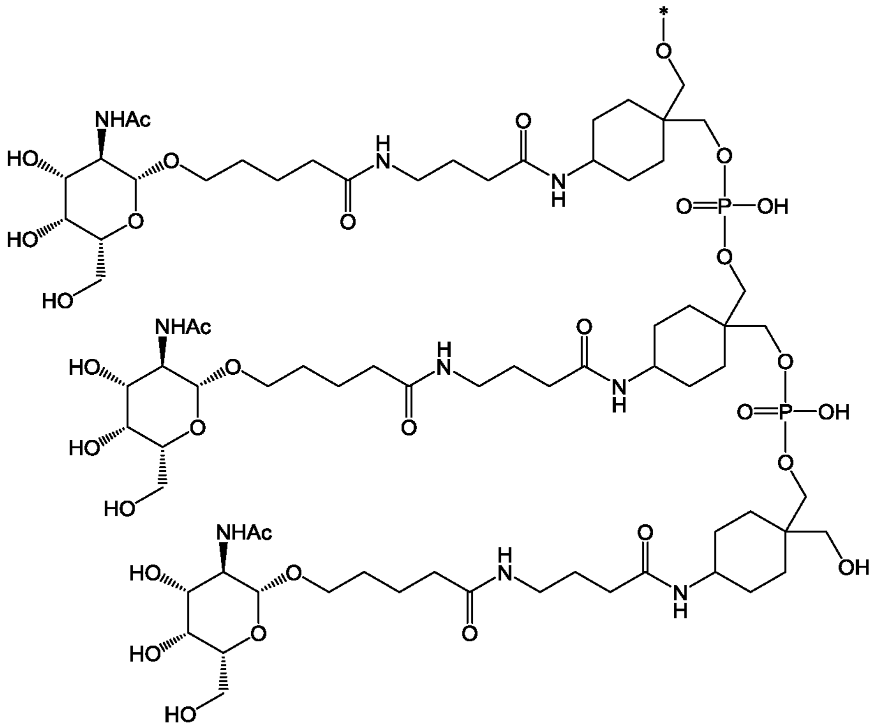

.

Wherein, * represents a site of attachment for an active drug molecule.

It is noted that the compound according to the second aspect of the present disclosure can be present in the form of a ligand.

In a third aspect of the present disclosure, provided is a conjugate represented by formula (Ic), a stereoisomer, a pharmaceutically acceptable salt, or a prodrug thereof,

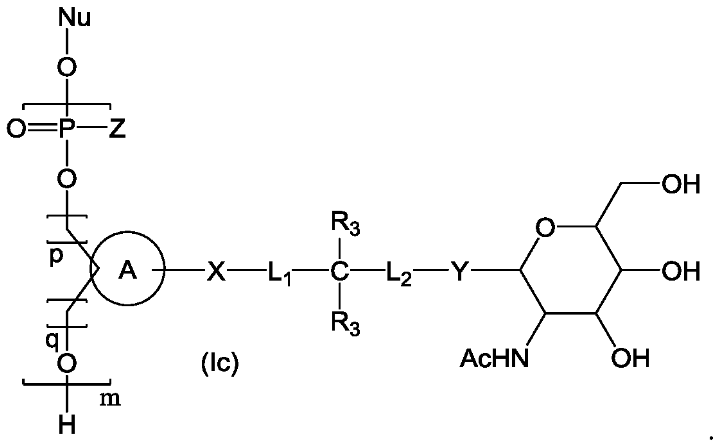

(Ic)

.

In formula (Ic), Nu represents an oligonucleotide,

m is selected from the group consisting of 1, 2, 3 and 4,

each Z is independently selected from the group consisting of hydroxyl and mercapto,

each p is independently selected from the group consisting of 1, 2 and 3,

each q is independently selected from the group consisting of 1, 2 and 3,

each A is independently an unsubstituted or substituted 4- to10-membered aliphatic ring,

each X is independently selected from the group consisting of NH, O and S,

each $L_1$ is independently selected from the group consisting of

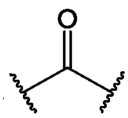

,

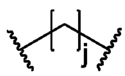

and

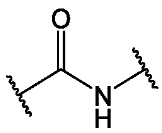

,

wherein j is selected from the group consisting of 1, 2, 3, 4, 5, 6, 7, 8, 9 and 10,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

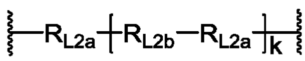

,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10, and

each Y is independently selected from the group consisting of NH, O and S.

In some embodiments of the present disclosure, the oligonucleotide is a single-stranded oligonucleotide or a double-stranded oligonucleotide.

In some embodiments of the present disclosure, the single-stranded oligonucleotide is selected from the group consisting of a modified or unmodified antisense oligonucleotide, a modified or unmodified nucleic acid aptamer, a modified or unmodified nuclease, a modified or unmodified deoxyribonuclease, a cyclic RNA, a sense strand of siRNA, and an antisense strand of siRNA.

In some embodiments of the present disclosure, the double-stranded oligonucleotide is selected from the group consisting of a modified or unmodified small interfering RNA, a modified or unmodified double-stranded RNA, a modified or unmodified microRNA, a modified or unmodified single guide RNA, a modified or unmodified small activating RNA, and a modified or unmodified short hairpin RNA.

In some specific embodiments of the present disclosure, in formula (Ic), Nu represents a small interfering RNA.

In some specific embodiments of the present disclosure, m in formula (Ic) is 3.

In some embodiments of the present disclosure, each Z in formula (Ic) is hydroxyl.

In some embodiments of the present disclosure, each p in formula (Ic) is independently selected from the group consisting of 1 and 2.

In some specific embodiments of the present disclosure, each p in formula (Ic) is 1.

In some embodiments of the present disclosure, each q in formula (Ic) is independently selected from the group consisting of 1 and 2.

In some specific embodiments of the present disclosure, each q in formula (Ic) is 1.

In some specific embodiments of the present disclosure, in formula (Ic), each p is 1 and each q is 1.

In some embodiments of the present disclosure, each A in formula (Ic) is independently selected from the group consisting of an unsubstituted or substituted 4- to 10-membered cycloalkane group and an unsubstituted or substituted 4- to 10-membered cycloolefine group.

In some embodiments of the present disclosure, each A in formula (Ic) is independently an unsubstituted or substituted 4- to 10-membered cycloalkane group.

In some embodiments of the present disclosure, each A in formula (Ic) is independently a 4- to 10-membered cycloalkane group, such as a monocyclic ring, a spirocyclic ring, and a bridged ring.

In some embodiments of the present disclosure, each A in formula (Ic) is independently selected from the group consisting of

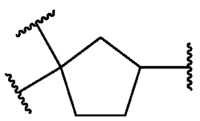,
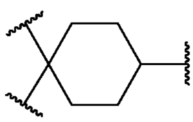,
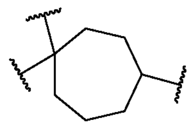,

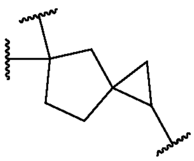 and 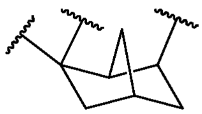.

In some specific embodiments of the present disclosure, each A in formula (Ic) is

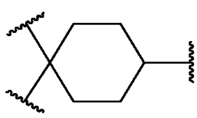.

In some specific embodiments of the present disclosure, each X in formula (Ic) is NH.

In some specific embodiments of the present disclosure, each $L_1$ in formula (Ic) is

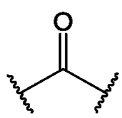.

In some specific embodiments of the present disclosure, each $R_3$ in formula (Ic) is H.

In some embodiments of the present disclosure, in formula (Ic), each $L_2$ is independently selected from the group consisting of $C_1$-$C_{10}$ alkylidene and

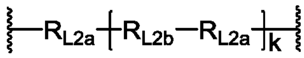,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.

In some specific embodiments of the present disclosure, each $R_{L2b}$ is -NH-C(O)-.

In some specific embodiments of the present disclosure, k is 1.

In some embodiments of the present disclosure, in formula (Ic), each $L_2$ is independently selected from the group consisting of:

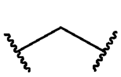, 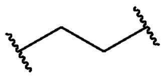, 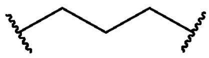, 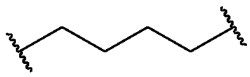, 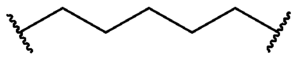,

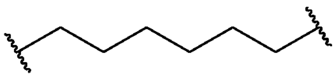, 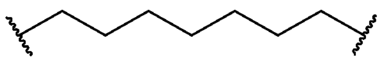, 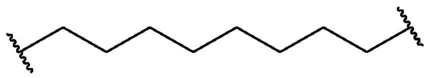,

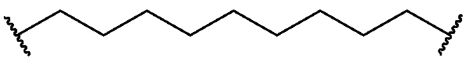, 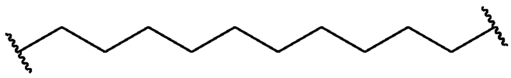,

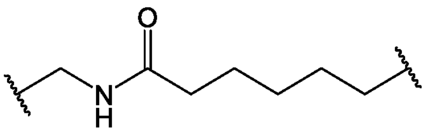, 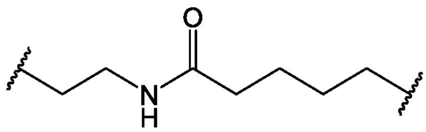,

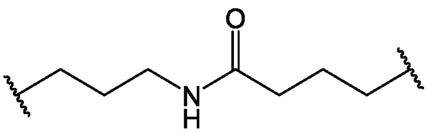, 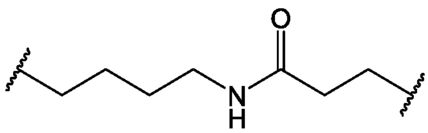

and

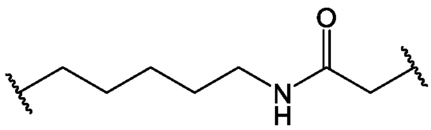.

In some specific embodiments of the present disclosure, in formula (Ic), each Y is O.

In some embodiments of the present disclosure, the conjugate has a structure represented by formula (IIc),

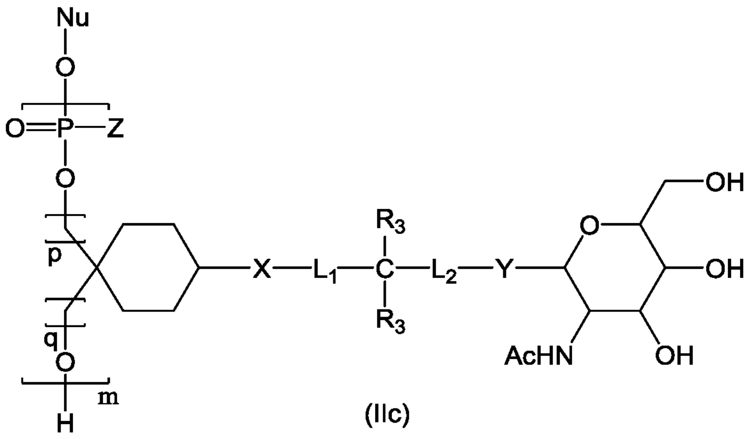

(IIc)

in formula (IIc), Nu represents an oligonucleotide,

m is selected from the group consisting of 1, 2, 3 and 4,

each Z is independently selected from the group consisting of hydroxyl and mercapto,

each p is independently selected from the group consisting of 1, 2 and 3,

each q is independently selected from the group consisting of 1, 2 and 3,

each X is independently selected from the group consisting of NH, O and S,

each $L_1$ is independently selected from the group consisting of

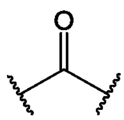

,

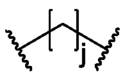

and

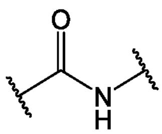

,

wherein j is selected from the group consisting of 1, 2, 3, 4, 5, 6, 7, 8, 9 and 10,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

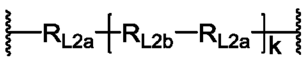

,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10, and

each Y is independently selected from the group consisting of NH, O and S.

In some specific embodiments of the present disclosure, in formula (IIc), Nu represents a small interfering RNA.

In some specific embodiments of the present disclosure, m in formula (IIc) is 3.

In some embodiments of the present disclosure, each Z in formula (IIc) is hydroxyl.

In some embodiments of the present disclosure, each p in formula (IIc) is independently selected from the group consisting of 1 and 2.

In some specific embodiments of the present disclosure, each p in formula (IIc) is 1.

In some embodiments of the present disclosure, each q in formula (IIc) is independently selected from the group consisting of 1 and 2.

In some specific embodiments of the present disclosure, each q in formula (IIc) is 1.

In some specific embodiments of the present disclosure, in formula (IIc), each p is 1 and each q is 1.

In some specific embodiments of the present disclosure, each $R_3$ in formula (IIc) is H.

In some embodiments of the present disclosure, in formula (IIc), each $L_2$ is independently selected from the group consisting of $C_1$-$C_{10}$ alkylidene and

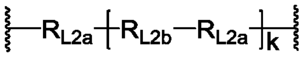,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.

In some specific embodiments of the present disclosure, each $R_{L2b}$ is -NH-C(O)-.

In some specific embodiments of the present disclosure, k is 1.

In some embodiments of the present disclosure, in formula (IIc), each $L_2$ is independently selected from the group consisting of

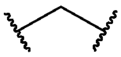, 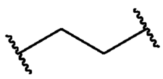, 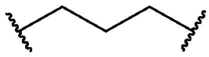, 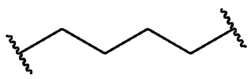, 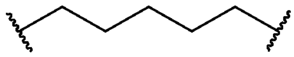,

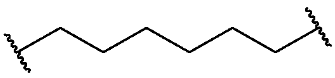, 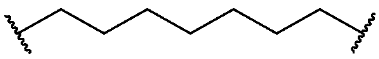, 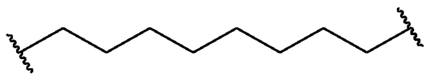,

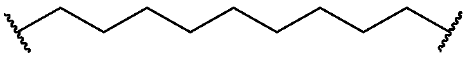, 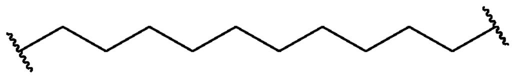,

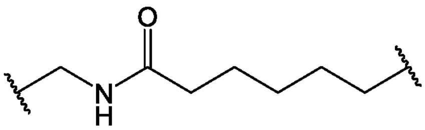. 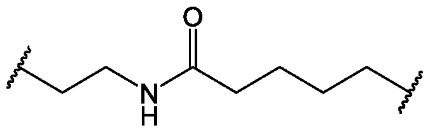.

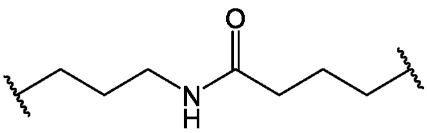, 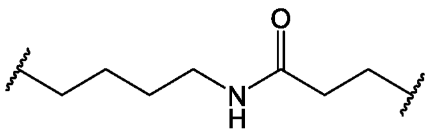

and

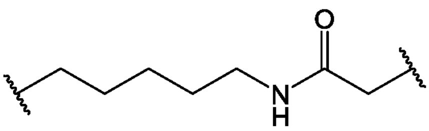.

In some specific embodiments of the present disclosure, in formula (IIc), each Y is O.

In some embodiments of the present disclosure, the conjugate has a structure represented by formula (IIIc),

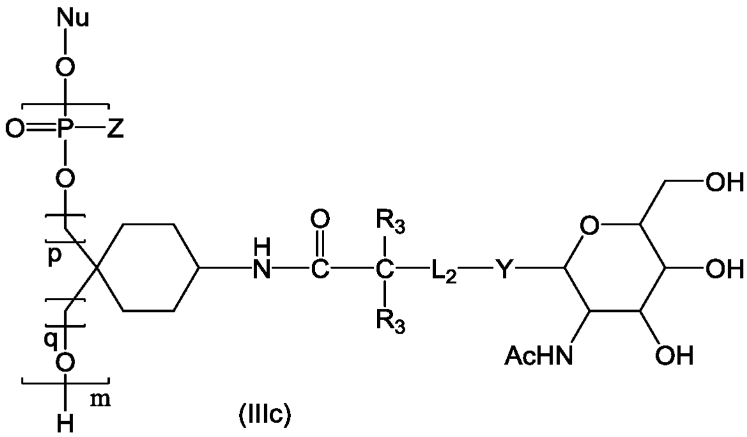

(IIIc)

in formula (IIIc), Nu represents an oligonucleotide,

m is selected from the group consisting of 1, 2, 3 and 4,

each Z is independently selected from the group consisting of hydroxyl and mercapto,

each p is independently selected from the group consisting of 1, 2 and 3,

each q is independently selected from the group consisting of 1, 2 and 3,

each $R_3$ is independently selected from the group consisting of H, $C_1$-$C_6$ alkyl, $C_1$-$C_6$ haloalkyl, and $C_1$-$C_6$ alkoxy,

each $L_2$ is independently selected from the group consisting of $C_1$-$C_{30}$ alkylidene and

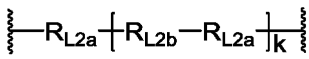,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10, and

each Y is independently selected from the group consisting of NH, O and S.

In some specific embodiments of the present disclosure, m in formula (IIIc) is 3.
In some embodiments of the present disclosure, each Z in formula (IIIc) is hydroxyl.
In some embodiments of the present disclosure, each p in formula (IIIc) is independently selected from the group consisting of 1 and 2.
In some specific embodiments of the present disclosure, each p in formula (IIIc) is 1.
In some embodiments of the present disclosure, each q in formula (IIIc) is independently selected from the group consisting of 1 and 2.
In some specific embodiments of the present disclosure, each q in formula (IIIc) is 1.
In some specific embodiments of the present disclosure, in formula (IIIc), each p is 1 and each q is 1.
In some embodiments of the present disclosure, each $R_3$ in formula (IIIc) is H.
In some embodiments of the present disclosure, in formula (IIIc), each $L_2$ is independently selected from the group consisting of $C_1$-$C_{10}$ alkylidene and

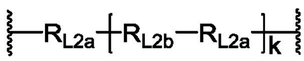,

wherein each $R_{L2a}$ is independently $C_1$-$C_5$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10.
In some specific embodiments of the present disclosure, each $R_{L2b}$ is -NH-C(O)-.
In some specific embodiments of the present disclosure, k is 1.

In some embodiments of the present disclosure, in formula (IIIc), each $L_2$ is independently selected from the group consisting of:

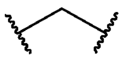, 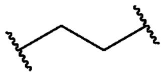, 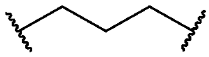, 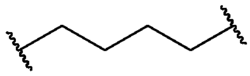, 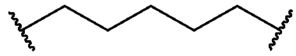,

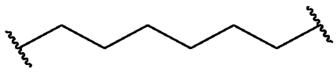, 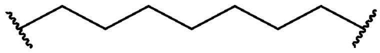, 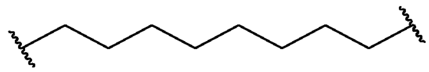,

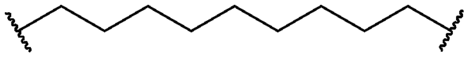, 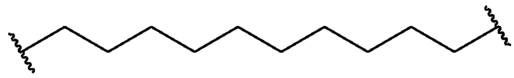,

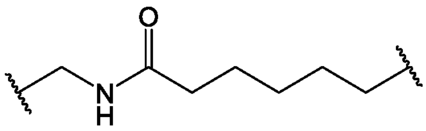, 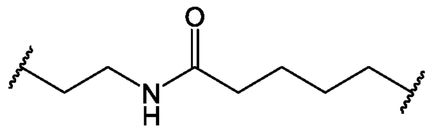,

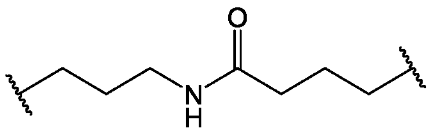, 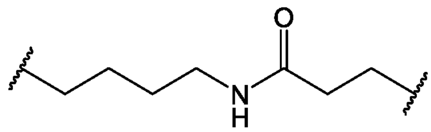

and

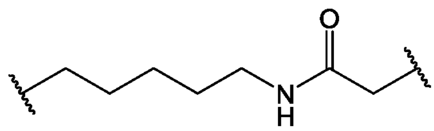.

In some specific embodiments of the present disclosure, in formula (IIIc), each Y is O.

In a fourth aspect of the present disclosure, provided is a composition comprising the conjugate of the third aspect.

In some embodiments of the present disclosure, the composition further comprises a pharmaceutically acceptable carrier or excipient.

In a fifth aspect of the present disclosure, provided is use of the compound of the first aspect, the ligand of the second aspect, the conjugate of the third aspect, or the composition of the fourth aspect in the manufacture of a medicament for preventing and/or treating a disease.

In some embodiments of the present disclosure, the disease is a liver-derived disease. Exemplarily, the liver-derived disease includes, but is not limited to, chronic non-alcoholic fatty liver disease, chronic alcoholic liver disease, autoimmune hepatitis, primary biliary cirrhosis, cirrhosis, primary liver cancer, hepatic encephalopathy, and viral hepatitis.

In a sixth aspect of the present disclosure, provided is a method for treating and/or preventing a disease, comprising administering to a subject a pharmaceutically acceptable amount of the conjugate of the third aspect or the composition of the fourth aspect.

In some embodiments of the present disclosure, the disease is a liver-derived disease. Exemplarily, the liver-derived disease includes, but is not limited to, chronic non-alcoholic fatty liver disease, chronic alcoholic liver disease, autoimmune hepatitis, primary biliary cirrhosis, cirrhosis, primary liver cancer, hepatic encephalopathy, and viral hepatitis.

In a seventh aspect of the present disclosure, provided is use of the compound of the first aspect, the ligand of the second aspect, the conjugate of the third aspect, or the composition of the fourth aspect in the manufacture of a medicament for reducing the expression or activity of a target gene.

In some embodiments of the present disclosure, the medicament is used to reduce the expression or activity of a target gene in a liver cell. Exemplarily, the target gene includes, but is not limited to, at least one of Apoa, ApoB, ApoC, ANGPTL3, PCSK9, SCD1, FVII, p53, C3, C4, C5, AGT, CFB, USP20, ASGR1, FTO, INHBE, HBV, and HCV.

In an eighth aspect of the present disclosure, provided is a method for reducing the expression or activity of a

target gene, comprising contacting a cell with the conjugate of the third aspect or the composition of the fourth aspect.

In some embodiments of the present disclosure, the cell is a liver cell. Exemplarily, the target gene includes, but is not limited to, at least one of Apoa, ApoB, ApoC, ANGPTL3, PCSK9, SCD1, FVII, p53, C3, C4, C5, AGT, CFB, USP20, ASGR1, FTO, INHBE, HBV, and HCV.

The additional aspects and advantages of the present disclosure will be further described in the following description, which will become obvious from the following description or be understood through the embodiments of the present disclosure.

## BRIEF DESCRIPTION OF DRAWINGS

The above and/or additional aspects and advantages of the present disclosure will become apparent and readily understood from the following description of examples in conjunction with the accompanying drawings, wherein:

FIG. 1 shows the relative expression level of a target gene in mice after administration of a trimeric CR01008 conjugate (RZ899015) with an L96 conjugate (RZ599001) according to examples of the present disclosure;

FIG. 2 shows a relative expression level of a target gene in mice after administration of a trimeric CR01008 conjugate (RZ899015), a trimeric CR01013 conjugate (RZ899026), a trimeric CR01014 conjugate (RZ899027), and an L96 conjuate (RZ599001) according to examples of the present disclosure; and

FIG. 3 shows the relative expression level of a target gene in mice after administration of a trimeric CR01013 conjugate (RZ897001), a trimeric CR01014 conjugate (RZ897002), and an L96 conjugate (RZ597002) according to examples of the present disclosure.

## DETAILED DESCRIPTION

Specific embodiments of the present disclosure are described in detail below. It should be understood that the specific embodiments described herein are only used to illustrate and explain the present disclosure, but not to limit the present disclosure.

Term Explanation

In order to more readily understand the present disclosure, a number of technical and scientific terms are specifically defined below. Unless defined otherwise, all other technical and scientific terms used herein have the meanings commonly understood by those of ordinary skills in the art to which the present disclosure belongs. The term "NH" refers to an imino with the structure formula of

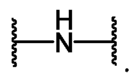

The term "CO" refers to a carbonyl with the structure formula:

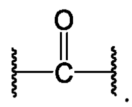

The structure formula of the term "trityl" is

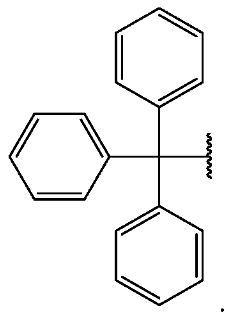

The structure formula of the term "4-methoxytrityl" is

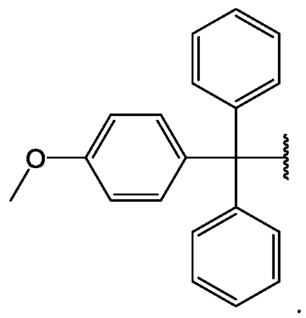

The structure formula of the term "4,4'-dimethoxytrityl" is

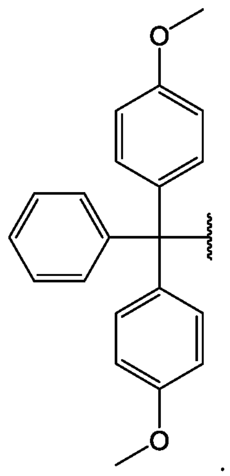

The structure formula of the term "4,4',4"-trimethoxytrityl" is

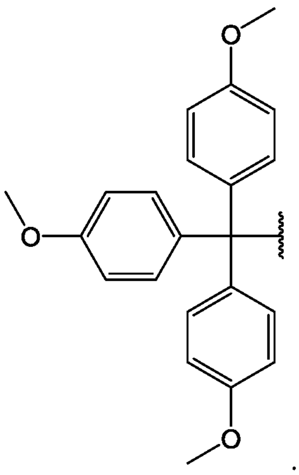

"〰" denotes the site of attachment between groups by a covalent bond.

In the structure formula of the compound or the ligand of the present disclosure, a bond "-" represents an unspecified configuration. If chiral isomerism exist in the chemical structure, the bond "——" may be "''''''''" or "◀", or contains both the configurations of "''''''''" and "◀". Although all of the above structure formula are drawn as certain isomeric forms for the sake of simplicity, the present disclosure may include all isomers, such as tautomers, rotamers, geometric isomers, diastereomers, racemates and enantiomers.

In the structure formula of the compound or the ligand described herein, a bond "═══" is not specified with a configuration; that is, it may be in a Z configuration or an E configuration, or contains both configurations of E and Z.

The following definitions used herein should be applied unless otherwise noted. For the purposes of the present disclosure, the chemical elements are consistent with the Periodic Table based on CAS number and Handbook of Chemistry and Physics (75th Edition), 1994. In addition, general principles of organic chemistry may refer to Organic Chemistry by Thomas Sorrell (University Science Books, Sausalito: 1999), and March s Advanced Organic Chemistry by Michael B. Smith and Jerry March (John Wiley & Sons, New York: 2007), the entire contents of which are incorporated herein by reference.

Unless stated otherwise or there is an obvious conflict in context, the articles "a," "an," and "the" used herein are intended to include "at least one" or "one or more". Therefore, these articles used herein refer to articles of one or more (i.e., at least one) objects. For example, "a component" means one or more components, that is, more than one component may be considered to be applied or used in an example of the embodiment.

The term "comprise" is an open-ended expression, and it includes the content specified in the present disclosure, but does not exclude other aspects.

The term "stereoisomer" refers to compounds having the same chemical structure, but different arrangement of atoms or groups in space. The stereoisomer includes enantiomer, diastereomer, conformational isomer (rotamer), geometric isomer (cis/trans) isomers, atropisomer, etc.

The term "chiral" refers to molecules which are non-superimposable on their mirror images, while the term "achiral" refers to molecules which are superimposable on their mirror images.

The "enantiomer" refers to two isomers of a compound which are non-superimposable but are mirror images of each other.

The "diastereomer" refers to stereoisomers which have two or more chiral centers and are not mirror images of each other. Diastereomers have different physical properties, such as melting points, boiling points, spectral properties, and reactivity. Diastereomer mixtures can be separated by high resolution analytical operations, for example, electrophoresis and chromatography, such as HPLC.

The stereochemical definitions and rules used in the present disclosure generally follow S.P.Parker, Ed., McGraw-Hill Dictionary of Chemical Terms(1984)McGraw-Hill Book Company, New York; and Eliel, E. and Wilen, S., "Stereochemistry of Organic Compounds", John Wiley & Sons, Inc., New York, 1994.

As used herein, the term "pharmaceutically acceptable salt" refers to organic and inorganic salts of the compounds of the present disclosure. Pharmaceutically acceptable salts are well known in the art, as documented in S.M. Berge et al., J. Pharmaceutical Sciences, 66, 1-19, 1977. Salts formed by pharmaceutically acceptable non-toxic acids include, but are not limited to, inorganic acid salts (such as hydrochloride, hydrobromide, phosphate, sulfate and perchlorate), and organic acid salts (such as acetate, oxalate, maleate, tartrate, citrate, succinate and malonate), which are formed by reaction with amino groups or by other methods documented in the book literature, for example, ion-exchange. Other pharmaceutically acceptable salts include adipate, alginate, ascorbate, aspartate, benzene sulphonate,

benzoate, bisulfate, borate, butyrate, camphorate, camphor sulfonate, cyclopentane propionate, digluconate, dodecyl sulfate, ethanesulfonate, formate, fumarate, glucoheptonate, glycerolphosphate, gluconate, hernisulfate, heptylate, hexanoate, hydriodate, 2-hydroxy-ethanesulfonate, lacturonate, lactate, laurate, lauryl sulfate, malate, malonate, mesylate, 2-naphthalenesulfonate, nicotinate, nitrate, oleate, palmitate, pamoate, pectate, persulfate, 3-phenpropionate, picrate, pivalate, propionate, stearate, thiocyanate, p-toluenesulfonate, undecanoate, valerate, and the like. Salts obtained from appropriate bases include salts of alkali metals, alkaline earth metals, ammonium, and N+($C_{1-4}$ alkyl)$_4$. The present disclosure also intends to conceive a quaternary ammonium salt formed by any compound containing a group of N. Water-soluble or oil-soluble or dispersed products can be obtained by quaternization. Alkali metals or alkaline earth metal salts include salts of sodium, lithium, potassium, calcium, magnesium, etc. Pharmaceutically acceptable salts further include appropriate, non-toxic ammonium/quaternary ammonium salts, and amine cations formed by counterions, such as halides, hydroxides, carboxylates, sulfates, phosphates, nitrates, $C_{1-8}$ sulfonates and aromatic sulfonates.

Most pharmaceutically acceptable salts are generally well known to those of ordinary skill in the art and include salts of active compounds which are prepared with relatively nontoxic acids or bases, depending on the particular substituent moieties found on the compounds described herein. When the compounds of the present disclosure contain relatively acidic functional groups, base addition salts can be obtained by contacting the neutral form of such compounds with a sufficient amount of the desired base, either in neat or in a suitable inert solvent or by ion exchange, whereby one basic counterion (base) in an ionic complex is substituted by another. Examples of pharmaceutically acceptable base addition salts include salts of sodium, potassium, calcium, ammonium, organic amino, or magnesium, or a similar salt.

The term "prodrug" used in the present disclosure represents a compound which can convert into the compound shown in formula (X) *in vivo.* Such conversion is affected by the hydrolysis of the prodrug in the blood or the enzymatic conversion of the prodrug to the parent structure in the blood or tissues. The prodrug compounds of the present disclosure can be an ester, and in the existing invention the esters that can be used as a prodrug include phenyl esters, aliphatic ($C_1$-$C_{24}$) esters, acyloxymethyl esters, carbonates, carbamates and amino acid esters. For example, a compound in the present disclosure contains a hydroxyl group, that is, the compound can be acylated to form a prodrug. Other forms of prodrugs include phosphate esters, such as those obtained by phosphorylation of the hydroxyl group of parent structure. A complete discussion of prodrugs can be found in the following literature: T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al, Prodrugs: Design and Clinical Applications, Nature Review Drug Discovery, 2008, 7, 255-270, and S. J. Hecker et al, Prodrugs of Phosphates and Phosphonates, Journal of Medicinal Chemistry, 2008, 51, 2328-2345.

In the context of the present disclosure, unless otherwise specified, "conjugating" refers to two or more chemical moieties each having specific function being covalently linked with each other. Correspondingly, "conjugate" refers to a compound formed by covalent linkage of individual chemical moieties. Further, "drug conjugate" represents a compound formed by covalently linking one or more chemical moieties having specific functions to active drugs. In the following, particularly in examples, the drug conjugate of the present disclosure is sometimes abbreviated as "conjugate". According to the context of the present disclosure, the drug conjugate should be understood as the general term of drug conjugates, or a drug conjugate represented by a certain structure formula.

The term "aliphatic ring" means a cyclic carbon skeleton structure, e.g., a monocyclic ring, a spirocyclic ring, and a bridged ring. The term "4- to 10-membered aliphatic ring" refers to a cyclic carbon skeleton structure with 4 to 10 carbon atoms.

The term "monocyclic ring" means a cycloalkyl group containing only one ring, known as monocyclic ring.

The term "spirocyclic ring" refers to a compound in which two rings share a common atom.

The term "bridged ring" refers to a structure formed where two or more rings share two non-adjacent ring atoms with each other.

The term "cycloalkane group" denotes a saturated monocyclic, bicyclic, or tricyclic system, monovalent or multivalent, containing 3-12 ring carbon atoms. In an embodiment, the cycloalkyl comprises 7-12 ring carbon atoms. In another embodiment, the cycloalkyl comprises 3-8 ring carbon atoms. In yet another embodiment, the cycloalkyl comprises 3-6 ring carbon atoms. The cycloalkyl may be independently unsubstituted or substituted with one or more of the substituents described in present disclosure.

The term "cycloolefine group" refers to a cyclized alkenyl. $C_{4-6}$ cycloalkenyl is intended to include $C_4$, $C_5$ and $C_6$ cycloalkenyl. Exemplary cycloalkenyl groups include, but are not limited to, cyclobutenyl, cyclopentenyl, and cyclohexenyl.

The term "cycloalkyne group" refers to a monocyclic alicyclic hydrocarbon containing one or more carbon-carbon triple bonds.

The term "alkyl" means a monovalent hydrocarbon group of 1-50 carbon atoms, 1-40 carbon atoms, 1-20 carbon atoms, 1-10 carbon atoms, 1-6 carbon atoms, or 1-4 carbon atoms in a saturated linear or branched chain, wherein the alkyl can be independently and optionally substituted by one or more substituents described in the present disclosure. Substituents include, but are not limited to, deuterium, amino, hydroxy, cyano, F, Cl, Br, I, mercapto, nitro, oxo (=O), etc.

Examples of alkyl include, but are not limited to, methyl (Me, $-CH_3$), ethyl (Et, $-CH_2CH_3$), n-propyl (n-Pr, $-CH_2CH_2CH_3$), isopropyl (i-Pr, $-CH(CH_3)_2$), n-butyl (n-Bu, $-CH_2CH_2CH_2CH_3$), isobutyl (iBu, $-CH_2CH(CH_3)_2$), sec-butyl (s-Bu, $-CH(CH_3)CH_2CH_3$), tert-butyl (t-Bu, $-C(CH_3)_3$), n-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), n-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$), n-heptyl, n-octyl, etc. The term "alkyl" and its prefix "alk" used herein both contain saturated carbon chains of linear and branched chains.

The term "alkylidene" used herein refers to a saturated divalent hydrocarbon group obtained by removing two hydrogen atoms from a linear or branched saturated hydrocarbon. Unless otherwise specified, alkylidene group contains 1-50 carbon atoms. In some embodiments, the alkylidene group contains 1-40 carbon atoms. In some other embodiments, the alkylidene group contains 1-20 carbon atoms. In yet other embodiments, the alkylidene group contains 1-10 carbon atoms. In still some embodiments, the alkylidene group contains 1-6 carbon atoms. Such examples include, but are not limited to: methylene ($-CH_2-$), ethylene ($-CH_2CH_2-$), isopropylidene ($-CH(CH_3)CH_2-$), and the like.

The term "alkenyl" refers to a straight or branched monovalent hydrocarbon group containing at least one carbon-carbon $sp^2$ double bond, which includes the localization of "cis" and "trans" or the localization of "E" and "Z". Wherein, the alkenyl group may optionally be substituted with one or more substituents described herein. In some embodiments, the alkenyl group comprises 2-50 carbon atoms. In some other embodiments, the alkenyl group comprises 3-50 carbon atoms. In yet other embodiments, the alkenyl group comprises 2-40 carbon atoms. In yet another embodiment, the alkenyl group comprises 2-20 carbon atoms. Examples of alkenyl groups include, but are not limited to: vinyl ($-CH=CH_2$), allyl ($-CH_2CH=CH_2$), and the like.

The term "alkenylene" means a straight or branched divalent hydrocarbon group containing at least one carbon-carbon $sp^2$ double bond.

The term "alkynyl" means a straight or branched monovalent hydrocarbon group containing 2-50 carbon atoms and at least one carbon-carbon sp triple bond. Wherein the alkynyl group may optionally be substituted with one or more of the substituents described herein. In an embodiment, the alkyne group comprises 3-12 carbon atoms. In another embodiment, the alkyne group comprises 2-6 carbon atoms. In yet another embodiment, the alkyne group comprises 2-4 carbon atoms. Examples of alkynyl groups include, but are not limited to: ethynyl ($-C\equiv CH$), propynyl ($-CH_2C\equiv CH$), 1-propynyl ($-C\equiv C-CH_3$), and the like.

The term "alkynylidene" denotes a straight or branched divalent alkynyl group containing at least one carbon-carbon sp triple bond.

The term "alkoxy" means an alkyl group connected to the rest of the molecule by an oxygen atom, wherein the alkyl group has the meaning as described in the present disclosure. Unless otherwise detailed, the alkoxy group contains 1-50 carbon atoms. In some embodiments, the alkoxy group contains 1-40 carbon atoms. In some other embodiments, the alkoxy group contains 1-20 carbon atoms. In yet other embodiments, the alkoxy group contains 1-10 carbon atoms. The alkoxy group may optionally be substituted with one or more of the substituents described herein. Examples of alkoxy groups include, but are not limited to: methoxy (MeO, $-OCH_3$), ethoxy (EtO, $-OCH_2CH_3$), 1-propoxy (n-PrO, n-propoxy, $-OCH_2CH_2CH_3$), 2-propoxy (i-PrO, i-propoxy, $-OCH(CH_3)_2$), 1-butoxy (n-BuO, n-butoxy, $-OCH_2CH_2CH_2CH_3$), 2-methyl-1-propoxy (i-BuO, i-butoxy, $-OCH_2CH(CH_3)_2$), 2-butoxy (s-BuO, s-butoxy, $-OCH(CH_3)CH_2CH_3$), 2-methyl-2-propoxy (t-BuO, t-butoxy, $-OC(CH_3)_3$), 1-pentyloxy (n-pentyloxy, $-OCH_2CH_2CH_2CH_2CH_3$), 2-pentyloxy ($-OCH(CH_3)CH_2CH_2CH_3$), 3-pentyloxy ($-OCH(CH_2CH_3)_2$), 2-methyl-2-butoxy ($-OC(CH_3)_2CH_2CH_3$), 3-methyl-2-butoxy ($-OCH(CH_3)CH(CH_3)_2$), 3-methyl-1-butoxy ($-OCH_2CH_2CH(CH_3)_2$), 2-methyl-1-butoxy ($-OCH_2CH(CH_3)CH_2CH_3$) and so on.

The term "alkylamino" includes "N-alkylamino" and "N,N-dialkylamino", wherein the amino group is independently substituted with one or two alkyl groups, respectively. The alkyl group has the meaning described herein. Some of these embodiments are that the alkylamino is a lower alkylamino group formed by one or two $C_1$-$C_6$ alkyl groups connected to a nitrogen atom. Some other embodiments are that the alkylamino is an alkylamino group formed by one or two lower $C_1$-$C_4$ alkyl groups connected to a nitrogen atom. Suitable alkylamino groups may be monoalkylamino or dialkylamino, examples of which include, but are not limited to, N-methylamino, N-ethylamino, N,N-dimethylamino, N,N-diethylamino, and the like.

The terms "haloalkyl", "haloalkenyl", "haloalkoxy", or "haloalkylamino" denotes that alkyl, alkenyl, alkoxy, or alkylamino group is substituted with one or more halogen atoms, wherein the alkyl, alkenyl, alkoxy, or alkylamino group has a meaning as described herein. Such examples include, but are not limited to: trifluoromethyl, 2,2,3,3-tetrafluoropropyl, trifluoromethoxy, trifluoromethylamino, and the like.

The terms "cycloalkyl" and "heterocycloalkyl" by themselves or in combination with other terms, mean cyclic forms of "alkyl" and "heteroalkyl," respectively. Additionally, for heterocycloalkyl, a heteroatom can occupy the position at

which the heterocycle is connected to the rest of the molecule. Examples of cycloalkyl include, but are not limited to: cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothiophen-2-yl, tetrahydrothiophen-3-yl, 1-piperazinyl, 2-piperazinyl, and the like. A "cycloalkenyl" and a"heterocycloalkenyl" refer to divalent derivatives of a cycloalkyl and heterocycloalkyl, respectively.

Like those described herein, the compounds of the present disclosure may optionally be substituted with one or more substituents, such as the compounds of the general formula above, or the particular examples in the embodiments, subclasses, and classes of compounds encompassed by the present disclosure.

Generally, the term "substituted" means that one or more hydrogen atoms in a given structure are replaced by specific substituents. Unless otherwise indicated, a substituted group may have a substituent at each substitutable position of the group. When more than one position in a given structure formula can be substituted with one or more substituents selected from specific groups, the substituents at each substitutable position may be the same or different.

The term "unsubstituted" means that the specified group has no substituents.

The term "optionally substituted with..." can be used interchangeably with the term "unsubstituted or substituted with...", i.e., the structure is unsubstituted or substituted with one or more of the substituents described herein. The substituents described herein include, but are not limited to: D, F, Cl, Br, I, $N_3$, CN, $NO_2$, OH, SH, $NH_2$, alkyl, haloalkyl, haloalkoxy, haloalkylamino, alkenyl, alkynyl, alkoxy, alkylamino, cycloalkyl, heterocyclyl, aryl, heteroaryl and the like.

Further, it is noted that, unless otherwise expressly indicated, the expressions "each... is independently", "...each independently is" and "...independently is" are interchangeable and should be understood in a broad sense, that is, it can mean that the specific options expressed between the same symbols in different groups do not affect each other, or it can mean the specific options expressed between the same symbols in the same group do not affect each other. Taking $R_3$ as an example, the specific options of $R_3$ between the structure formula "$C_1$-$C_{50}$ alkylidene optionally substituted with $R_3$" and the structure formula "-C(O)-NH-$C_{1}$-$_{50}$ alkylidene optionally substituted with $R_3$" do not affect each other.

The term "small interfering RNA (siRNA)" refers to a double-stranded RNA that comprises a sense and an antisense strands, and each strand is 17 to 30 nucleotides in length. The siRNA mediates the targeted cleavage of RNA transcripts via RNA-induced silencing complex (RISC) pathway through the formation of a RISC. Specifically, the siRNA directs the specific degradation of mRNA sequences through the known process of RNA interference (RNAi), which inhibits the translation of mRNA into amino acids and thereby transformation into proteins.

In the context of the present disclosure, the term "antisense strand (or called as guide strand)" includes a region that is substantially complementary to a target sequence. The term "sense strand (or passenger strand)" refers to an iRNA strand that comprises a region that is substantially complementary to the antisense strand. The term "substantially complementary" means fully complementary or at least partially complementary. For example, the antisense strand is fully complementary or at least partially complementary to a target sequence. In the case of partial complementarity, the mismatch may be present within the internal or terminal region of the molecule, wherein the most tolerated mismatch is present within the terminal region, e.g., within the 5, 4, 3 or 2 nucleotides at the 5'- and/or 3'-end of the iRNA.

It is noted that the antisense strand being "at least partially substantially complementary" to the mRNA means that the antisense strand has a polynucleotide that is substantially complementary to a contiguous portion of the mRNA of interest.

In the context of the present disclosure, an "oligonucleotide" refers to deoxyribonucleic acid (DNA) or ribonucleic acid (RNA), generally consisting of 10 to 50 nucleotides. Oligonucleotides can regulate gene expression through a number of processes including ribonucleic acid interference, ribonuclease-mediated target degradation, splicing regulation, non-coding RNA inhibition, gene activation, and programmed genome editing.

In the context of the present disclosure, "antisense oligonucleotides (ASOs)" are single-stranded oligonucleotide molecules, typically consisting of 10 to 50 nucleotides. After entering the cell, ASO binds to its complementary target mRNA by the base complementary pairing under the action of ribonuclease H1 to inhibit the expression of the target gene.

In the context of the present disclosure, unless otherwise specified, uppercase letters C, G, U, and A represent the base composition of the nucleotides, lowercase m represents that the nucleotide adjacent to the left side of the letter m is a 2'-methoxy modified nucleotide, lowercase f represents that the nucleotide adjacent to the left side of the letter f is a 2'-fluoro modified nucleotide, and lowercase s represents that the two nucleotides adjacent to both sides of the letter s are linked by a phosphorothioate linkage.

In the context of the present disclosure, the term "pharmaceutically acceptable carrier" includes any solvent, dispersion medium, coating material, surfactant, antioxidant, preservative (such as an antibacterial agent and an antifungal agent), isotonic agent, salt, drug stabilizer, binder, excipient, dispersing agent, lubricant, sweetener, flavoring agent, coloring agent, or combination thereof. These carriers are known to a person skilled in the art (as described in Remington's Pharmaceutical Sciences, 18th Ed. Mack Printing Company, 1990, pp. 1289-1329). Except for any conventional carrier that is incompatible with the active ingredient, the uses thereof in treatment or in a pharmaceutical composition are encompassed.

In the context of the present disclosure, the term "pharmaceutically acceptable excipient" may include any solvent, solid excipient, diluent, other liquid excipient, etc., which is suitable for the specific target dosage form. Except to the extent that any conventional excipient is incompatible with the siRNA of the present disclosure, for example, producing any adverse biological effects or harmful interactions with any other components of a pharmaceutically acceptable composition, the uses thereof are also contemplated by the present disclosure.

The term "subject", as used herein, refers to any animal, e.g., mammal or marsupial. The subject of the present disclosure includes, but is not limited to, human, non-human primate (e.g., monkeys), mouse, pig, horse, donkey, cow, sheep and any kind of poultry.

In the context of the present disclosure, "treatment" "alleviation" or "amelioration" may be used interchangeably herein. These terms refer to an approach for obtaining beneficial or desirable results, including but not limited to therapeutic benefit. "Therapeutic benefit" means eradication or amelioration of potential disorder to be treated. Also, a therapeutic benefit is achieved by eradicating or ameliorating one or more of physiological symptoms associated with the potential disorder such that an improvement is observed in the subject, notwithstanding that the subject may still be afflicted with the potential disorder.

In the context of the present disclosure, "prevention" and "prevent" are used interchangeably. These terms refer to an approach for obtaining beneficial or desirable results, including but not limited to prophylactic benefit. In order to obtain "prophylactic benefit", a conjugate, a RNAi reagent, or a composition may be administered to a subject at risk of developing a particular disease, or to a subject reported with one or more physical symptoms of a disease, even though the diagnosis of this disease may not have been made yet.

## General Experiments

The present disclosure is described in detail below in conjunction with examples. The reagents and culture media used in the following examples are commercially available, and the nucleic acid electrophoresis, real-time PCR, and other operations applied were carried out according to the protocols known to those skills in the art.

Unless otherwise specified, the siRNA sequences used herein were synthesized by Suzhou Biosyntech Co., Ltd, and the PCR primers used herein were synthesized by Tsingke (Beijing) Biotechnology Co., Ltd, respectively. The C57BL/6J mice as experimental animals used herein were purchased from SPF (Beijing) Biotechnology Co., Ltd.

Unless otherwise specified, the meanings of the base compositions and modifications described in various examples of the present disclosure are as follows: uppercase letters A, U, G, C and T represent the base composition of the nucleotides, lowercase m represents that the nucleotide represented by its upstream letter is a methoxy-modified nucleotide, lowercase f represents that the nucleotide represented by its upstream letter is a fluorine-modified nucleotide, and lowercase s represents that the nucleotides represented by two letters adjacent to both sides of the letter s are linked by a phosphorothioate linkage.

Unless otherwise indicated, data for activity assay *in vivo* are reported as X$\pm$SD, and the experimental data were plotted and analyzed using GraphPad prism 8.0 software.

The reagent ratios described in the examples of the present disclosure are calculated as volume-to-volume (v/v), unless otherwise noted.

The reagents used in the examples of the present disclosure are detailed in Tables 1-1 and 1-2:

Table 1-1

| Reagent | Abbreviation | CAS number | Molecular weight |
|---|---|---|---|
| Maleic anhydride | MA | 108-31-6 | 98.06 |
| N- (methoxymethyl)-N-(trimethylsilylmethyl) benzylamine | - | 93102-05-7 | 237.41 |
| Trifluoroacetic acid | TFA | 76-05-1 | 114.02 |
| Lithium aluminum hydride ($LiAlH_4$) | - | 16853-85-3 | 37.95 |
| Wet palladium carbon (10 mass% loading) | Pd/C | - | - |
| Palladium carbon hydroxide (10 mass% loading) | $Pd(OH)_2/C$ | - | - |
| N,N-diisopropylethylamine | DIEA | 7087-68-5 | 129.24 |
| N,N-dimethylformamide | DMF | 68-12-2 | 73.09 |
| Benzotriazole-N,N,N',N'- tetramethylurea hexafluorophosphate | HBTU | 94790-37-1 | 379.25 |

(continued)

| Reagent | Abbreviation | CAS number | Molecular weight |
|---|---|---|---|
| 2-(7-azobenzotriazole)-N,N,N',N'- tetramethylurea hexafluorophosphate | HATU | 148893-10-1 | 380.24 |
| 4,4'-Bismethoxy triphenylmethyl chloride/4,4'-dimethoxy triphenyl chloromethane | DMTrCl | 40615-36-9 | 338.83 |
| Pyridine | Py | 110-86-1 | 79.1 |
| Bis(diisopropylamino) (2-cyanoethoxy) phosphine | - | 102691-36-1 | 301.41 |
| 4,5-Dicyanoimidazole | DCI | 1122-28-7 | 118.1 |
| Succinic anhydride | SA | 108-30-5 | 100.07 |
| 4-Dimethylaminopyridine | DMAP | 1122-58-3 | 122.17 |
| Triethylamine | TEA | 121-44-8 | 101.19 |
| Aminoalkyl-CPG | - | - | - |
| 4M hydrochloric acid in 1,4-dioxane solution | - | - | - |
| Trans-4-(Boc-amino)cyclohexyl formaldehyde | - | 181308-57-6 | 227.3 |
| N-benzyloxycarbonyl-4-aminobutyric acid | - | 5105-78-2 | 237.25 |
| 5-[[(2R,3R,4R,5R,6R)-3-acetamido -4,5-diacetoxy -6-(acetoxymethyl)-2-tetrahydropyranyl]oxy] valeric acid | - | - | 447.43 |

The reagents shown in Table 1-1 were purchased from Beijing Ouhe Technology Co., LTD.

Table 1-2

| Reagent | Manufacturer |
|---|---|
| 1×PBS | M&C GENE TECHNOLOGY(BEIJING) LTD. |
| RNA Extraction Kit (HanWei) | Zhejiang Hanwei Technology Co., Ltd. |
| Reverse Transcription System | Promega Corporation |
| SYBR Select Master Mix | ABI |
| RNA Later | Thermo Fisher Scientific |

The instruments used in examples of the present disclosure are detailed in Table 2:

Table 2

| Instrument | Manufacturer |
|---|---|
| Fully automatic nucleic acid extraction equipment | Zhejiang Hanwei Technology Co., Ltd. |
| High-speed freezing centrifuge | Eppendorf |
| NANODROP OneC | Thermo Fisher Scientific |
| Gradient PCR Amplifier | Eppendorf |
| Fluorescence quantitative PCR instrument | ABI StepOne Plus |
| Gel imager | Shanghai Tanon Science & Technology Co.,Ltd. |
| Electrophoresis equipment | Beijing Liuyi Biotechnology Co., Ltd. |
| Automatic Tissue Homogenizer-Tissuelyser II | Shanghai Jingxin Industrial Development Co., Ltd |

Example 1 Preparation of compounds

(1) Synthesis of compound CR01008

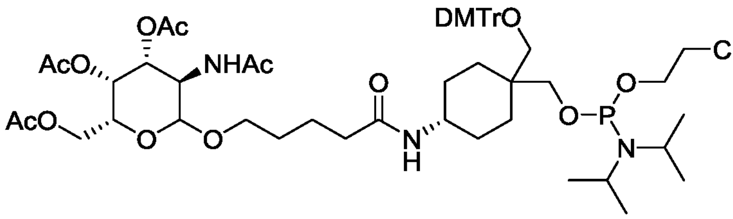

**CR01008**

Synthetic route of compound CR01008

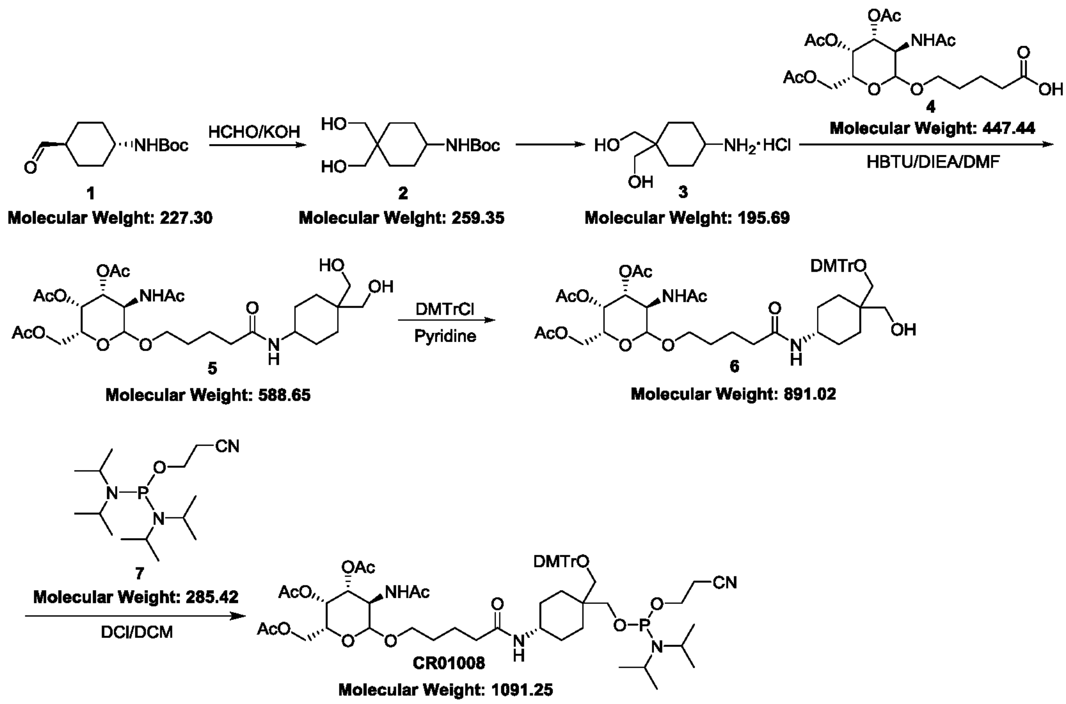


(1-1) Synthesis of compound 2

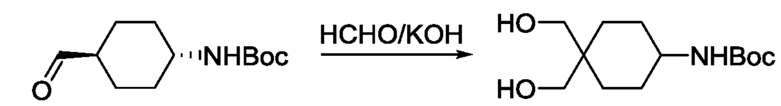


**1** **2**

**Molecular Weight: 227.30** **Molecular Weight: 259.35**

Compound 1 (trans-4-(Boc-amino)cyclohexylformaldehyde, 10.0 g, 1.0 eq) and aqueous formaldehyde solution (8.9 g, 37 mass%, 2.4 eq) were dissolved in 33 ml of methanol, and 13 ml of an aqueous KOH solution at a concentration of 45.3 mass% was added dropwise. After the dropwise addition was completed, the reaction system was stirred for 30 min at 25°C, heated up to 60°C, refluxed and reacted at 60°C for 2 h. After the reaction was completed, the reaction solution was cooled to room temperature and evaporated to dryness under reduced pressure to obtain a crude product as white solid. The crude product was slurried with a small amount of water, and then filtered to obtain compound 2 as a white solid (9 g,

78.9% yield). MS-ESI (*m/z*) = 260 [M + H]$^+$.

(1-2) Synthesis of compound 3

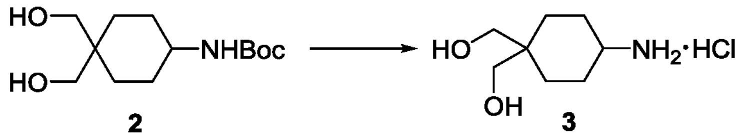

2 3

**Molecular Weight: 259.35** **Molecular Weight: 195.69**

The compound 2 (9 g, 1 eq) prepared according to step (1-1) was dissolved in 70 ml of 1,4-dioxane, added with a solution of hydrogen chloride in 1,4-dioxane (45 ml, 4 M) and stirred at 25°C for 1 h of reaction. After the reaction was completed, the reaction solution was evaporated to dryness under reduced pressure to obtain compound 3 as a white solid (6.8 g, 100% yield).

(1-3) Synthesis of compound 5

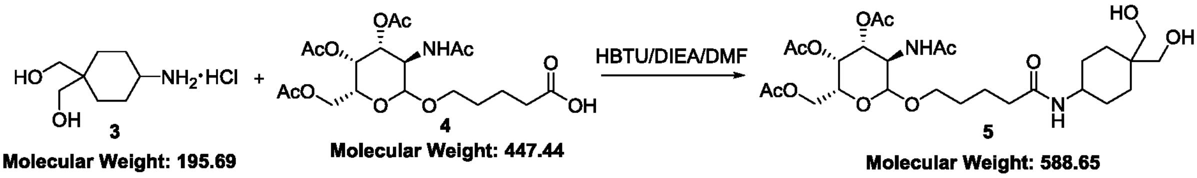


3 4 5

**Molecular Weight: 195.69** **Molecular Weight: 447.44** **Molecular Weight: 588.65**

The compound 3 (1.8 g, 2.0 eq) prepared according to step (1-2), compound 4 (5-[[(2R,3R,4R,5R,6R)-3-acetamido-4,5-diacetoxy-6-(acetyloxymethyl)-2-tetrahydropyranyl]oxy ]pentanoic acid, 2.1 g, 1.0 eq), and DIEA (N,N-diisopropylethylamine, 3.5 g, 6.0 eq) were dissolved in 15 ml of DMF, added with HBTU (1.9 g, 1.1 eq), and stirred at 25°C under $N_2$ atmosphere for 3 h of reaction. After the reaction was completed, the reaction solution was evaporated to dryness under reduced pressure and purified by reverse phase chromatography (22 vol% aqueous solution of acetonitrile) to obtain compound 5 as a white solid (1.78 g, 64.4% yield). MS-ESI (*m/z*) = 589[M + H]$^+$.

(1-4) Synthesis of compound 6

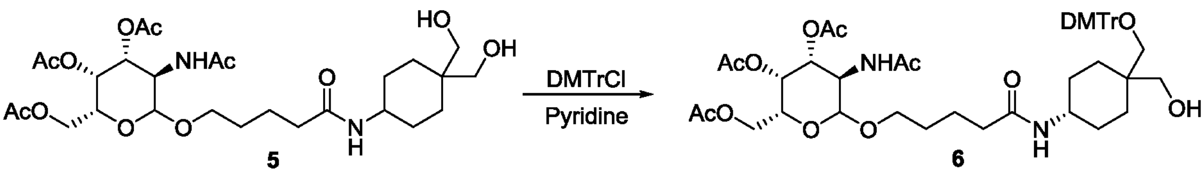


5 6

**Molecular Weight: 588.65** **Molecular Weight: 891.02**

The compound 5 (1.54 g, 1.0 eq) prepared according to step (1-3) was dissolved in 15 ml of pyridine, the reaction system was cooled down to 0°C in an ice-water bath and DMTrCl (4,4'-dimethoxytriphenylmethyl chloride, 1.32 g, 1.5 eq) was added at 0°C to perform 3 h of reaction at 25°C. The reaction was quenched by adding 15 ml of methanol to the reaction solution. After the reaction was completed, the reaction solution was evaporated to dryness under reduced pressure and purified by reverse phase chromatography (60 vol% aqueous solution of acetonitrile) to obtain compound 6 as a yellow solid (1 g, 42.7% yield). MS-ESI (*m/z*) = 891 [M + H]$^+$.

(1-5) Synthesis of compound CR01008

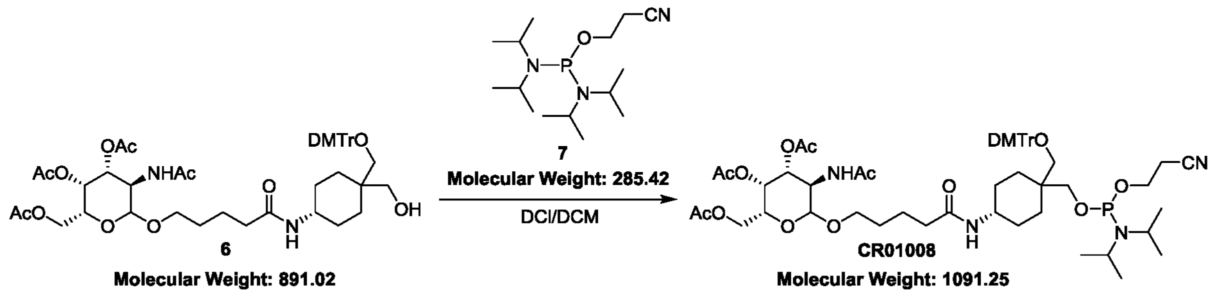

6
Molecular Weight: 891.02
7
CR01008
Molecular Weight: 1091.25

The compound 6 (1.08 g, 1.0 eq) prepared according to step (1-4) was dissolved in 20 ml of anhydrous dichloromethane, DCI (115 mg, 0.8 eq) and compound 7 (bis(diisopropylamino)(2-cyanoethoxy)phosphine, 732 mg, 2.1 eq) were added separately, nitrogen replacement was performed three times, and the reaction system was stirred at 25°C for 2 h of reaction. After the reaction was completed, the reaction solution was added with 20 ml of saturated sodium bicarbonate aqueous solution and extracted with 20 ml of dichloromethane 3 times (3×20 ml). The organic phases were combined, evaporated to dryness under reduced pressure, purified by reverse phase chromatography (72 vol% aqueous solution of acetonitrile) and then dried under vacuum for 12 h to obtain the compound CR01008 as a white powder (1 g, 76.0% yield). MS-ESI (m/z) = 1091 $[M + Na]^+$.

$^1H$ NMR (400 MHz, DMSO-$d_6$) δ 1.05 (d, *J* = 6.7 Hz, 6H).1.14 (d, *J* = 6.7 Hz, 6H), 1.37 - 1.17 (m, 5H), 1.60 - 1.40 (m, 6H),1.68 - 1.62 (m, 1H),1.80 (s, 3H),1.80 (s, 3H),1.92 (s, 3H), 2.02 (s, 5H),2.13 (s, 3H),2.71 (t, *J* = 5.9 Hz, 2H), 2.79 (d, *J* = 8.4 Hz, 1H), 2.87 (d, *J* = 8.4 Hz, 1H),3.36 (s, 1H), 3.58 - 3.39 (m, 3H), 3.69 - 3.60 (m, 2H), 3.75 (s, 7H), 3.90 (dt, *J* = 11.2, 8.8 Hz, 1H), 4.05 (s, 3H),4.51 (d, *J* = 8.4 Hz, 1H), 4.99 (dd, *J* = 11.3, 3.4 Hz, 1H), 5.24 (d, *J* = 3.4 Hz, 1H), 5.78 (s, 1H), 6.93 - 6.87 (m, 4H),7.35 - 7.21 (m, 7H), 7.44 - 7.37 (m, 2H), 7.66 (d, *J* = 7.8 Hz, 1H), 7.84 (d, *J* = 9.2 Hz, 1H).

(2) Synthesis of compound CR01008Z

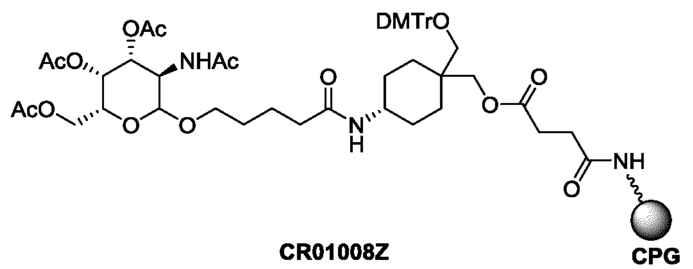
CR01008Z

Compound CR01008Z was prepared by connecting the compound 6 used in the synthesis of the compound CR01008 to the solid phase support CPG.

Synthetic route of compound CR01008Z:

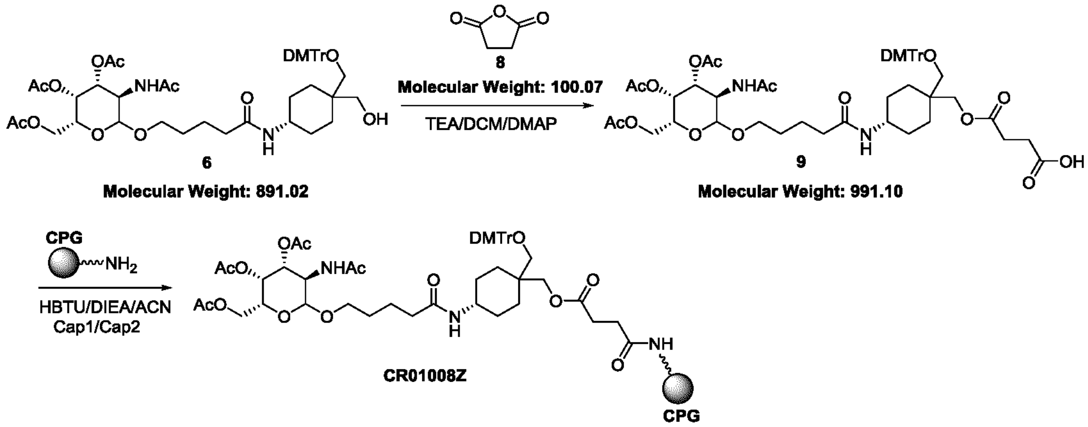


(2-1) Synthesis of compound 9

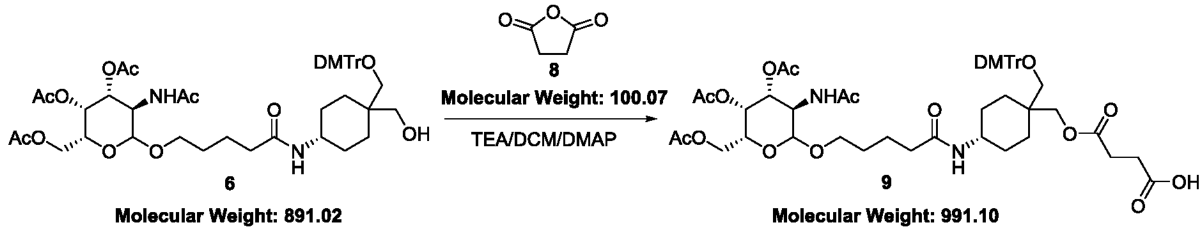


The compound 6 prepared according to step (1-4) (500 mg) was dissolved in 10 ml of dichloromethane, compound 8 (succinic anhydride, 112 mg), DMAP (6.8 mg) and TEA (226.2 mg) were added, nitrogen replacement was performed three times, and the reaction system was stirred at 25°C for 16 h of reaction. The reaction solution was purified by flash chromatography to obtain compound 9 (300 mg, 53.6% yield). MS-ESI (*m/z*) = 1013 $[M + Na]^+$.

(2-2) Synthesis of compound CR01008Z

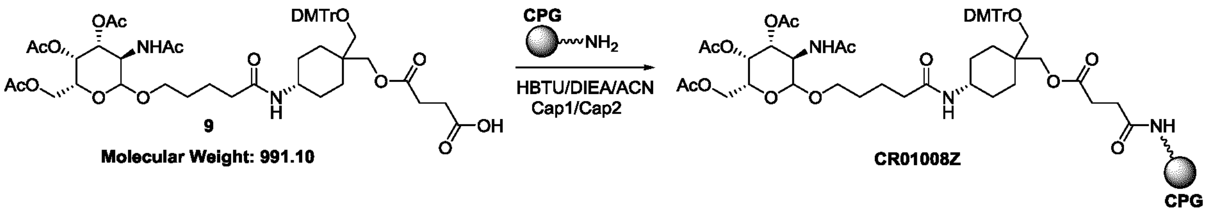


The compound 9 (50 mg) prepared according to step (2-1), amino CPG (1.25 g, 80 μmol/g, 0.1 mmol), HBTU (27 mg), and DIEA (12 mg) were added to a 20 ml vial, and the reaction was carried out on a shaker for 16 h. After the reaction was completed, the reaction solution was filtered to obtain a filter cake. The filter cake was washed once with 10 ml of acetonitrile (1×10 ml) and then dried under vacuum. The dried filter cake, DMAP (3 mg), Cap1 (10 ml, 200 V) and Cap2 (1 ml, 20 V) were added to a 20 ml vial, and the reaction was carried out on a shaker for 6 h. After the reaction was completed, the reaction solution was filtered to obtain a filter cake. The filter cake was washed once with 10 ml of acetonitrile (1×10 ml) and then dried under vacuum to obtain the compound CR01008Z (1.03 g, loading amount: 20-30 μmol/g).

Wherein, Cap1 and Cap2 were capping agents, Cap1 was a solution of 20 vol% N-methylimidazole in a pyridine/acetonitrile mixture, the volume ratio of pyridine to acetonitrile was 3:5, and Cap2 was a solution of 20 vol% acetic anhydride in acetonitrile.

(3) Synthesis of compound CR01013

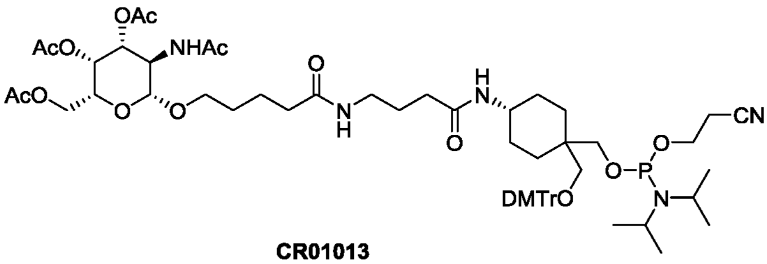

**CR01013**

Synthetic route of compound CR01013:

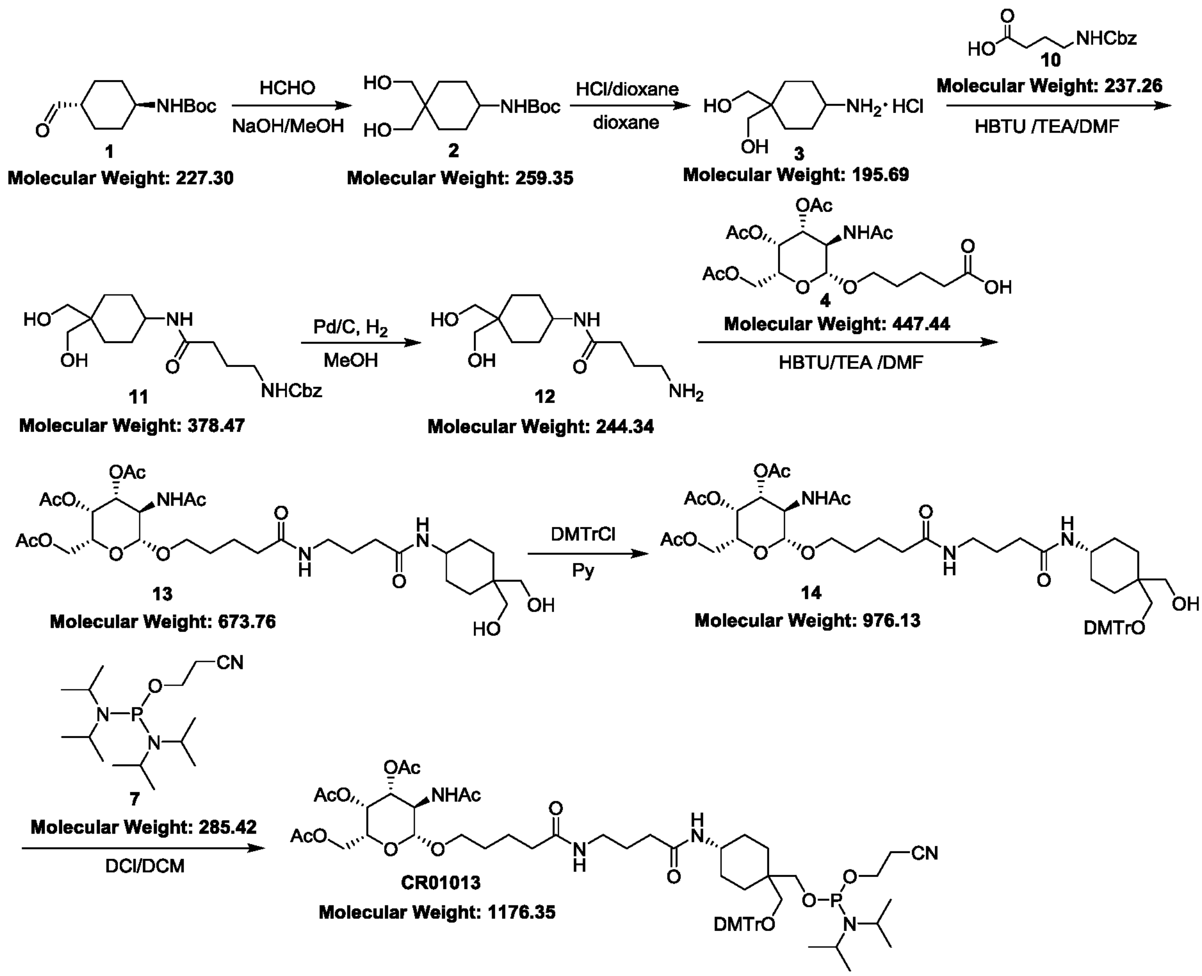


(3-1) Synthesis of compound 2

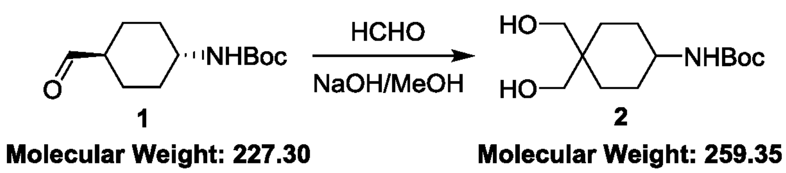


Compound 1 (trans-4-(Boc-amino)cyclohexylcarboxaldehyde, 4.9 g) was dissolved in 17 ml of methanol. An aqueous solution of formaldehyde (4.21 g, at a concentration of 37 mass%) and an aqueous solution of sodium hydroxide

(6.5 ml, at a concentration of 45.3 mass%) were added dropwise. After the dropwise addition was completed, the reaction system was heated to 60°C, and stirred at 60°C for 2 h of reaction. After the reaction was completed, the reaction solution was cooled to room temperature and evaporated to dryness under reduced pressure to obtain a crude product as a white solid. The crude product was slurried with a small amount of water, filtered and dried to obtain compound 2 as a white solid (4.8 g, 85.9% yield). ESI-MS (*m*/*z*) = 260.2[M+H]$^+$.

(3-2) Synthesis of compound 3

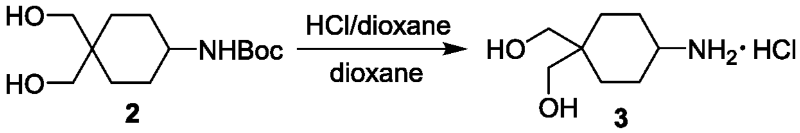


2 Molecular Weight: 259.35 3 Molecular Weight: 195.69

The compound 2 (4.8 g) prepared according to step (3-1) was dissolved in 25 ml of 1,4-dioxane, a solution of hydrochloric acid in 1,4-dioxane (25 ml, 4 M) was added, and the reaction system was stirred and reacted at 25°C for 2 h. After the reaction was completed, the reaction solution was evaporated to dryness under reduced pressure to obtain compound 3 as a white solid (3.6 g, 99.4% yield).

(3-3) Synthesis of compound 11

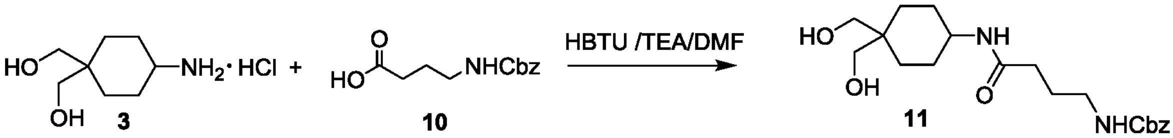


3 Molecular Weight: 195.69 + 10 Molecular Weight: 237.26 11 Molecular Weight: 378.47

The compound 3 (3.6 g) prepared according to step (3-2) was dissolved in 36 ml of DMF, and then TEA (5.62 g), compound 10 (N-benzyloxycarbonyl-4-aminobutyric acid, 5.28 g), and HBTU (8.43 g) were added, and the reaction system was stirred and reacted at 25°C for 16 h. After the reaction was completed, 200 ml of saturated aqueous sodium bicarbonate solution was added. The reaction solution was extracted with 100 ml of ethyl acetate three times (3×100 ml), the organic phases were combined, washed with 50 ml of saturated aqueous solution of sodium chloride (1×50 ml), dried over anhydrous sodium sulfate, evaporated to dryness under reduced pressure, and then purified by column chromatography (elution gradient: dichloromethane: methanol=10:1) to obtain compound 11 as a white solid (2.3 g, 33.0% yield). ESI MS (*m*/*z*) = 379.5[M+H]$^+$.

(3-4) Synthesis of compound 12

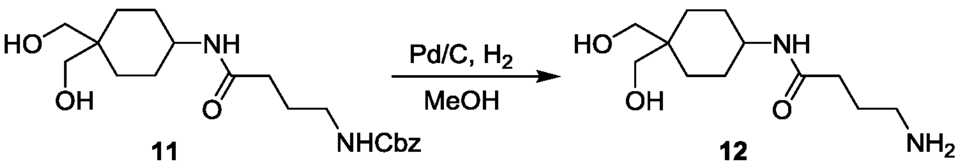


11 Molecular Weight: 378.47 12 Molecular Weight: 244.34

The compound 11 (2.3 g) prepared in step (3-3) was dissolved in 23 ml of methanol, wet palladium carbon (230 mg, 10 mass% loading) was added, hydrogen replacement was performed three times, and the reaction system was stirred and reacted at 25°C for 16 h under a hydrogen atmosphere (15 psi). After the reaction was completed, the reaction solution was filtered to obtain a filtrate, and the filtrate was evaporated to dryness under reduced pressure to obtain compound 12 as a yellow oil (1.48 g, 99.8% yield).

(3-5) Synthesis of compound 13

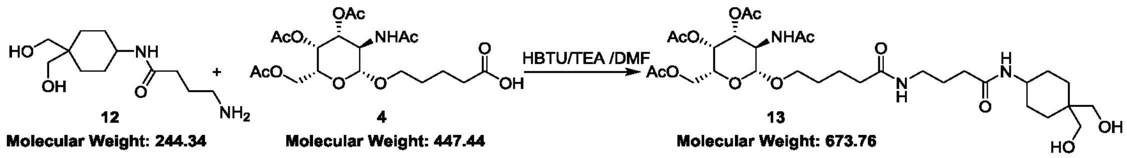


The compound 12 (1.48 g) prepared in step (3-4) was dissolved in 15 ml of DMF, triethylamine (TEA, 1.22 g), compound 4 (1.35 g), and HBTU (3.45 g) were added, and the reaction system was stirred and reacted at 25°C for 16 h. After the reaction was completed, 150 ml of saturated aqueous solution of sodium bicarbonate was added, the reaction solution was extracted with 50 ml of ethyl acetate for three times (3×50 ml). The organic phases were combined, washed with 30 ml of saturated aqueous solution of sodium chloride (1×30 ml), dried over anhydrous sodium sulfate, evaporated to dryness under reduced pressure, and then purified by column chromatography (elution gradient: water: acetonitrile=5:1) to obtain compound 13 as a white solid (1.3 g, 31.8% yield). ESI-MS (m/z): 674.3 $[M+H]^+$.

(3-6) Synthesis of compound 14

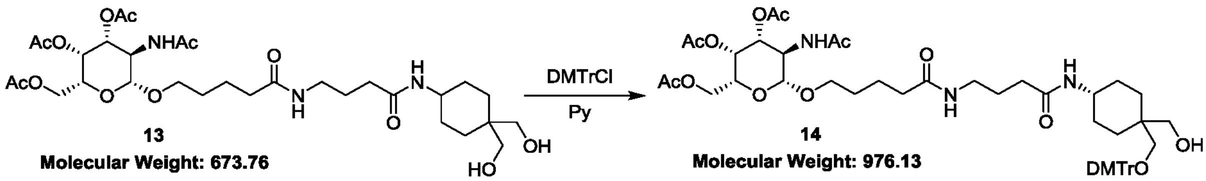


The compound 13 (1.1 g) prepared in step (3-5) was dissolved in 11 ml of pyridine, the reaction system was cooled down to 0°C in an ice-water bath, and DMTrCl (813 mg) was added in batches at 0°C. The reaction system was stirred and reacted at 0°C for 1 h. After the reaction was completed, the reaction solution was added with methanol to quench the reaction, evaporated to remove solvent, and purified by column chromatography (elution gradient: water: acetonitrile=1:4) to obtain compound 14 as a white solid (800 mg, 50.3% yield). ESI-MS (m/z):976.5$[M+H]^+$.

(3-7) Synthesis of compound CR01013

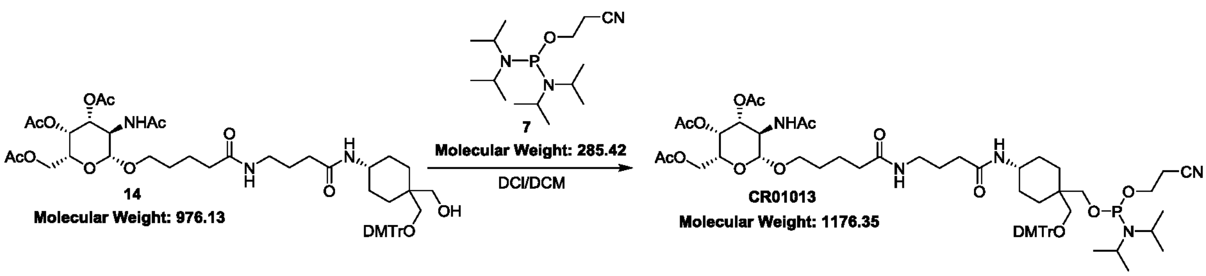


At room temperature, compound 14 (550 mg) was dissolved in 5 ml of dichloromethane (DCM), and 4,5-dicyanoimidazole (DCI, 53.2 mg) and compound 7 (2-cyanoethyl N,N,N',N'-tetraisopropyl phosphordiamidite, 255.4 mg) were added. Nitrogen replacement was performed three times, and the reaction system was stirred at 25°C in nitrogen atmosphere for 1 h. After the reaction was completed, the reaction solution was washed twice with 5 ml of saturated aqueous sodium bicarbonate solution (2×5 ml) and then once with 30 ml of saturated aqueous sodium chloride solution (1×30 ml). The organic phase was separated, dried over anhydrous sodium sulfate, evaporated under reduced pressure to remove solvent, and purified by column chromatography (elution gradient: dichloromethane: methanol=20:1) to obtain compound CR01013 as a white solid (532 mg, 80.4% yield). ESI-MS (m/z): 1176.7$[M+H]^+$.

$^1$H NMR (400 MHz, DMSO-$d_6$) δ 0.95 - 1.05 (d, $J$ = 6.7 Hz, 5H), 1.06 - 1.15 (q, $J$ = 7.6 Hz, 8H), 1.15 - 1.21 (t, $J$ = 7.2

Hz, 14H), 1.72 - 1.80 (s, 3H), 1.84 - 1.92 (s, 3H), 1.94 - 2.07 (d, $J$ = 16.0 Hz, 7H), 2.07 - 2.14 (s, 3H), 2.64 - 2.72 (q, $J$ = 5.8 Hz, 2H), 2.74 - 2.89 (d, $J$ = 8.5 Hz, 2H), 3.35 - 3.56 (m, 4H), 3.57 - 3.70 (m, 4H), 3.71 - 3.77 (s, 6H), 3.81 - 3.93 (m, 1H), 3.96 - 4.09 (d, $J$ = 6.4 Hz, 3H), 6.82 - 6.97 (d, $J$ = 8.7 Hz, 4H), 7.17 - 7.27 (t, $J$ = 8.7 Hz, 5H), 7.27 - 7.34 (t, $J$ = 7.6 Hz, 2H), 7.34 - 7.43 (d, $J$ = 7.5 Hz, 2H).

(4) Synthesis of compound CR01013Z

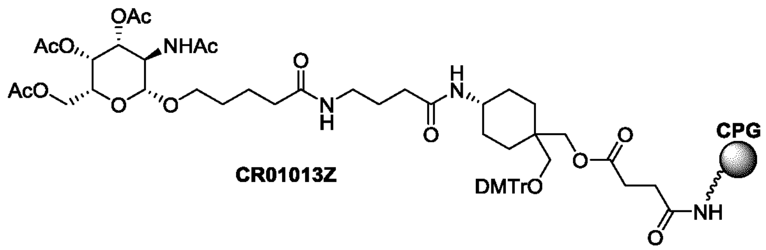

CR01013Z

Compound CR01013Z was prepared by connecting the compound 14 used in the synthesis of the compound CR01013 to the solid phase support CPG.

Synthetic route of compound CR01013Z:

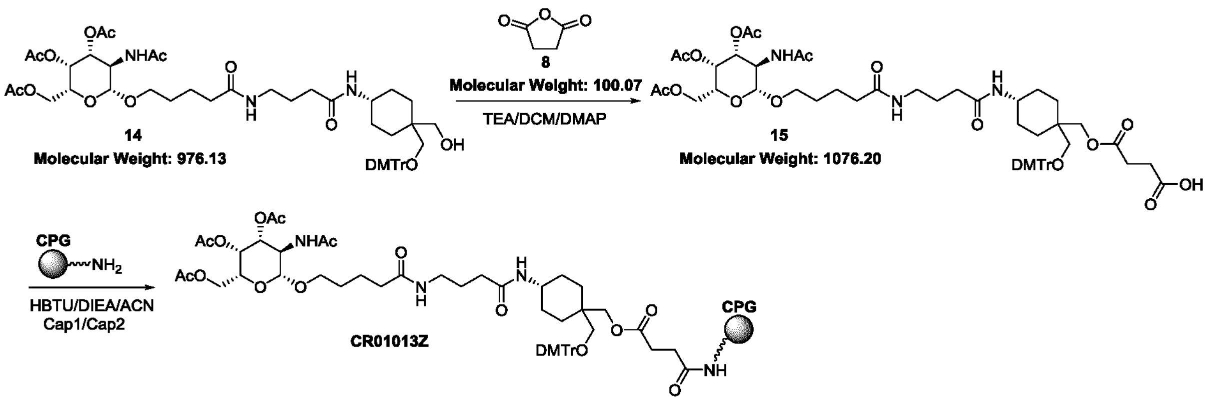


14
Molecular Weight: 976.13
15
Molecular Weight: 1076.20
CR01013Z

(4-1) Synthesis of compound 15

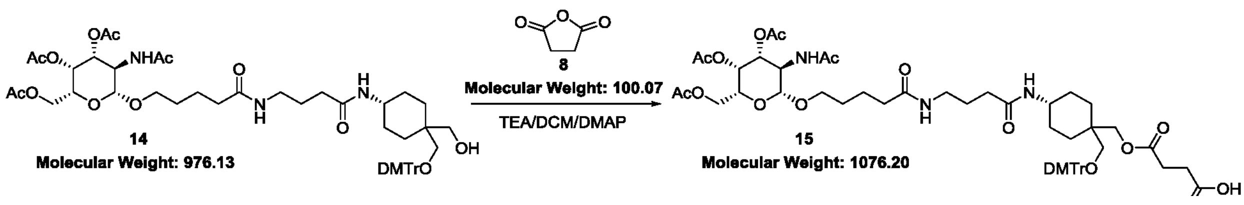


14
Molecular Weight: 976.13
15
Molecular Weight: 1076.20

At room temperature, the compound 14 (100 mg, 0.10 mmol) was dissolved in 2 ml of dichloromethane, and triethylamine (25.9 mg, 0.25 mmol), DMAP (1.25 mg, 0.01 mmol) and compound 8 (succinic anhydride, 15.4 mg, 0.15 mmol) were added. The reaction system was stirred at 25°C for 16 h. After the reaction was completed, the reaction system was evaporated to remove solvent and purified by column chromatography (elution gradient was water: acetonitrile = 2:1) to obtain compound 15 as a yellow oil (110 mg, 0.10 mmol, 100% yield). ESI-MS (m/z) = 1099.3 [M+Na]$^+$.

(4-2) Synthesis of compound CR01013Z

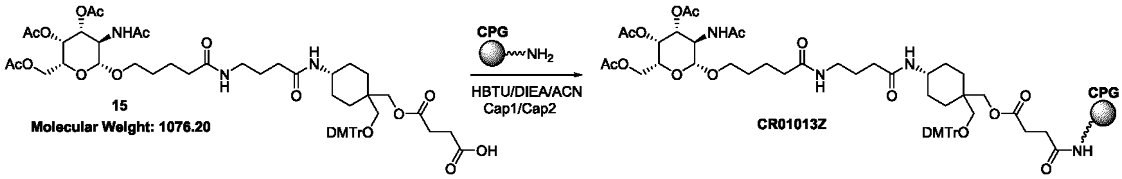


The compound 15 (50 mg, 0.04 mmol) was dissolved in 10 ml of acetonitrile, HBTU (24.2 mg, 0.06 mmol), DIEA (11.0 mg, 0.08 mmol) and amino-CPG (1.06 g, loading amount: 80 μmol/g) were added, and the reaction system was stirred and reacted at 25°C for 16 h. After the reaction was completed, the reaction solution was filtered to obtain a filter cake, and the filter cake was washed twice with 50 ml of dichloromethane (2×50 ml), three times with 50 ml of acetonitrile (3×50 ml), and once with 50 ml of ethyl acetate (1×50 ml) in sequence, and then dried under vacuum. Cap1 (4.8 ml), Cap2 (0.54 ml) and DMAP (2.59 mg) were added to the dried filter cake, and the reaction system was stirred and reacted at 25°C for 5 h. After the reaction was completed, the reaction solution was filtered to obtain a filter cake. The filter cake was washed three times with 50 ml of acetonitrile (3×50 ml) and dried under vacuum to obtain compound CR01013Z (900 mg, loading amount: 20-30 μmol/g).

Wherein, Cap1 and Cap2 were capping agents, Cap1 was a solution of 20 vol% N-methylimidazole in a pyridine/acetonitrile mixture, the volume ratio of pyridine to acetonitrile was 3:5, and Cap2 was a solution of 20 vol% acetic anhydride in acetonitrile.

(5) Synthesis of reference compound CR01014

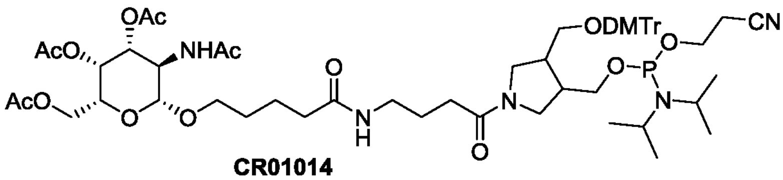

CR01014

Synthetic route of reference compound CR01014:

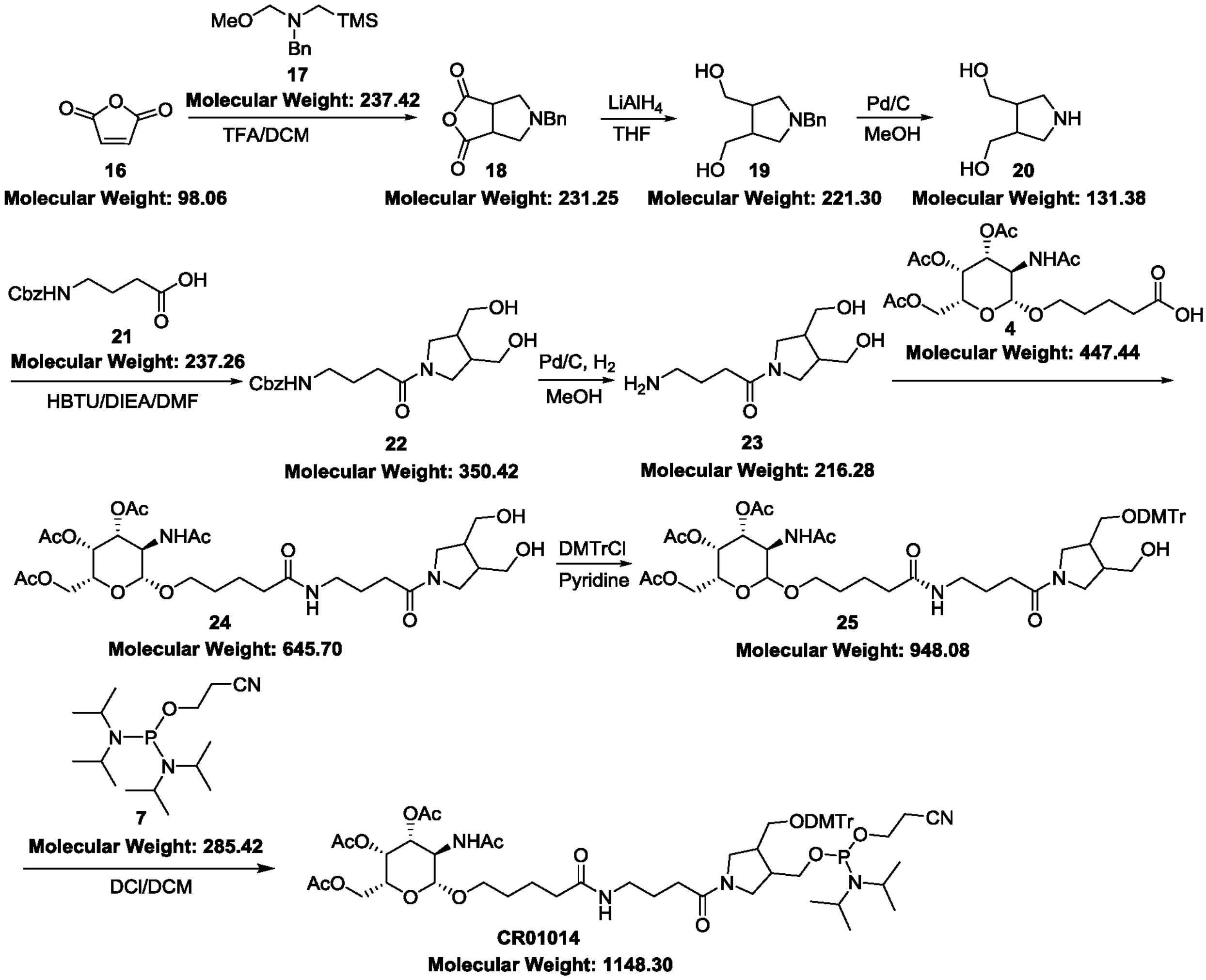


(5-1) Synthesis of compound 18

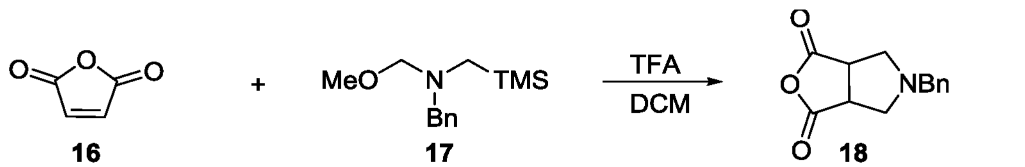


16 Molecular Weight: 98.06

17 Molecular Weight: 237.42

18 Molecular Weight: 231.25

Compound 16 (maleic anhydride, 5.0 g), compound 17 (N-(methoxymethyl)-N-(trimethylsilylmethyl)benzylamine, 12.1 g) and TFA (trifluoroacetic acid, 0.58 g) were dissolved in 35 ml of dichloromethane, nitrogen replacement was performed three times, and the reaction system was stirred and reacted at 25°C for 3 h. After the reaction was completed, the reaction solution was washed once with 10 ml of purified water (1×10 ml) and once with 10 ml of saturated aqueous sodium chloride solution (1×10 ml) in sequential. The organic phase was separated, dried over anhydrous sodium sulfate, and evaporated to dryness under reduced pressure to obtain compound 18 (11 g), which was directly used in the next reaction without purification. MS-ESI (m/z) = 232.0 $[M + H]^+$.

(5-2) Synthesis of compound 19

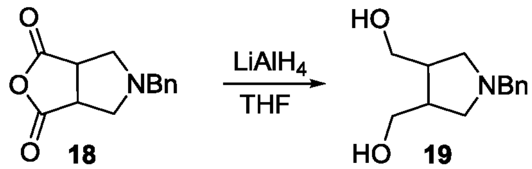


18 19

Molecular Weight: 231.25 Molecular Weight: 221.30

The compound 18 (4.1 g) prepared in step (5-1) was dissolved in 20 ml of tetrahydrofuran, nitrogen replacement was performed three times, a solution of $LiAlH_4$ in tetrahydrofuran (1.0 M, 17.7 ml) was added dropwise at 0°C, and the reaction system was cooled down to 0°C in an ice-water bath, stirred and reacted at 0°C for 1 h. After the reaction was completed, 32 ml of purified water and then 24 ml of 1.0 M aqueous sodium hydroxide solution were added dropwise to the reaction solution sequentially, the reaction solution was filtered to obtain a filtrate, and the filtrate was evaporated to dryness under reduced pressure to obtain compound 19 (6.0 g), which was directly used in the next reaction without purification. MS-ESI (m/z) = 222.3 $[M + H]^+$.

(5-3) Synthesis of compound 20

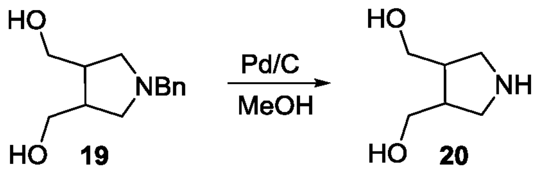


19 20

Molecular Weight: 221.30 Molecular Weight: 131.38

The compound 19 (5.0 g) prepared in step (5-2) was dissolved in 50 ml of methanol, and wet palladium carbon (0.5 g, 10 mass% loading) and palladium carbon hydroxide (0.5 g, 10 mass% loading) were separately added. The hydrogen replacement was performed three times, and the reaction system was heated to 40°C, stirred and reacted at 40°C for 16 h. After the reaction was completed, the reaction solution was filtered to obtain a filtrate, and the filtrate was evaporated to dryness under reduced pressure to obtain compound 20 (2.9 g). MS-ESI (m/z) = 132.18 $[M + H]^+$.

(5-4) Synthesis of compound 22

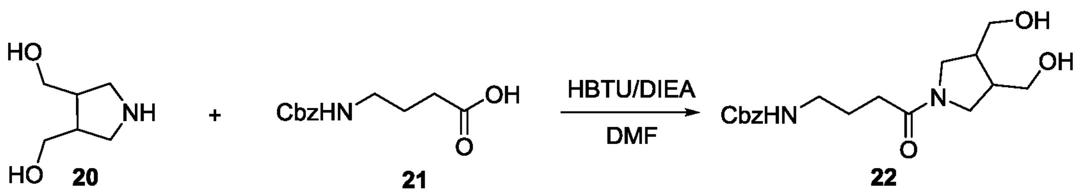


20 21 22

Molecular Weight: 131.38 Molecular Weight: 237.26 Molecular Weight: 350.42

The compound 20 (4.0 g) prepared in step (5-3), compound 21 (N-benzyloxycarbonyl-4-aminobutyric acid, 2.8 g) and DIEA (N,N-diisopropylethylamine, 15.6 g) were dissolved in 20 ml of N,N-dimethylformamide (DMF), HBTU (benzotriazole-N,N,N',N'-tetramethyluronium hexafluorophosphate, 17.2 g) was added, nitrogen replacement was performed three times, and the reaction system was stirred and reacted at room temperature for 3 h. After the reaction was completed, the reaction solution was evaporated to dryness under reduced pressure and purified by reverse phase chromatography to obtain compound 22 (1.46 g, 35.6% yield). MS-ESI (m/z) = 367.41 $[M + H]^+$.

(5-5) Synthesis of compound 23

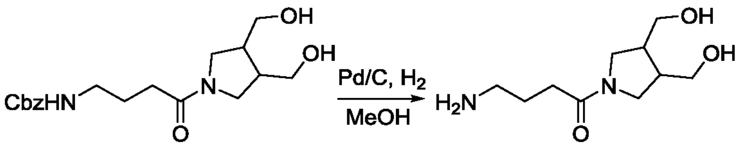


22
Molecular Weight: 350.42

23
Molecular Weight: 216.28

The compound 22 (1.46 g) prepared in step (5-4) was dissolved in 10 ml of methanol, wet palladium carbon (0.15 g, 10 mass%) was added, hydrogen replacement was performed three times, and the reaction system was stirred and reacted at 25°C for 16 h. After the reaction was completed, the reaction solution was filtered to obtain a filtrate, and the filtrate was evaporated to dryness under reduced pressure to obtain a crude compound 23 (1.06 g), which was directly used in the next reaction without purification. MS-ESI (m/z) = 233.28 $[M + H]^+$.

(5-6) Synthesis of compound 24

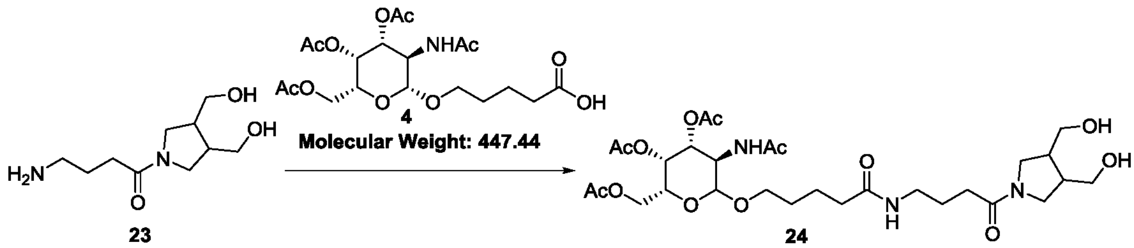

4
Molecular Weight: 447.44

23
Molecular Weight: 216.28

24
Molecular Weight: 645.70

The compound 23 (1.02 g) prepared in step (5-5) and compound 4 (5-[[(2R,3R,4R,5R,6R)-3-acetamido-4,5-diacetyloxy-6-(acetyloxymethyl)-2-tetrahydropyranyl]o xy]pentanoic acid, 1.41 g) were dissolved in 14 ml of N,N-dimethylformamide, and DIEA (N,N-diisopropylethylamine, 0.81 g) and 2-(7-azobenzotriazolyl)-N,N,N',N'-tetramethyluronium hexafluorophosphate (1.8 g) were added. The nitrogen replacement was performed three times, and the reaction system was stirred and reacted at room temperature for 3 h. After the reaction was completed, the reaction solution was evaporated to dryness under reduced pressure and purified by flash chromatography to obtain compound 24 (1.28 g, 63.5% yield). MS-ESI (m/z) = 648.68 $[M + H]^+$.

(5-7) Synthesis of compound 25

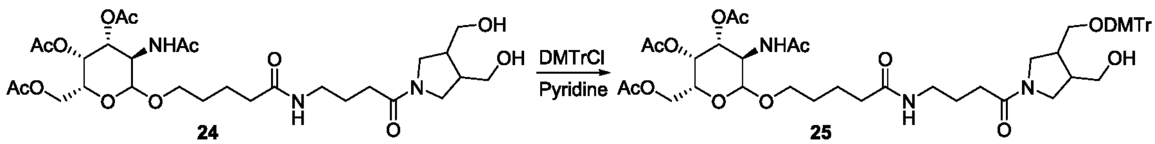


24
Molecular Weight: 645.70

25
Molecular Weight: 948.08

The compound 24 (1.18 g) prepared in step (5-6) was dissolved in 12 ml of pyridine, DMTrCl (4,4'-dimethoxytriphenylmethyl chloride, 0.98 g) was added in batches, nitrogen replacement was performed three times, and the reaction system was stirred and reacted at room temperature for 3 h. After the reaction was completed, the reaction solution was evaporated to dryness under reduced pressure and purified by flash chromatography to obtain compound 25 (1.0 g, 61.1% yield). MS-ESI (m/z) = 951.0 $[M + H]^+$.

(5-8) Synthesis of compound CR01014

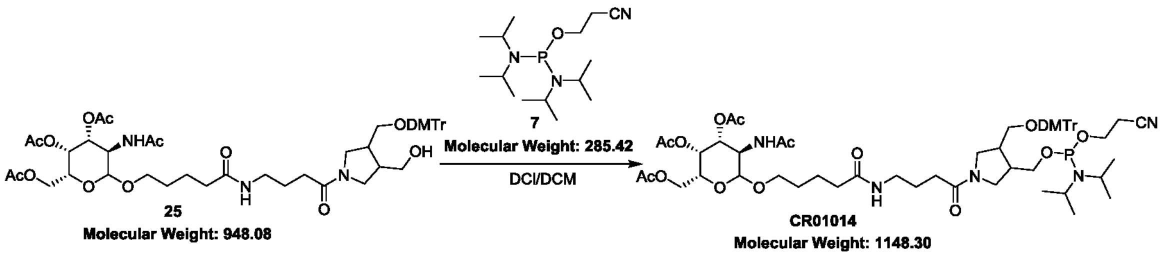


The compound 25 (300 mg) prepared in step (5-7) was dissolved in 6 ml of dichloromethane, compound 7 (bis(diisopropylamino)(2-cyanoethoxy)phosphine, 152 mg) and 4,5-dicyanoimidazole (DCI, 30 mg) were added in batches, nitrogen replacement was performed three times and the reaction system was stirred and reacted at room temperature for 3 h. After the reaction was completed, the reaction solution was evaporated to dryness under reduced pressure and purified by flash chromatography to obtain the compound CR01014 (270 mg, 74.4% yield). MS-ESI (m/z) = 1151.27 $[M + H]^+$.

$^1H$ NMR (400 MHz, DMSO-d6) δ: 0.92 - 1.03 (d, J = 6.7 Hz, 8H), 1.04 - 1.13 (d, J = 6.8 Hz, 7H), 1.16 - 1.29 (m, 4H), 1.38 - 1.53 (dq, J = 7.2, 14.6 Hz, 4H), 1.55 - 1.68 (dt, J = 9.0, 15.1 Hz, 2H), 1.75 - 1.82 (s, 3H), 1.86 - 1.94 (s, 3H), 1.97 - 2.07 (s, 6H), 2.09 - 2.15 (s, 3H), 2.15 - 2.29 (m, 2H), 2.64 - 2.76 (d, J = 5.5 Hz, 3H), 2.99 - 3.16 (dt, J = 6.7, 14.4 Hz, 4H), 3.20 - 3.30 (d, J = 14.8 Hz, 1H), 3.37 - 3.53 (tt, J = 7.1, 14.6 Hz, 6H), 3.53 - 3.66 (dt, J = 8.4, 17.5 Hz, 3H), 3.74 - 3.78 (s, 6H), 4.00 - 4.09 (s, 3H), 6.84 - 6.96 (d, J = 8.3 Hz, 4H), 7.19 - 7.28 (t, J = 7.6 Hz, 5H), 7.28 - 7.41 (dt, J = 7.8, 22.9 Hz, 4H), 7.70 - 7.88 (d, J = 5.9 Hz, 2H).

(6) Synthesis of reference compound CR01014Z

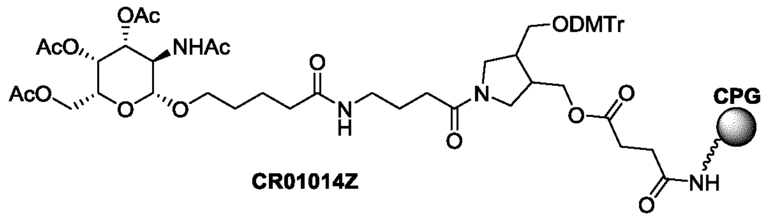

CR01014Z

Compound CR01014Z was prepared by connecting the compound used in the synthesis of the compound CR01014 to the solid phase support CPG.

Synthetic route of reference compound CR01014Z:

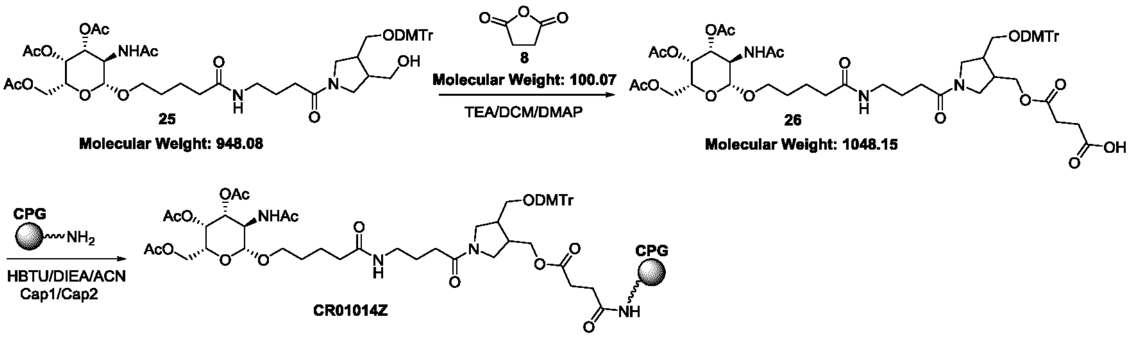


(6-1) Synthesis of compound 26

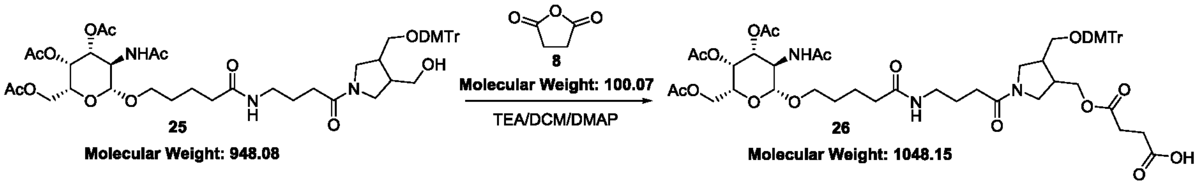


The compound 25 (100 mg) prepared in step (5-7) was dissolved in 2 ml of dichloromethane, compound 8 (succinic anhydride, 15.7 mg), 4-dimethylaminopyridine (DMAP, 1.2 mg) and triethylamine (TEA, 19.7 mg) were added, and nitrogen replacement was performed for three times. The reaction system was stirred and reacted at room temperature for 16 h. After the reaction was completed, the reaction solution was evaporated to dryness under reduced pressure and purified by flash chromatography to obtain compound 26 (73 mg, 66.7% yield). MS-ESI (m/z) = $[M + H]^+$.

(6-2) Synthesis of compound CR01014Z

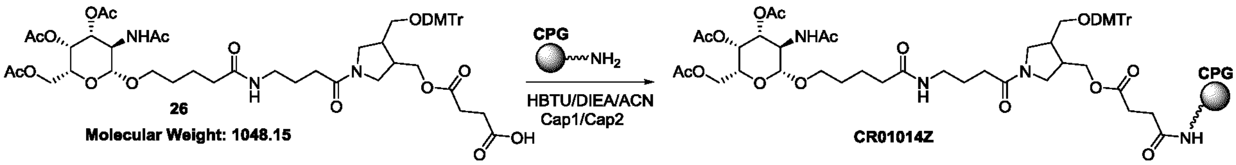


The compound 26 (50 mg) prepared in step (6-1), amino CPG (1.19 g,), benzotriazole-N,N,N',N'-tetramethyluronium hexafluorophosphate (27 mg), and N,N-diisopropylethylamine (12 mg) were added to a 20 ml sample bottle, and the reaction was carried out on a shaker for 16 h. After the reaction was completed, the reaction solution was filtered to obtain a filter cake. The filter cake was washed once with 10 ml of acetonitrile (1×10 ml) and then dried under vacuum. The dried filter cake, 4-dimethylaminopyridine (DMAP, 3 mg), Cap1 (10 ml) and Cap2 (1 ml) were added to a 20 ml sample bottle and the reaction was carried out on a shaker for 6 h. After the reaction was completed, the reaction solution was filtered to obtain a filter cake. The filter cake was washed once with 10 ml of acetonitrile (1×10 ml) and then dried under vacuum to obtain the compound CR01014Z (1.03 g, loading amount: 20-30 μmol/g).

Wherein, Cap1 and Cap2 were capping agents, Cap1 was a solution of 20 vol% N-methylimidazole in a pyridine/acetonitrile mixture, the volume ratio of pyridine to acetonitrile was 3:5, and Cap2 was a solution of 20 vol% acetic anhydride in acetonitrile.

(7) Synthesis of reference compound L96-PS

Compound L96-PS was purchased from Asymchem Laboratories (Tianjin) Co., Ltd, with a loading amount of 120 ±12 μmol/g as determined by UV/HPLC.

Structure formula of compound L96-PS:

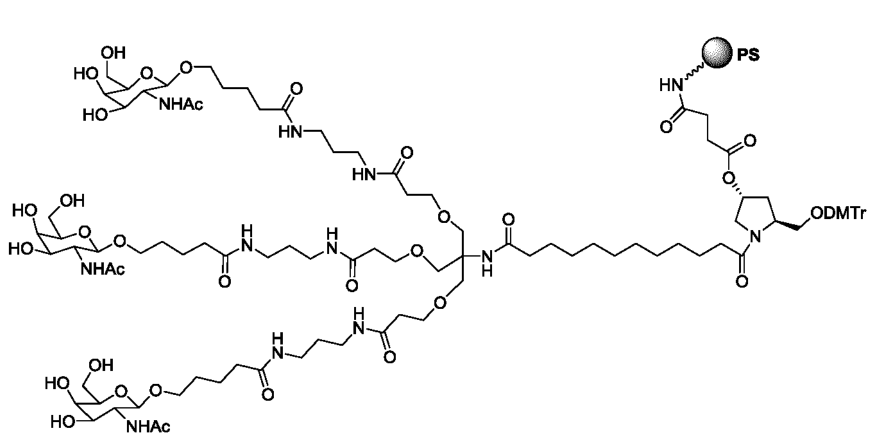

wherein, PS represents a polystyrene resin as solid phase support.

Example 2 Synthesis of siRNA conjugate

(1) Polymerization of compounds

According to the solid-phase nucleic acids synthesis using phosphoramidite method, compounds not connected to the solid phase support (i.e., CR01008, CR01013 and CR01014) were connected one by one in cycles starting from the above compounds connected to the solid phase support (i.e., CR01008Z, CR01013Z and CR01014Z). Specifically, CR01008 is connected one by one in cycles starting from CR01008Z, CR01013 is connected one by one in cycles starting from CR01013Z, and the compound CR01014 is connected one by one in cycles starting from CR01014Z.

The connecting of each compound comprised a four-step reaction of deprotection, coupling, capping, and oxidation or sulfurization. The synthesis condition was given below.

Each compound not connected to the solid phase support (i.e., CR01008, CR01013 and CR01014) was prepared with acetonitrile to a solution with a concentration of 0.1 M.

The condition for deprotection reaction in each step was identical, including a temperature of 25°C, a reaction time of 70 seconds, a solution of dichloroacetic acid in dichloromethane (3 vol%) as a deprotection agent, and a molar ratio of the dichloroacetic acid to the protecting group 4,4'-dimethoxytrityl on the solid phase support of 5: 1.

The condition for coupling reaction in each step was identical, including a temperature of 25°C, a molar ratio of the compound connected to the solid phase support to the compound unconnected to the solid phase support of 1: 10, a molar ratio of the compound connected to the solid phase support to a coupling agent of 1: 65, a reaction time of 600 seconds, a solution of 0.5 M 5-ethylthio-1H-tetrazole in acetonitrile as a coupling agent, and a solution of 0.2 M xanthane hydride in a acetonitrile/pyridine mixture (volume ratio of acetonitrile: pyridine = 1:1) as a thio agent.

The condition for capping reaction in each step was identical, including a temperature of 25°C, a reaction time of 2 min, a mixed solution of Cap1 and Cap2 in a molar ratio of 1:1 as capping agent, a solution of 20 vol% N-methylimidazole in a pyridine/acetonitrile mixture as Cap1, a volume ratio of pyridine to acetonitrile of 3:5, a solution of 20 vol% acetic anhydride in acetonitrile as Cap2, and a molar ratio of N-methylimidazole in the capping agent Cap1: acetic anhydride in the capping agent Cap2: the compound connected to the solid phase support of 1:1:1.

The condition for oxidation reaction or sulfurization reaction in each step was identical. The condition for oxidation reaction included a temperature of 25°C, a reaction time of 3 seconds, 0.05 M iodine water as an oxidation agent, and a molar ratio of iodine to the nucleic acid sequence connected to the solid phase support in the coupling reaction of 30: 1. The oxidation reaction was carried out in a mixed solvent of water/pyridine (volume ratio of water: pyridine = 1:9). The condition for sulfurization reaction included a temperature of 25°C, a reaction time of 360 seconds, a solution of 0.2 M xanthane hydride in pyridine as a thio agent, and a molar ratio of the thio agent to the compound connected to the solid phase support in the coupling reaction of 4:1. The sulfurization reaction was carried out in a mixed solvent of water/pyridine (volume ratio of water: pyridine = 1:9).

The trimeric CR01008 (noted as (CR01008)×3), trimeric CR01013 (noted as (CR01013)×3), and trimeric CR01014 (noted as (CR01014)×3) were separately prepared by the above method.

Structure formula of the trimeric CR01008:

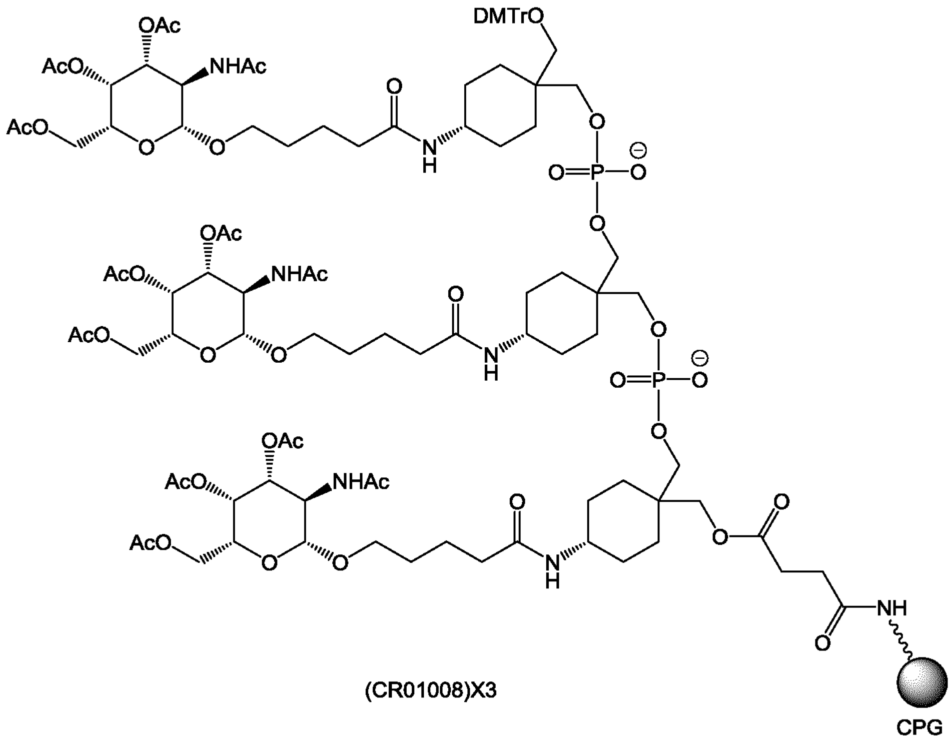

(CR01008)X3

Structure formula of the trimeric CR01013:

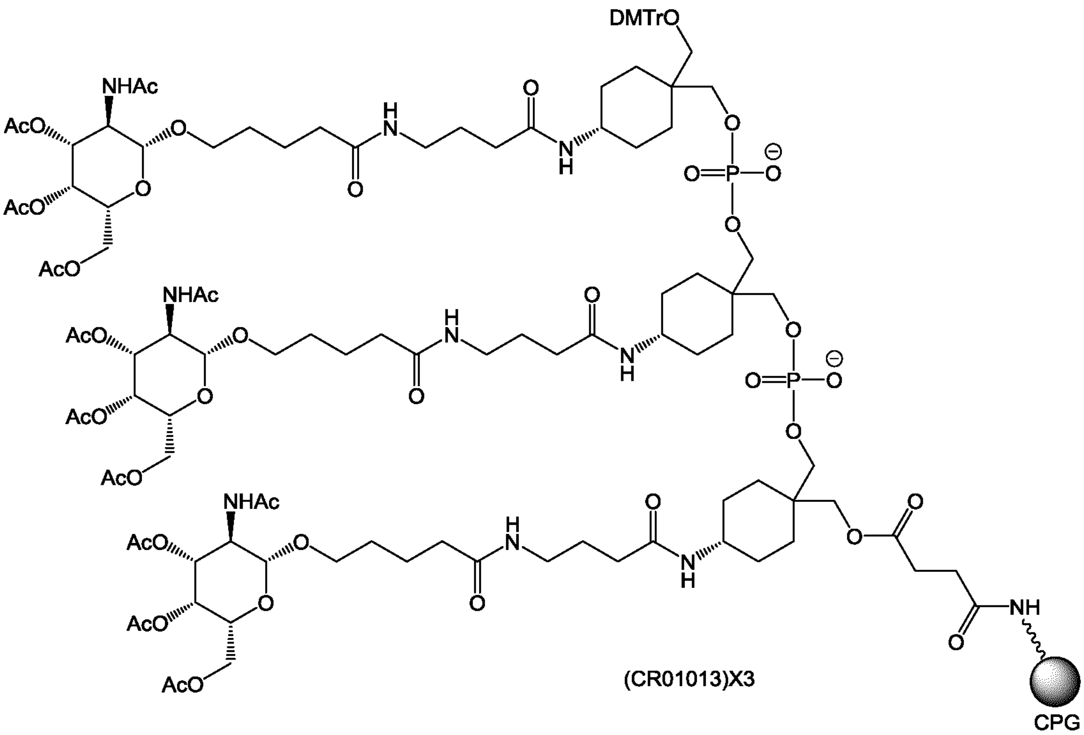

(CR01013)X3

Structure formula of the trimeric CR01014:

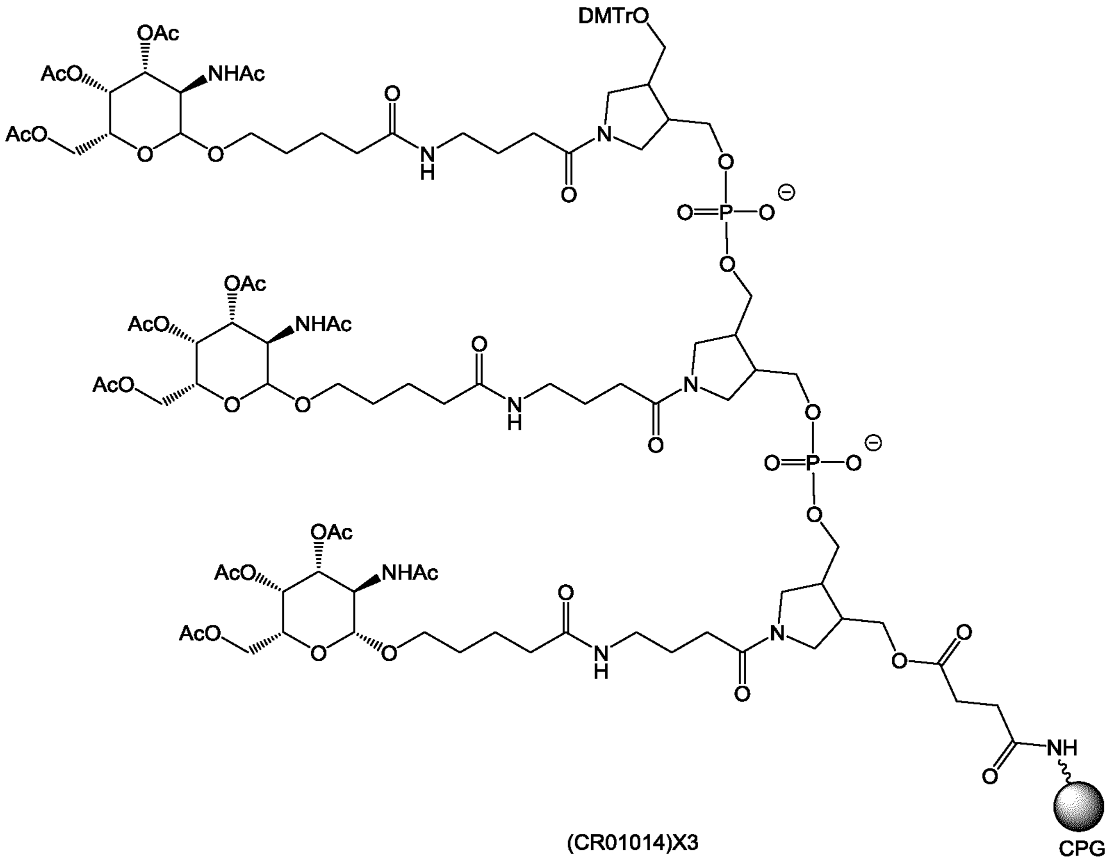

(CR01014)X3

(2) Synthesis of sense strand (SS)

According to the solid-phase nucleic acids synthesis using phosphoramidite method, nucleoside monomers were linked one by one in the direction from 3' to 5' according to the nucleotides sequence, starting from the compounds connected to the solid phase support (i.e., trimeric CR01008, trimeric CR01013, trimeric CR01014 and L96) in cycles. The linking of each nucleoside monomer comprised a four-step reaction of deprotection, coupling, capping, and oxidation or sulfurization. The synthesis condition was given below.

The nucleoside monomers were prepared to a solution of 0.1 M nucleoside monomer in acetonitrile.

The condition for deprotection reaction in each step was identical, including a temperature of 25°C, a reaction time of 70 seconds, a solution of dichloroacetic acid in dichloromethane (3 vol%) as a deprotection agent, and a molar ratio of the dichloroacetic acid to the protecting group 4,4'-dimethoxytrityl on the solid phase support of 5:1.

The condition for coupling reaction in each step was identical, including a temperature of 25°C, a molar ratio of the nucleic acid sequence linked to the solid phase support to the nucleoside monomers of 1:10, a molar ratio of the nucleic acid sequence linked to the solid phase support to a coupling agent of 1:65, a reaction time of 600 seconds, a solution of 0.5 M 5-ethylthio-1H-tetrazole in acetonitrile as a coupling agent, and a solution of 0.2 M xanthane hydride in a acetonitrile/pyridine mixture (volume ratio of acetonitrile: pyridine = 1:1) as a thio agent.

The condition for capping reaction in each step was identical, including a temperature of 25°C, a reaction time of 2 min, a mixed solution of Cap1 and Cap2 in a molar ratio of 1:1 as a capping agent, a solution of 20 vol% N-methylimidazole in a pyridine/acetonitrile mixture as Cap1, a volume ratio of pyridine to acetonitrile of 3:5, a solution of 20 vol% acetic anhydride in acetonitrile as Cap2, and a molar ratio of N-methylimidazole in the capping agent Cap1: acetic anhydride in the capping agent Cap2: the nucleic acid sequence connected to the solid phase support of 1:1:1.

The condition for oxidation reaction in each step was identical. The condition for oxidation reaction included a temperature of 25°C, a reaction time of 3 seconds, 0.05 M iodine water as an oxidation agent, and a molar ratio of iodine to the nucleic acid sequence connected to the solid phase support in the coupling reaction of 30: 1. The oxidation reaction was carried out in a mixed solvent of water/pyridine (volume ratio of water: pyridine = 1:9). The condition for sulfurization reaction included a temperature of 25°C, a reaction time of 360 seconds, a solution of 0.2 M xanthane hydride in pyridine as a thio agent, and a molar ratio of the thio agent to the nucleic acid sequence connected to the solid phase support in the coupling reaction of 4:1. The sulfurization reaction was carried out in a mixed solvent of water/pyridine (volume ratio of water: pyridine = 1:9).

After the last nucleoside monomer was linked, the nucleic acid sequence connected to the solid phase support was cleaved, deprotected, purified and desalted in turn, and then freeze-dried to obtain the sense strand.

The conditions for cleavage and deprotection were as follows: the synthesized nucleotide sequence connected to the solid phase support was added into a 25 mass% aqueous ammonia solution for 16 h of reaction at 55°C, wherein the aqueous ammonia solution was used in an amount of 0.5 ml/μmol. The solvent was removed, and the residue was concentrated under vacuum to dryness. After the treatment by aqueous ammonia solution, the resulting product was dissolved with 0.4 ml/μmol N-methylpyrrolidone according to the amount of single-stranded nucleic acid, and then added with 0.3 ml/μmol triethylamine and 0.6 ml/μmol triethylamine trihydrofluoroacetate to remove 2'-O-TBDMS protection from the ribose.

The conditions for purification and desalination were as follows: the nucleic acids were purified using a preparative ion chromatography column (Source 15Q) with a gradient elution by NaCl. Specifically, eluent 1 was 20 mM sodium phosphate (pH 8.1) in a mixed solvent of water/acetonitrile (volume ratio of water: acetonitrile = 9: 1); eluent 2 was 1.5 M sodium chloride and 20 mM sodium phosphate (pH 8.1) in a mixed solvent of water/acetonitrile (volume ratio of water: acetonitrile = 9: 1); elution gradient was eluent 1: eluent 2 = (100: 0) to (50: 50). The eluate was collected, combined and desalted by using a reverse phase chromatography purification column. The conditions for desalination comprised using a sephadex column (filler: Sephadex-G25) for desalination, and deionized water for elution.

Detection: The purity detection was performed using ion exchange chromatography (IEX-HPLC), and the molecular weight was measured by liquid chromatography-mass spectrometry (LC-MS). The measured value of the molecular weight and the theoretical value of the molecular weight were compared. When the measured value approximately equaled to the theoretical value, it indicated that a sense strand of siRNA conjugated with a compound at 3' end was obtained.

Taking trimeric CR01008 as an example, the sense strand has a structure formula as shown below:

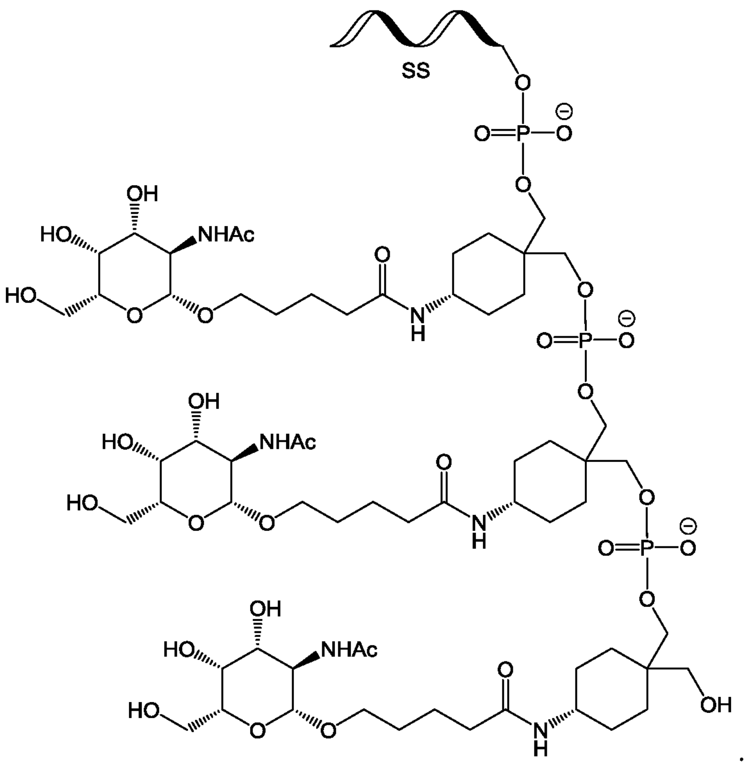

Taking trimeric CR01013 as an example, the sense strand has a structure formula as shown below:

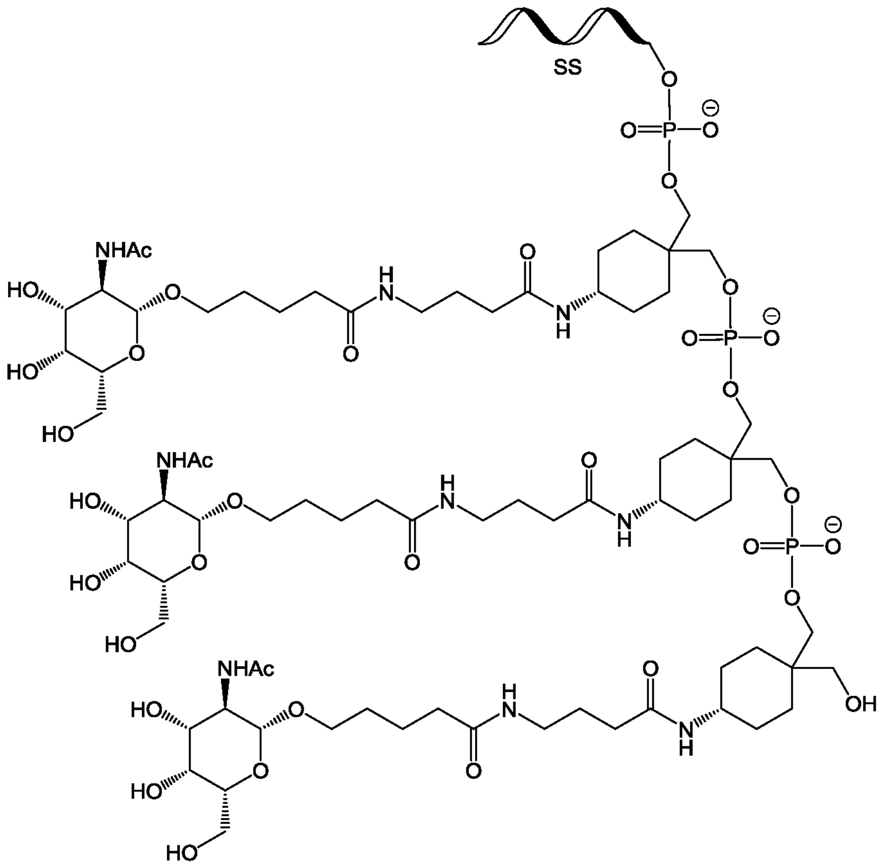

(2) Synthesis of antisense strand (AS)

Antisense strands were synthesized using a general solid phase support. The reaction conditions of deprotection, coupling, capping, oxidation or sulfurization, cleavage and deprotection, and purification and desalination in the solid phase synthesis method were the same as those used for the synthesis of the sense strand in step (1).

Detection: The purity detection was performed using ion exchange chromatography (IEX-HPLC), and the molecular weight was measured by liquid chromatography-mass spectrometry (LC-MS). The measured value of the molecular weight and the theoretical value of the molecular weight were compared. When the measured value approximately equaled to the theoretical value, it indicated that an antisense strand of siRNA was obtained. (3) Synthesis of siRNA conjugate:

The sense strand synthesized in step (1) and the antisense strand synthesized in step (2) were mixed at an equimolar ratio, dissolved in water for injection, heated to 95°C, slowly cooled to room temperature and left to stand at room temperature for 10 min to allow the sense and antisense strands to form a double-stranded structures by hydrogen bonds, thereby obtaining the siRNA conjugates shown in Table 3. As can be seen from the data in Table 4, the sense strand (SS) and antisense strand (AS) exhibited a favorable connection to the ligand and can maintain a higher purity.

Table 3 Sense and antisense strands of siRNA conjugate

| Number of conjugat e | Number of ligand | Sequence direction 5'-3' |
|---|---|---|
| RZ59900 1 | L96 | SS: UmsUmsUmUmAmAmUfCfCfUmCmAmCmUmCmUmAmAmAm_L96 (SEQ ID NO: 1) AS: UmsUfsUmAmGmAfGmUmGmAmGmGmAmUfUmAfAmAmAmsUmsGm (SEQ ID NO: 2) |
| RZ89901 5 | CR0100 8 | SS: UmsUmsUmUmAmAmUfCfCfUmCmAmCmUmCmUmAmAmAm(CR01008)×3 (SEQ ID NO: 1) AS: UmsUfsUmAmGmAfGmUmGmAmGmGmAmUfUmAfAmAmAmsUmsGm (SEQ ID NO: 2) |
| RZ89902 6 | CR0101 3 | SS: UmsUmsUmUmAmAmUfCfCfUmCmAmCmUmCmUmAmAmAm(CR01013)×3 (SEQ ID NO: 1) AS: UmsUfsUmAmGmAfGmUmGmAmGmGmAmUfUmAfAmAmAmsUmsGm (SEQ ID NO: 2) |
| RZ89902 7 | CR0101 4 | SS: UmsUmsUmUmAmAmUfCfCfUmCmAmCmUmCmUmAmAmAm(CR01014)×3 (SEQ ID NO: 1) AS: UmsUfsUmAmGmAfGmUmGmAmGmGmAmUfUmAfAmAmAmsUmsGm (SEQ ID NO: 2) |

(continued)

| Number of conjugat e | Number of ligand | Sequence direction 5'-3' |
|---|---|---|
| RZ59700 2 | L96 | SS: CmsCmsAmAmGmAmGfCfAfCmCmAmAmGmAmAmCmUmAm_L96 (SEQ ID NO: 3)<br>AS: UmsAfsGmUmUmCfUmUmGmGmUmGmCmUfCmUfUmGmGmsUmsUm (SEQ ID NO: 4) |
| RZ89700 1 | CR0101 3 | SS: CmsCmsAmAmGmAmGfCfAfCmCmAmAmGmAmAmCmUmAm(CR01013)×3 (SEQ ID NO: 3)<br>AS: UmsAfsGmUmUmCfUmUmGmGmUmGmCmUfCmUfUmGmGmsUmsUm (SEQ ID NO: 4) |
| RZ89700 2 | CR0101 4 | SS: CmsCmsAmAmGmAmGfCfAfCmCmAmAmGmAmAmCmUmAm(CR01014)×3 (SEQ ID NO: 3)<br>AS: UmsAfsGmUmUmCfUmUmGmGmUmGmCmUfCmUfUmGmGmsUmsUm (SEQ ID NO: 4) |

Table 4 Detection results of siRNA conjugate

| Number of conjugate | Sense strand | | | Antisense strand | | |
|---|---|---|---|---|---|---|
| | Theoretical molecular weight | Measured molecular weight | Purity | Theoretical molecular weight | Measured molecular weight | Purity |
| RZ599001 | 7940.1 | 7940.5 | 96.9% | 7096.83 | 7097.2 | 95.2% |
| RZ899015 | 7723.7 | 7724.7 | 93.0% | 7096.83 | 7096.8 | 98.3% |
| RZ899026 | 7979 | 7980.4 | 98.5% | 7096.83 | 7097.2 | 98.9% |
| RZ899027 | 7894.7 | 7896.1 | 95.6% | 7096.83 | 7097.2 | 98.9% |
| RZ597002 | 8125.33 | 8125.9 | 98.3% | 6932.59 | 6933.5 | 99.0% |
| RZ897001 | 8164.23 | 8165.1 | 92.9% | 6932.59 | 6932.3 | 96.7% |
| RZ897002 | 8079.93 | 8081.2 | 96.1% | 6932.59 | 6932.3 | 96.7% |

Example 3 *In vivo* toxicity assay of siRNA-conjugates

C57BL/6J mice were randomly divided into 2 groups, with 2 mice in each group, half male and half female. In each experiment, mice were given a single subcutaneous injection of siRNA-conjugate at a dose of 300 mg/kg body weight (calculated based on siRNA) and observed for 14 days. No animal exhibited mortality or clinical symptoms related to adverse drug reactions. After the observation was completed, all mice were subjected to gross dissection, from which no abnormalities were found. Therefore, these results indicated that the siRNA-conjugates had good safety and low toxicity at animal level.

Example 4 Inhibition of mRNA expression level of target gene in mice by siRNA-conjugates

6-8 weeks old female C57BL/6J mice were randomly grouped based on body weight. The drug dose for each mouse was calculated according to the body weight of the mouse. Each siRNA conjugate was prepared with PBS into a solution of the corresponding concentration (calculated based on siRNA) for administration. Mice were given a single subcutaneous injection in the abdomen at a volume of 5 ml/kg body weight (calculated based on siRNA). The PBS control group was administered with an equal volume of PBS solution without containing the siRNA conjugate. The day of administration was noted as Day 1 (D1). At predetermined time points, such as 8 days (noted as D8), 15 days (noted as D15), and 29 days (noted as D29) after administration, five mice from each group were sacrificed. These sacrificed mice were subjected to gross dissection, and they liver tissues were collected. The liver tissues were cut into pieces of approximately 2 $mm^3$ and stored in RNA Later.

Liver tissue samples from different experimental groups at different time points were taken from the above RNA later, and homogenized in an automatic tissue homogenizer-Tissuelyser II for 60 seconds. According to the standard procedure for total RNA extraction, the total RNA was extracted using an automatic nucleic acid extractor (purchased from Zhejiang Hanwei Technology Co., Ltd.) and a nucleic acid extraction kit (purchased from Zhejiang Hanwei Technology Co., Ltd.).

A reverse transcription kit (Promega, Reverse Transcription System, A3500) was used, 1 μg of the above total RNA was taken, Oligo $(dT)_{15}$ primers for reverse transcription were selected, a 20 μL of reverse transcription system was prepared according to the instructions of the reverse transcription kit, and the reverse transcription reaction was carried out. After the reaction was completed, 80 μL of RNase-Free water was added to the reverse transcription system to obtain the cDNA solution. Next, the mRNA expression of target genes in liver tissues was measured with a real-time fluorescence quantitative PCR kit (ABI, SYBR™ Select Master Mix, Catalog number: 4472908). In the real-time fluorescence quantitative PCR assay, a primer for the target gene and a primer for the internal reference gene were used to detect the target gene and the internal reference gene, respectively. A 20 μL Real-time PCR reaction system per PCR reaction well was prepared according to the protocol documented in the instructions of the real-time fluorescence quantitative PCR kit, and each reaction system consisted of 5 μL of cDNA solution obtained by the reverse transcription reaction described above, 10 μL of SYBR™ Select Master Mix, 0.5 μL of 10 μM upstream primer, 0.5 μL of 10 μM downstream primer, and 4μL of RNase-Free $H_2O$. The prepared reaction system was placed in a real-time fluorescence quantitative PCR instrument (ABI, StepOnePlus™), and real-time PCR amplification was performed by a three-step method. The amplification procedure comprised pre-denaturing at 95°C for 10 min, denaturing at 95°C for 30 seconds, annealing at 60°C for 30 seconds, and extending at 72°C for 30 seconds. The above denaturing, annealing and extending processes were repeated for 40 cycles. In this real-time fluorescence quantitative PCR assay, the ΔΔCt method was used to relatively quantify the mRNA expression level of the target gene and the inhibition rate in each test group, which was calculated as

follows:

$$\Delta Ct(\text{test group}) = Ct(\text{target gene in test group}) - Ct(\text{internal reference gene in test group})$$

$$\Delta Ct(\text{control group}) = Ct(\text{target gene in control group}) - Ct(\text{internal reference gene in control group})$$

$$\Delta\Delta Ct(\text{test group}) = \Delta Ct(\text{test group}) - \Delta Ct(\text{mean of control group})$$

$$\Delta\Delta Ct(\text{control group}) = \Delta Ct(\text{control group}) - \Delta Ct(\text{mean of control group})$$

wherein, ΔCt(mean of control group) is the arithmetic mean of each ΔCt(control group) of five mice sacrificed at the same time point in the control group. Each mouse in both the test and control groups thereby corresponds to a ΔΔCt value.

The mRNA expression level of the target gene in the test group was normalized using the control group as the baseline, and the mRNA expression level of the target gene in the control group was defined as 100%.

$$\text{Relative expression level of mRNA of target gene in test group} = 2^{-\Delta\Delta Ct\ (\text{test group})} \times 100\%$$

Inhibition rate of mRNA of target gene in test group = (1-relative expression level of mRNA of target gene in test group) × 100%

Example 4-1 *In vivo* activity assay of the trimeric CR01008 carrier conjugated with siRNA of superoxide dismutase 1

According to the method for inhibiting the mRNA expression of the target gene in mice, in this example, the *in vivo* inhibitory activity of the siRNA sequence RZ899015 conjugated with trimeric CR01008 carrier at 3' end of sense strand (RZ899015 for short), and the L96 conjugate RZ599001 (RZ599001 for short) on the target gene SOD1 in mice was evaluated. RZ899015 and RZ599001 had identical nucleic acid sequences and chemical modifications, and differed only in the delivery carrier structure.

6-8 weeks old C57BL/6J mice were randomly divided into 3 groups according to their body weight, i.e., PBS control group, RZ899015 group and RZ599001 group, with 5 mice in each group. PBS solution, RZ899015 and RZ599001 were respectively administered subcutaneously into the abdomen of mice of each group, at a dose of 1 mg/kg per mouse and a volume of 5 mL/kg. The day of administration was recorded as day 1 (D1), and the mice were sacrificed 8 days after the administration (D8).

The experimental results are shown in FIG. 1 and Table 6, indicating that at day 8, the trimeric CR01008 conjugate RZ899015 group and the L96 conjugate RZ599001 group exhibited comparable activity *in vivo.*

Table 5 Primer sequence in Example 4-1

| Target | | Primer | Primer sequence (5'-3') |
|---|---|---|---|
| Target gene | SOD1 | Upstream primer | GGGTTCCACGTCCATCAGTA (SEQ ID NO: 5) |
| | | Downstream primer | ACACCGTCCTTTCCAGCAGT (SEQ ID NO: 6) |
| Internal reference gene | GAPDH | Upstream primer | TGCACCACCAACTGCTTAG (SEQ ID NO: 7) |
| | | Downstream primer | GGATGCAGGGATGATGTTC (SEQ ID NO: 8) |

Table 6 Inhibitory activity for target gene in mice

| Group | D8 | |
|---|---|---|
| | Average inhibition rate | ±SD value |
| PBS | 0.00% | 9.00 |
| RZ599001 | 80.41% | 4.77 |
| RZ899015 | 80.39% | 2.48 |

Example 4-2 *In vivo* activity assay of the siRNA targeting SOD1 conjugated with trimeric CR01008 carrier, CR01013 carrier and CR01014 carrier

According to the method for inhibiting the mRNA expression of the target gene in mice, in this example, the *in vivo* inhibitory activity of the siRNA sequence RZ899015 conjugated with trimeric CR01008 carrier at 3' end of sense strand (RZ899015 for short), the siRNA sequence RZ899026 conjugated with trimeric CR01013 carrier (RZ899026 for short), the siRNA sequence RZ899027 conjugated with trimeric CR01014 carrier (RZ899027 for short), and the L96 conjugate RZ599001 (RZ599001 for short) on the target gene SOD1 in mice was evaluated. RZ899015, RZ899026, RZ899027 and RZ599001 had identical nucleic acid sequences and chemical modifications, and differed only in the delivery carrier structure. Among them, RZ899015 (conjugated with CR01008 carrier) differed from RZ899026 (conjugated with CR01013 carrier) only in the length of the linker of the carrier.

6-8 weeks old C57BL/6J mice were randomly divided into 5 groups according to their body weight, i.e., PBS control group, RZ899015 group, RZ899026 group, RZ899027 group and RZ599001 group, with 20 mice in each group. PBS solution, RZ899015, RZ899026, RZ899027 and RZ599001 were respectively administered subcutaneously into the abdomen of mice in each group, at a dose of 3 mg/kg per mouse and a volume of 5 mL/kg. The day of administration was recorded as day 1 (D1), and 5 mice from each group were sacrificed on days 25 (D25), days 29 (D29), days 43 (D43), and days 57 (D57) after the administration.

As shown in FIG. 2 and Table 8, the experimental results showed that the trimeric CR01008 conjugate (RZ899015) and the trimeric CR01013 conjugate (RZ899026) exhibited superior *in vivo* inhibitory effects on the target gene and longer-lasting efficacy compared to the L96 conjugate (RZ599001). Notably, on D57, RZ899015 and RZ899026 still maintained 70.39% and 62.42% inhibition activity on target gene, respectively, while the L96 conjugate (RZ599001) maintained only 52.19% inhibition activity.

Table 7 Primer sequence in Example 4-2

| Target | | Primer | Primer sequence (5'-3') |
|---|---|---|---|
| Target gene | SOD1 | Upstream primer | GGGTTCCACGTCCATCAGTA (SEQ ID NO: 5) |
| | | Downstream primer | ACACCGTCCTTTCCAGCAGT (SEQ ID NO: 6) |
| Internal reference gene | GAPDH | Upstream primer | TGCACCACCAACTGCTTAG (SEQ ID NO: 7) |
| | | Downstream primer | GGATGCAGGGATGATGTTC (SEQ ID NO: 8) |

Table 8 Inhibitory activity for target gene in mice

| Group | D15 | | D29 | | D43 | | D57 | |
|---|---|---|---|---|---|---|---|---|
| | Average inhibition rate | ±SD value | Average inhibition rate | ±SD value | Average inhibition rate | ±SD value | Average inhibition rate | ±SD value |
| PBS | 0.00% | 23.77 | 0.00% | 5.63 | 0.00% | 28.78 | 0.00% | 10.58 |
| RZ599001 | 97.74% | 0.87 | 95.23% | 1.41 | 82.89% | 3.84 | 52.19% | 7.76 |
| RZ899015 | 97.60% | 0.51 | 97.07% | 0.49 | 93.14% | 1.35 | 70.39% | 5.07 |
| RZ899026 | 97.29% | 0.72 | 94.52% | 1.64 | 89.00% | 1.66 | 62.42% | 14.47 |
| RZ899027 | 95.87% | 0.79 | 86.48% | 2.34 | 42.91% | 13.47 | 3.66% | 12.90 |

Example 4-3 *In vivo* activity assay of the siRNA targeting angiopoietin-like protein 3 conjugated with trimeric CR01013 and CR01014 carriers

According to the method for inhibiting the mRNA expression of the target gene in mice, in this example, the *in vivo* inhibitory activity of the siRNA sequence RZ897001 conjugated with trimeric CR01013 carrier at 3' end of the sense strand (RZ897001 for short), the siRNA sequence RZ897002 conjugated with trimeric CR01014 carrier (RZ897002 for short), and the L96 conjugate RZ597002 (RZ597002 for short) on the target gene ANGPTL3 in mice was evaluated. RZ897001, RZ897002 and RZ597002 had identical nucleic acid sequences and chemical modifications, and differed only in the delivery carrier structure.

6-8 weeks old C57BL/6J mice were randomly divided into 4 groups according to their body weight, i.e., PBS control group, RZ897001 group, RZ897002 group, and RZ597002 group, with 20 mice in each group. PBS solution, RZ897001, RZ897002 and RZ597002 were respectively administered subcutaneously into the abdomen of mice in each group, at a dose of 3 mg/kg per mouse and a volume of 5 mL/kg. The day of administration was recorded as day 1 (D1), and 5 mice from each group were sacrificed on days 15 (D15), days 29 (D29), days 43 (D43), and days 57 (D57) after the administration.

As shown in FIG. 3 and Table 10, the experimental results showed that the trimeric CR01013 conjugate (RZ897001) exhibited superior *in vivo* inhibitory effect on the target gene and longer-lasting efficacy compared to the L96 conjugate (RZ597002). On D43, RZ897001 maintained 74.45% inhibition activity on target gene, while the L96 conjugate (RZ597002) maintained only 62.84% inhibition activity. On D57, RZ897001 still maintained 51.58% inhibition activity on target gene, while the L96 conjugate (RZ597002) maintained only 31.17% inhibition activity.

Table 9 Primer sequence in Example 4-3

| Target | | Primer | Primer sequence (5'-3') |
|---|---|---|---|
| Target gene | ANGPTL3 | Upstream primer | GAGGAGCAGCTAACCAACTTAAT (SEQ ID NO: 9) |
| | | Downstream primer | TCTGCATGTGCTGTTGACTTAAT (SEQ ID NO: 10) |
| Internal reference gene | GAPDH | Upstream primer | TGCACCACCAACTGCTTAG (SEQ ID NO: 7) |
| | | Downstream primer | GGATGCAGGGATGATGTTC (SEQ ID NO: 8) |

Table 10 Inhibitory activity for target gene in mice

| Group | D15 | | D29 | | D43 | | D57 | |
|---|---|---|---|---|---|---|---|---|
| | Average inhibition rate | ±SD value | Average inhibition rate | ±SD value | Average inhibition rate | ±SD value | Average inhibition rate | ±SD value |
| PBS | 0.00% | 27.44 | 0.00% | 26.64 | 0.00% | 22.54 | 0.00% | 18.17 |
| RZ597002 | 95.24% | 2.19 | 84.95% | 6.43 | 62.84% | 15.69 | 37.17% | 17.54 |
| RZ897001 | 94.70% | 2.43 | 88.36% | 3.64 | 74.45% | 7.00 | 51.58% | 8.21 |
| RZ897002 | 90.86% | 3.43 | 77.84% | 5.24 | 49.72% | 20.12 | 23.69% | 3.85 |

Any reference in this specification to "an embodiment", "some embodiments", "example", "specific examples" and "some examples", etc. means that a particular feature, structure, material or characteristic described in connection with the embodiment or example is included in at least one embodiment or example of the present disclosure. In this specification, the schematic expressions of the above terms are not necessarily directed to the same embodiment or example. Furthermore, the specific features, structures, materials or characteristics described may be combined in any suitable manner in any one or more embodiments or examples. In addition, those skilled in the art may combine and compose the different embodiments or examples and the features of the different embodiments or examples described in this specification without mutual contradiction.

Although the examples of the present disclosure haven been shown and described above, it should be understood that the above examples are exemplary and cannot be understood as a limitation to the present disclosure. Those skilled in the art can make changes, modifications, substitutions and variations to the above examples within the scope of the present disclosure.

## Claims

1. A compound represented by formula (Ia), a stereoisomer, a pharmaceutically acceptable salt, or a prodrug thereof,

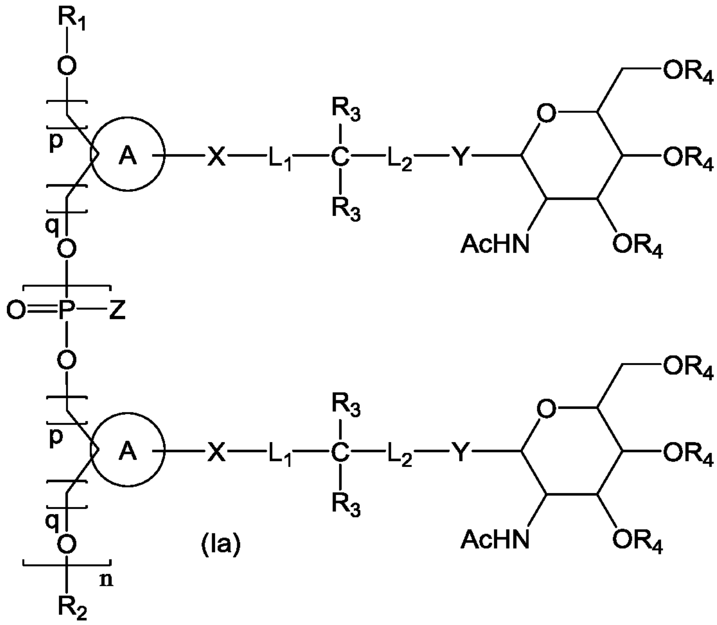

(Ia)

in formula (Ia), $R_1$ represents a hydroxyl protecting group,

$R_2$ is selected from the group consisting of:

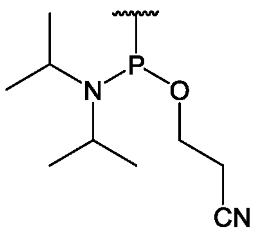
,
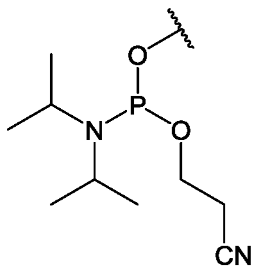
,

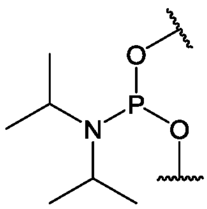
,
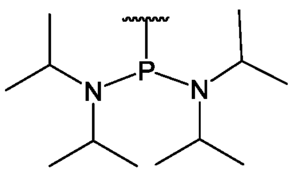
and
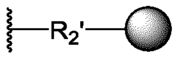
,

wherein

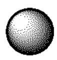

represents a solid phase support, and $R_2$' is a covalent linking group connected to the solid phase support,
n is selected from the group consisting of 0, 1, 2 and 3,
each Z is independently selected from the group consisting of hydroxyl and mercapto,
each p is independently selected from the group consisting of 1, 2 and 3,
each q is independently selected from the group consisting of 1, 2 and 3,

each A is independently an unsubstituted or substituted 4- to10-membered aliphatic ring,
each X is independently selected from the group consisting of NH, O and S,
each $L_1$ is independently selected from the group consisting of

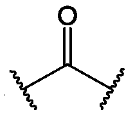, 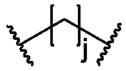

and

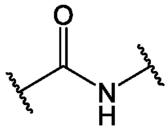,

wherein j is selected from the group consisting of 1, 2, 3, 4, 5, 6, 7, 8, 9 and 10,
each $R_3$ is independently selected from the group consisting of H, $C_1$-$C_6$ alkyl, $C_1$-$C_6$ haloalkyl, and $C_1$-$C_6$ alkoxy,
each $L_2$ is independently selected from the group consisting of $C_1$-$C_{30}$ alkylidene and

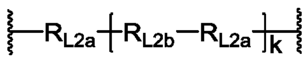,

wherein each $R_{L2a}$ is independently $C_1$-$C_{10}$ alkylidene, each $R_{L2b}$ is independently selected from the group consisting of O, S, NH, and -NH-C(O)-, and k is selected from the group consisting of 1, 2, 3, 4, 5, 6, 7, 8, 9, and 10,
each Y is independently selected from the group consisting of NH, O and S, and
each $R_4$ is independently selected from the group consisting of:

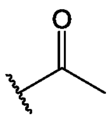, 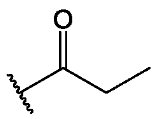,

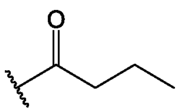, 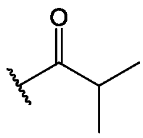, 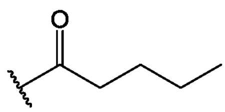, 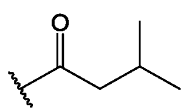, 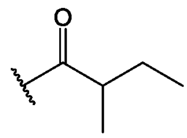,

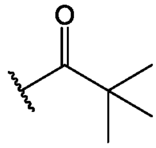, 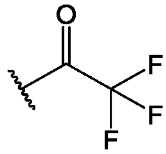 and 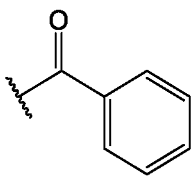.

2. The compound according to claim 1, wherein each A is independently selected from the group consisting of

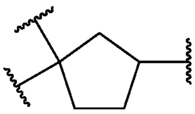, 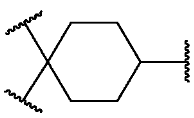, 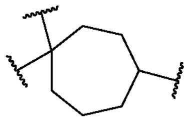, 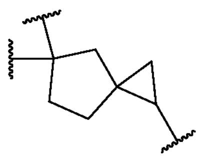

and

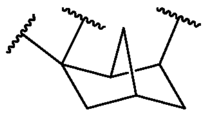.

3. The compound according to claim 1, wherein each X is NH.

4. The compound according to claim 1, wherein each $L_1$ is

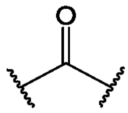.

5. The compound according to claim 1, wherein $R_1$ is selected from the group consisting of trityl, 4-methoxytrityl, 4,4'-dimethoxytrityl and 4,4',4''-trimethoxytrityl.

6. The compound according to claim 1, wherein each $L_2$ is independently selected from the group consisting of:

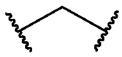, 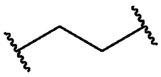, 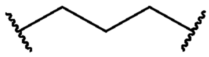, 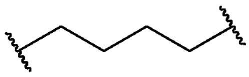, 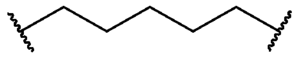,

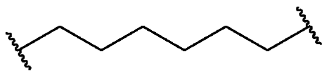, 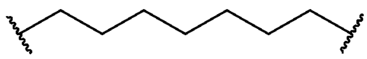, 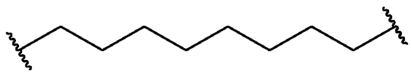,

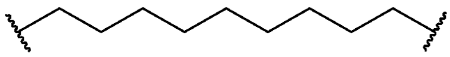, 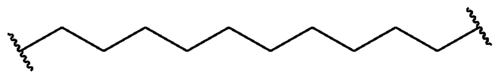,

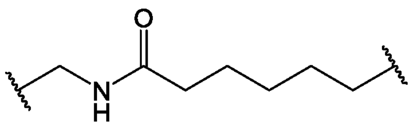, 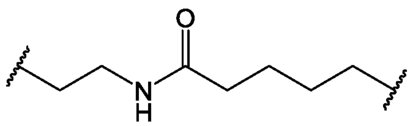,

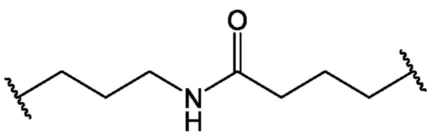, 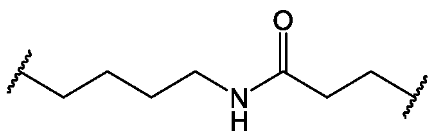,

and

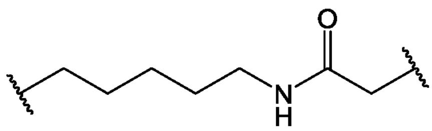.

7. The compound according to claim 1, wherein the compound has a structure represented by formula (IIa),

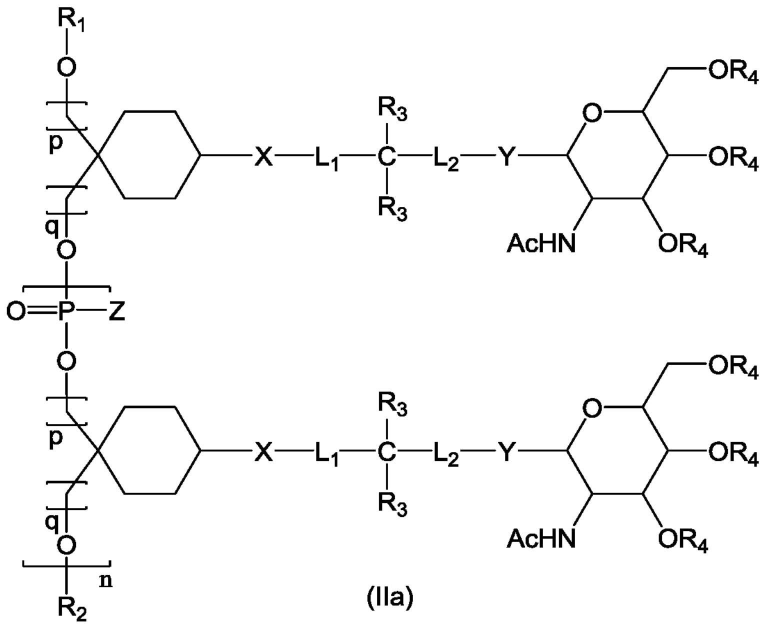

(IIa)

in formula (IIa), $R_1$, $R_2$, n, p, q, Z, X, $L_1$, $R_3$, $L_2$, Y, and $R_4$ are as defined in formula (Ia) of claim 1.

**8.** The compound according to claim 1, wherein the compound has a structure represented by formula (IIIa):

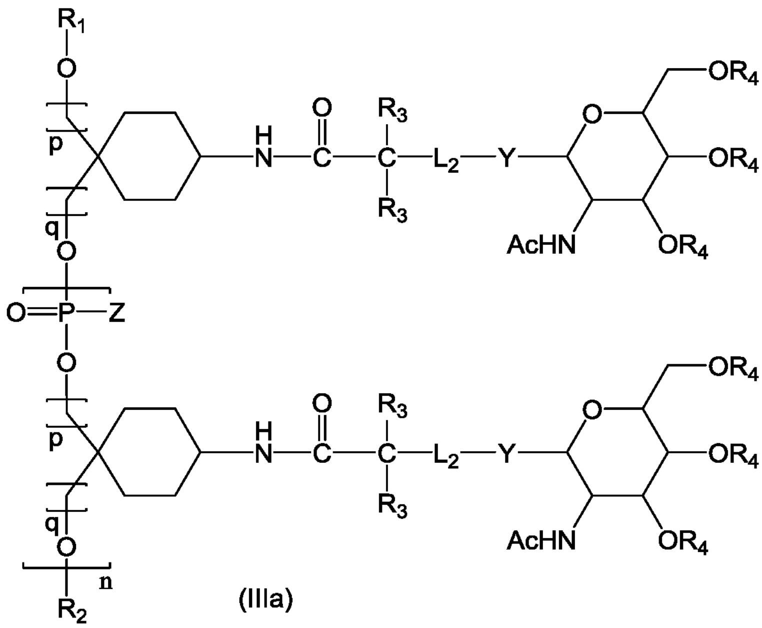

(IIIa)

in formula (IIIa), $R_1$, $R_2$, n, p, q, Z, $R_3$, $L_2$, Y, and $R_4$ are as defined in formula (Ia) of claim 1.

**9.** The compound according to claim 8, wherein when n is 0, the compound has a structure represented by the following formula:

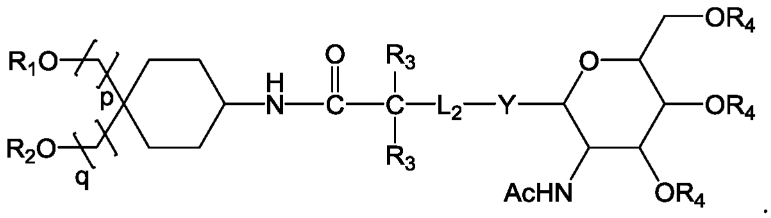

**10.** The compound according to claim 1, wherein the compound has a structure represented by formula (IVa):

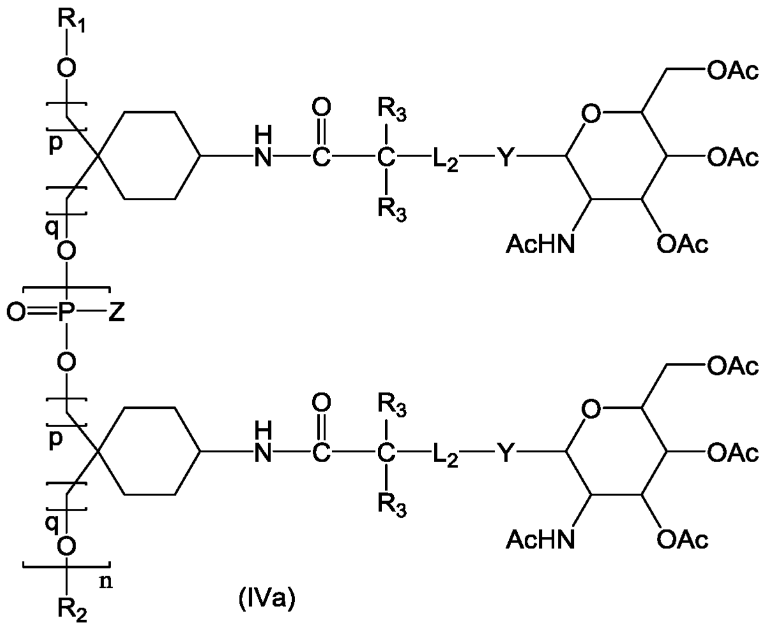

(IVa)

in formula (IVa), $R_1$, $R_2$, n, p, q, Z, $R_3$, $L_2$, and Y are as defined in formula (Ia) of claim 1.

**11.** The compound according to claim 10, wherein when n is 0, the compound has a structure represented by the following formula:

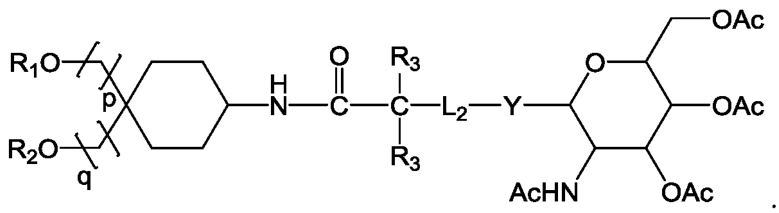

**12.** The compound according to claim 1, wherein the compound has a structure selected from the group consisting of:

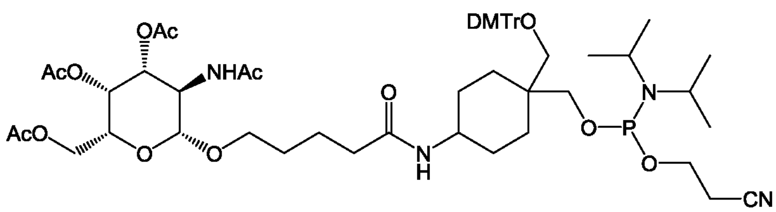

,

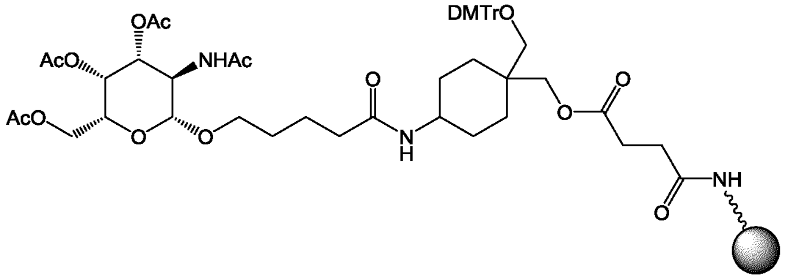

,

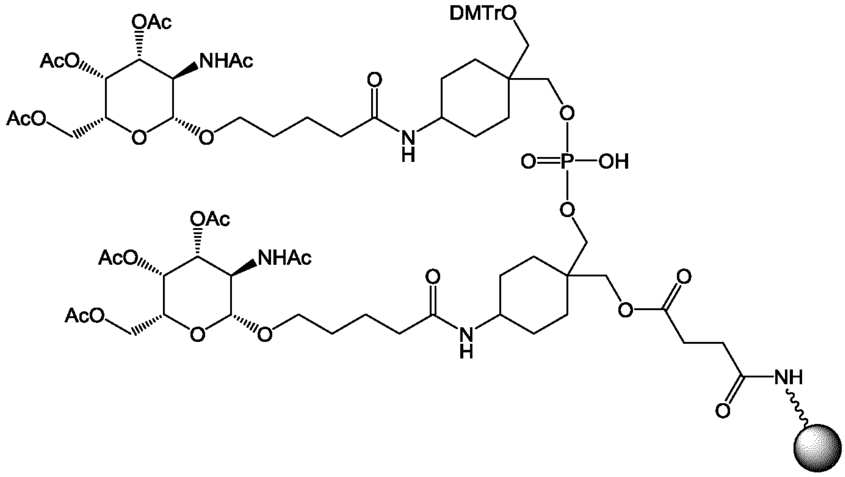

,

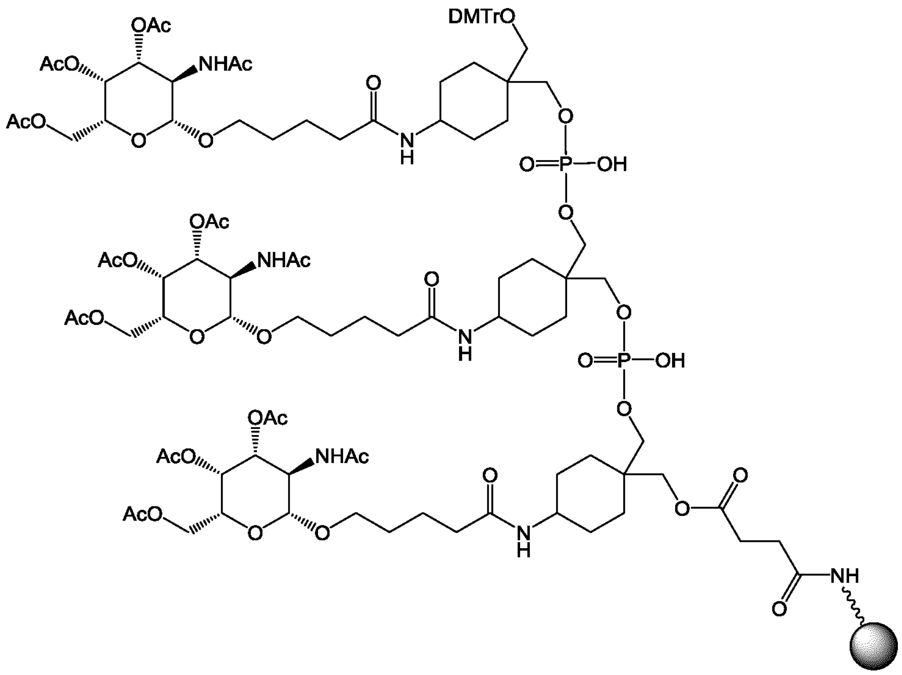

,

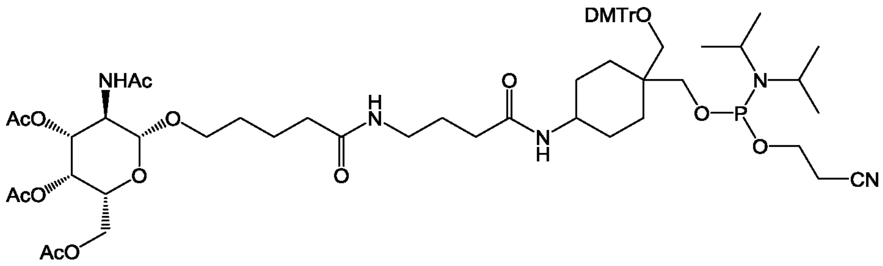

,

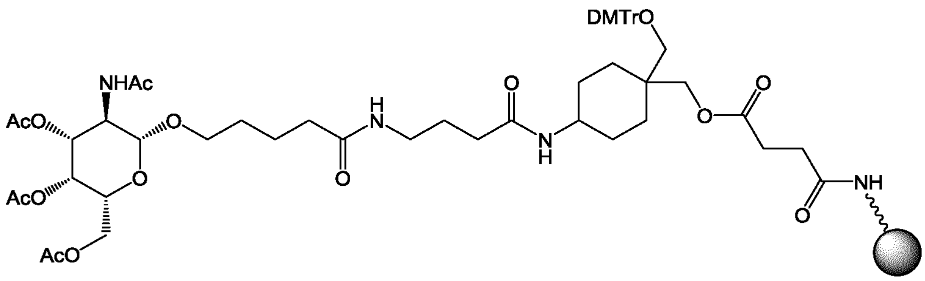

,

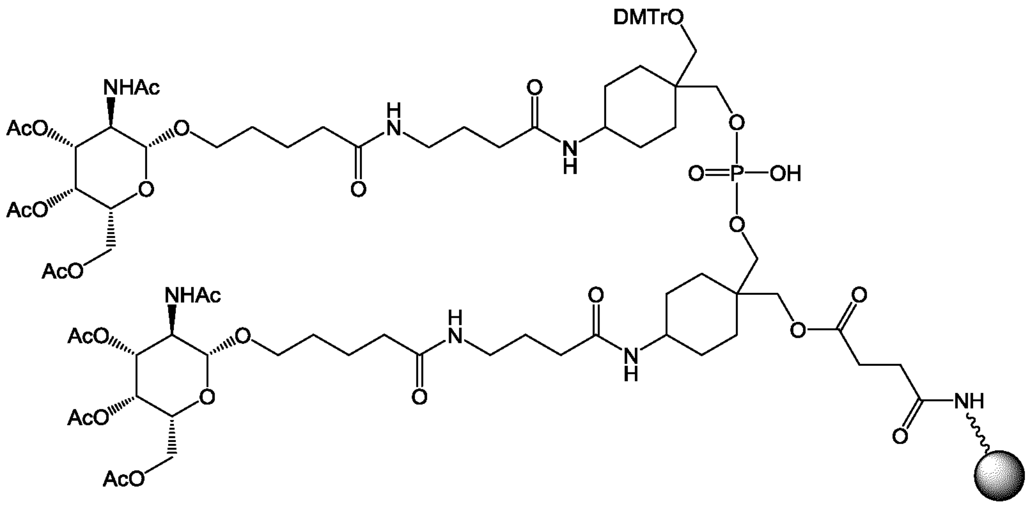

,

and

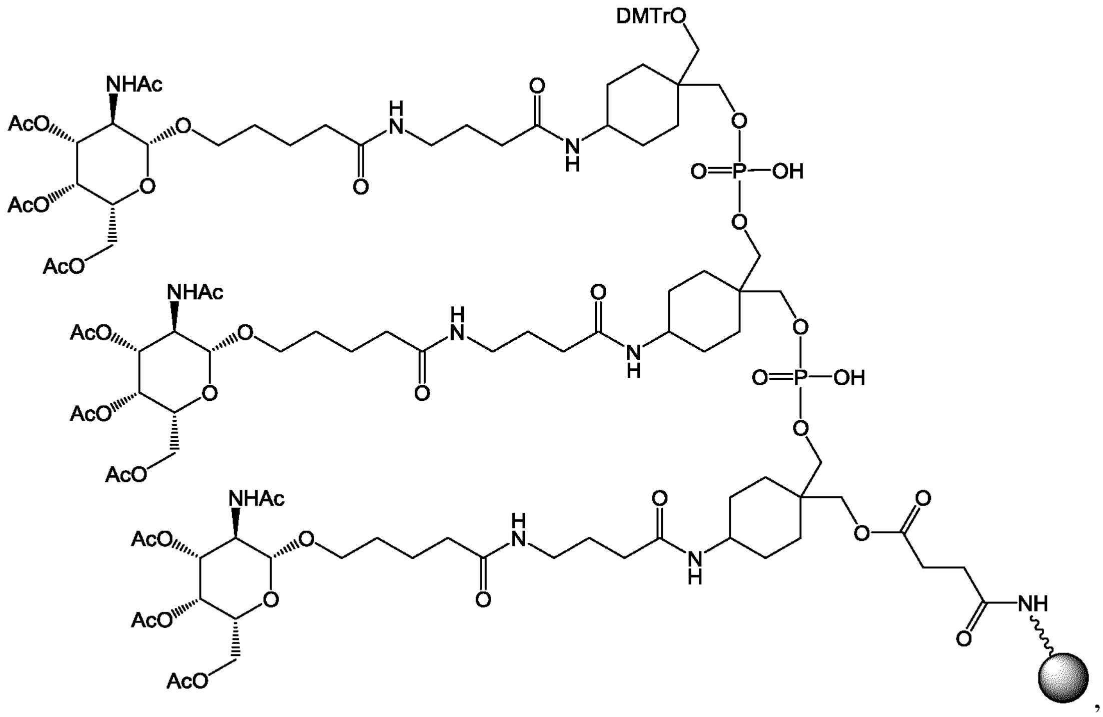

wherein,

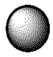

represents a solid phase support.

**13.** A compound having a structure represented by formula (IIIb), a stereoisomer, a pharmaceutically acceptable salt, or a prodrug thereof,

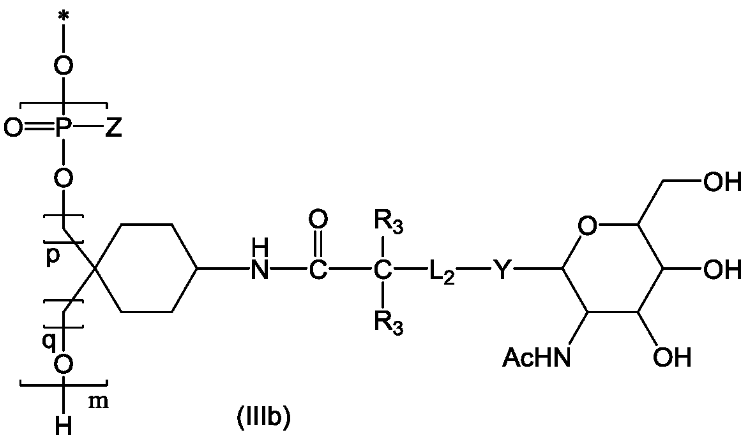

(IIIb)

in formula (IIIb), * represents a site of attachment for an active drug molecule,

m is selected from the group consisting of 1, 2, 3 and 4, and
Z, p, q, $R_3$, $L_2$ and Y are as defined in formula (Ia) of claim 1.

**14.** The compound according to claim 13, wherein the compound has a structure selected from the group consisting of:

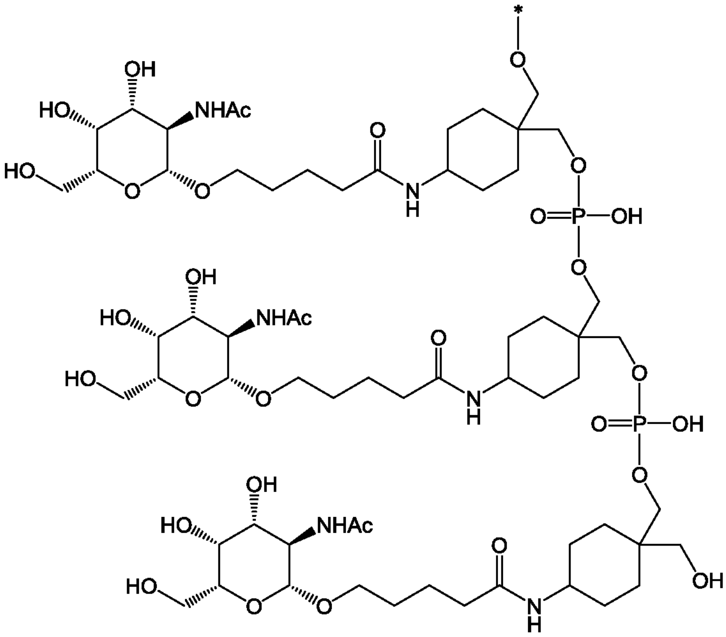

,

and

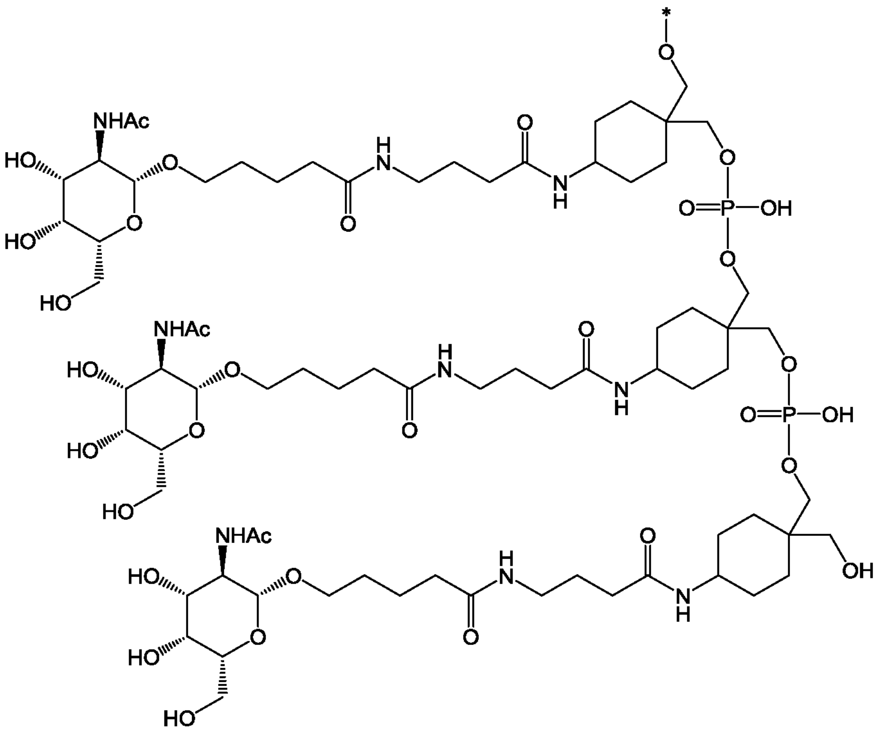

,

wherein, * represents a site of attachment for an active drug molecule.

**15.** A conjugate having a structure represented by formula (IIIc),

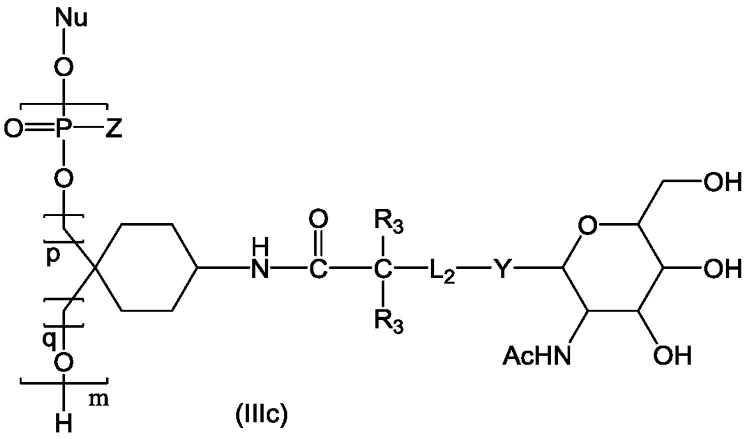

(IIIc)

in formula (IIIc), Nu represents an oligonucleotide,

m is selected from the group consisting of 1, 2, 3 and 4, and
Z, p, q, $R_3$, $L_2$ and Y are as defined in formula (Ia) of claim 1.

**16.** A composition comprising the conjugate according to claim 15.

**17.** Use of the compound according to any one of claims 1 to 14, the conjugate according to claim 15, or the composition according to claim 16 in the manufacture of a medicament for preventing and/or treating a disease.

**18.** Use of the compound according to any one of claims 1 to 14, the conjugate according to claim 15, or the composition according to claim 16 in the manufacture of a medicament for reducing the expression or activity of a target gene.

**19.** A method for reducing the expression or activity of a target gene, comprising contacting the conjugate according to claim 15 or the composition according to claim 16 with a cell.

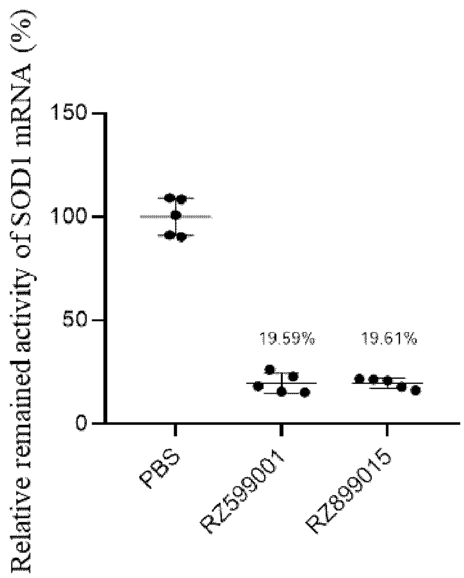
Relative remained activity of SOD1 mRNA (%)
150
100
50
0
19.59%
19.61%
PBS
RZ599001
RZ899015

FIG. 1

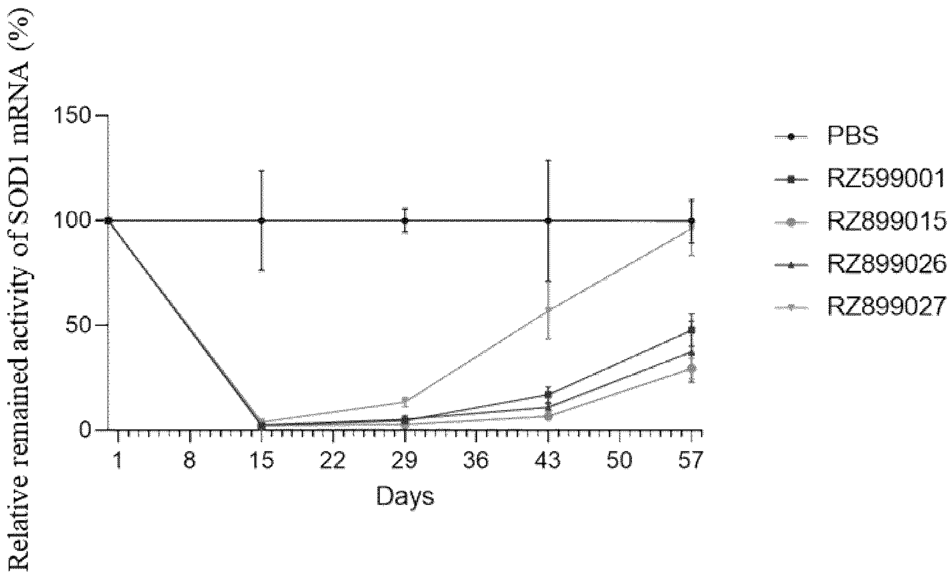
Relative remained activity of SOD1 mRNA (%)
150
100
50
0
1
8
15
22
29
36
43
50
57
Days
PBS
RZ599001
RZ899015
RZ899026
RZ899027

FIG. 2

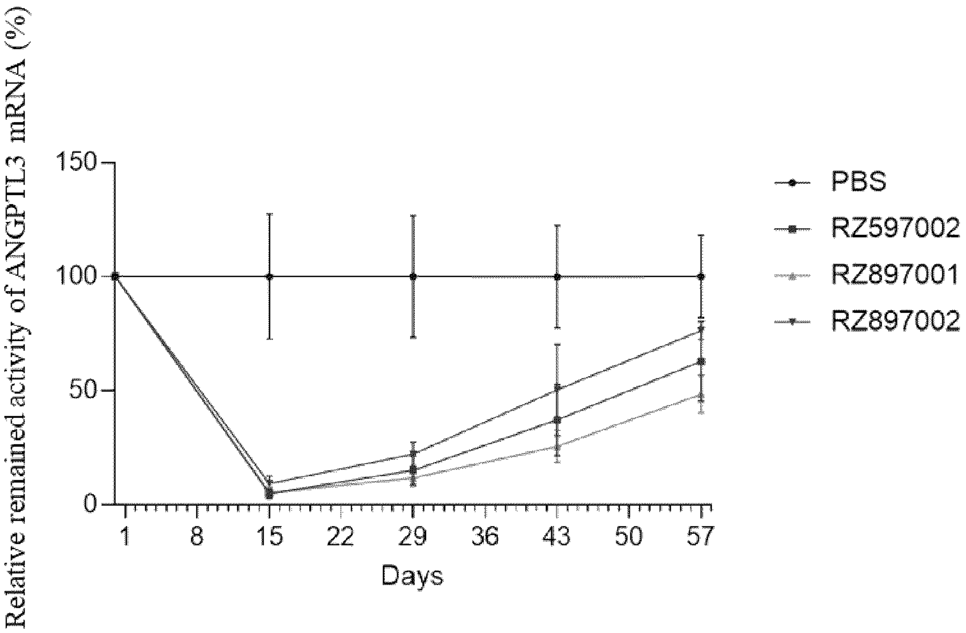
Relative remained activity of ANGPTL3 mRNA (%)
150
100
50
0
1
8
15
22
29
36
43
50
57
Days
PBS
RZ597002
RZ897001
RZ897002

FIG. 3

**INTERNATIONAL SEARCH REPORT**

International application No. **PCT/CN2023/088483**

**A. CLASSIFICATION OF SUBJECT MATTER**

C07H 15/04(2006.01)i; A61K31/7088(2006.01)i; A61K31/713(2006.01)i

According to International Patent Classification (IPC) or to both national classification and IPC

**B. FIELDS SEARCHED**

Minimum documentation searched (classification system followed by classification symbols)

IPC:C07H 15/-,A61K,

Documentation searched other than minimum documentation to the extent that such documents are included in the fields searched

Electronic data base consulted during the international search (name of data base and, where practicable, search terms used)

CNKI, CNABS, CNTXT, ENTXT, DWPI, REGISTRY, CAPLUS, MARPAT, 干扰RNA, ASODN, 小核酸, siRNA, 根据式I进行的结构式检索, structural formula search according to formula I.

**C. DOCUMENTS CONSIDERED TO BE RELEVANT**

| Category* | Citation of document, with indication, where appropriate, of the relevant passages | Relevant to claim No. |
|---|---|---|
| A | CN 110959011 A (SUZHOU RIBO LIFE SCIENCE CO., LTD.) 03 April 2020 (2020-04-03) entire document, in particular claims 38 and 69 | 1-19 |
| A | CN 111377984 A (SUZHOU RIBO LIFE SCIENCE CO., LTD.) 07 July 2020 (2020-07-07) entire document, in particular claims 11 and 22 | 1-19 |
| A | CN 112876534 A (SUZHOU RIBO LIFE SCIENCE CO., LTD.) 01 June 2021 (2021-06-01) entire document, in particular claims 7 and 8 | 1-19 |
| A | CN 112759620 A (SUZHOU RIBO LIFE SCIENCE CO., LTD.) 07 May 2021 (2021-05-07) entire document, in particular claims 6 and 13 | 1-19 |
| A | CN 111377985 A (SUZHOU RIBO LIFE SCIENCE CO., LTD.) 07 July 2020 (2020-07-07) entire document | 1-19 |
| A | CN 112390835 A (SUZHOU RIBO LIFE SCIENCE CO., LTD.) 23 February 2021 (2021-02-23) entire document | 1-19 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 December 2023** | **22 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/088483**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/088483**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19**
because they relate to subject matter not required to be searched by this Authority, namely:

Claim 19 relates to a method for treatment of the human or animal body (PCT Rule 39.1(iv)). Nevertheless, a search is still carried out on the basis of the technical subject matter of the use of the compound/composition in the preparation of a drug for reducing the expression or activity of a target gene.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/088483**

| Patent document cited in search report | Publication date (day/month/year) | Patent family member(s) | Publication date (day/month/year) |
|---|---|---|---|
| CN 110959011 A | 03 April 2020 | TW 201929905 A | 01 August 2019 |
| | | AU 2018394875 A1 | 09 April 2020 |
| | | AU 2018394875 B2 | 03 August 2023 |
| | | JP 2021509402 A | 25 March 2021 |
| | | US 2020338201 A1 | 29 October 2020 |
| | | US 11633482 B2 | 25 April 2023 |
| | | CA 3087106 A1 | 04 July 2019 |
| | | US 2023257827 A1 | 17 August 2023 |
| | | ZA 202003833 B | 26 January 2022 |
| | | KR 20200105812 A | 09 September 2020 |
| | | EP 3732185 A1 | 04 November 2020 |
| | | EP 3732185 A4 | 10 November 2021 |
| | | RU 2020121741 A | 01 February 2022 |
| | | WO 2019128611 A1 | 04 July 2019 |
| CN 111377984 A | 07 July 2020 | None | |
| CN 112876534 A | 01 June 2021 | None | |
| CN 112759620 A | 07 May 2021 | None | |
| CN 111377985 A | 07 July 2020 | None | |
| CN 112390835 A | 23 February 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Handbook of Chemistry and Physics. 1994
- Organic Chemistry. Thomas Sorrell, 1999
- **MICHAEL B. SMITH** ; **JERRY MARCH**. March s Advanced Organic Chemistry. John Wiley & Sons, 2007
- Dictionary of Chemical Terms. McGraw-Hill, 1984
- **ELIEL, E.** ; **WILEN, S.** Stereochemistry of Organic Compounds. John Wiley & Sons, Inc., 1994
- **S.M. BERGE et al.** *J. Pharmaceutical Sciences*, 1977, vol. 66, 1-19
- Pro-drugs as Novel Delivery Systems. **T. HIGUCHI** ; **V. STELLA**. A.C.S. Symposium Series. American Pharmaceutical Association and Pergamon Press, 1987, vol. 14
- **J. RAUTIO et al.** Prodrugs: Design and Clinical Applications. *Nature Review Drug Discovery*, 2008, vol. 7, 255-270
- **S. J. HECKER et al.** Prodrugs of Phosphates and Phosphonates. *Journal of Medicinal Chemistry*, 2008, vol. 51, 2328-2345
- Remington's Pharmaceutical Sciences. Mack Printing Company, 1990, 1289-1329